# EUROPÄISCHE PATENTSCHRIFT

(11) **EP 3 197 880 B1**
(45) Veröffentlichungstag und Bekanntmachung des Hinweises auf die Patenterteilung: **24.10.2018**
(21) Anmeldenummer: 15777624.6
(22) Anmeldetag: 22.09.2015
(51) Int. Cl.: C07D 401/12, C07D 405/06, C07D 213/69, A61P 7/02, A61P 29/00, A61P 1/04, A61P 27/02, A61K 31/4418, A61K 31/4439, A61K 31/4545, A61K 31/496, A61K 9/20

(54) **SUBSTITUIERTE OXOPYRIDIN-DERIVATE MIT ANTI-INFLAMMATORISCHER UND ANTI-THROMBOTISCHER WIRKUNG**
SUBSTITUTED OXOPYRIDINE DERIVATIVES WITH ANTI-INFLAMMATORY AND ANTI-THROMBOTIC EFFECT
DÉRIVÉS D'OXOPYRIDINE SUBSTITUÉS À EFFET ANTI-INFLAMMATOIRE ET ANTI-THROMBOTIQUE

(30) Priorität: 24.09.2014 EP 14186081
(43) Veröffentlichungstag der Anmeldung: 02.08.2017
(73) Patentinhaber: Bayer Pharma Aktiengesellschaft, 13353 Berlin (DE)
(72) Erfinder: ROEHRIG, Susanne, 40724 Hilden (DE); HILLISCH, Alexander, 42651 Solingen (DE); HEITMEIER, Stefan, 42489 Wülfrath (DE); SCHMIDT, Martina Victoria, 51063 Köln (DE); SCHLEMMER, Karl-Heinz, 42113 Wuppertal (DE); TERSTEEGEN, Adrian, 42111 Wuppertal (DE); TELLER, Henrik, 18258 Schwaan (DE)
(74) Vertreter: BIP Patents
(86) Internationale Anmeldenummer: PCT/EP2015/071650
(87) Internationale Veröffentlichungsnummer: WO 2016/046159

(56) Entgegenhaltungen:
- EP-A1- 1 526 131
- WO-A1-2013/056034
- WO-A1-2015/120777

## Beschreibung

Die Erfindung betrifft substituierte Oxopyridin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationskaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombinherstellung in dieser ersten Phase klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist in den Blickpunkt gerückt, dass neben der Stimulation über Tissue Faktor die Aktivierung des Gerinnungssystems an insbesondere negativ geladenen Oberflächen erfolgen kann, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa statt, der im Folgenden an Zelloberflächen gebundenen Faktor XI zu Faktor XIa aktiviert. Dieser führt wie zuvor beschrieben zur weiteren Aktivierung der Gerinnungskaskade. Daneben aktiviert Faktor XIIa ebenfalls gebundenes Plasmaprokallikrein zu Plasmakallikrein (PK), das zum einen zu weiterer Faktor XII-Aktivierung im Rahmen einer Potentierungsschleife führt, was insgesamt eine Verstärkung der Initiation der Gerinnungskaskade zur Folge hat. Zusätzlich stellt PK eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse beeinflussen kann. Damit stellt die Aktivierung von PK ein wichtiges Bindeglied zwischen koagulativen und inflammatorischen Prozessen dar.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen können ferner in extrakorporalen Blutkreisläufen, wie z. B. während einer Hämodialyse, sowie in Gefäß- beziehungsweise Herzklappenprothesen und Stents auftreten.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Aktivierung der Gerinnung kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein. Thromboembolische Erkrankungen gehören daher nach wie vor zu den häufigsten Ursachen von Morbidität und Mortalität in den meisten industrialisierten Ländern.

Die aus dem Stand der Technik bekannten Antikoagulantien, d.h. Stoffe zur Hemmung oder Verhinderung der Blutgerinnung, weisen verschiedene, Nachteile auf. Eine effiziente Behandlungsmethode beziehungsweise Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz und haben ihre Wirksamkeit in verschiedenen Studien unter Beweis gestellt. Allerdings kann es auch unter der Einnahme dieser Arzneimittel insbesondere bei prädisponierten Patienten zu Blutungskomplikationen kommen. Daher ist bei antithrombotischen Arzneimitteln die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vitro und in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) nicht zu spontanen Blutungen und fiel nur im Rahmen von Operationen und Traumen auf, zeigte aber eine Protektion gegenüber bestimmten thromboembolischen Ereignissen.

Daneben ist Plasmakallikrein (PK) mit weiteren Erkrankungen assoziiert, die mit erhöhten Gefäßpermeabilitäten oder chronisch-entzündlichen Erkrankungen einhergehen, wie es z.B. bei der diabetischen Retinopathie, dem Makulaödem und dem hereditären Angioödem beziehungsweise chronisch-entzündlichen Darmerkrankungen der Fall ist. Der diabetischen Retinopathie liegt in erster Linie eine Mikrogefäßschwäche zu Grunde, in deren Folge es zu einer Basalmembranverdickung der Gefäße und zum Verlust von gefäßummantelnden Perizyten, später zum Gefäßverschluss mit retinaler Ischämie kommt, welche aufgrund der hervorgerufenen retinalen Hypoxie zu verstärkter Gefäßpermeabilität mit nachfolgender Ausbildung eines Makulaödems und aufgrund aller vorliegender Prozesse zur Erblindung des Patienten führen kann. Beim Hereditären Angioödem (HAE) kommt es durch verminderte Bildung des physiologischen Kallikrein-Inhibitors C1-Esterase-Inhibitors zur unkontrollierten Plasmakallikrein-Aktivierung und damit zu Entzündungen mit fulminanter Ödembildung und starken Schmerzen. Aus tierexperimentellen Ansätzen gibt es Hinweise, dass die Inhibition von Plasmakallikrein die erhöhte Gefäßpermeabilität inhibiert und somit die Ausbildung eines Makulaödems beziehungsweise der diabetischen Retinopathie verhindern beziehungsweise die akute Symptomatik des HAE verbessern kann. Orale Plasmakallikrein-Inhibitoren könnten ebenfalls zur Prophylaxe des HAE eingesetzt werden.

Bei der Progression von chronisch-entzündlichen Darmerkrankungen (CED) haben vor allem die mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen Erkrankungen, und/oder ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen.

WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren. WO 2014/154794, WO 2014/160592, WO 2015/011087 und WO 2015/063093 beschreiben substituierte Pyridin-2-one und ihre Verwendung als Faktor XIa Inhibitoren.

Gegenstand der Erfindung sind Verbindungen der Formel 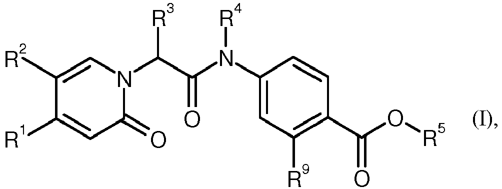 in welcher
- R¹: für eine Gruppe der Formel 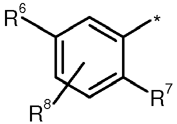 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R²: für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
- R³: für Wasserstoff, C₁-C₅-Alkyl, C₁-C₄-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo-Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
   und
worin Oxo-Heterocyclyl substituiert sein kann mit 1 bis 2 Substituenten unabhängig voneinander ausgewählt aus der Gruppe bestehend aus Oxo, Fluor, Methyl, Ethyl, Difluormethyl und Trifluormethyl,
- R⁴: für Wasserstoff steht,
- R⁵: für eine Gruppe der Formel 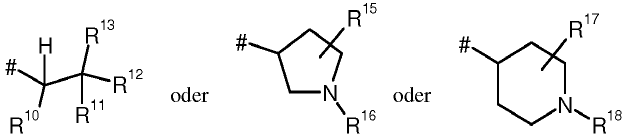 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, -N⁺(CH₃)₃, ein 5- oder 6-gliediges stickstoffhaltiges Heterocyclyl oder -NH(C=O)R¹⁴ steht,
worin Heterocyclyl substituiert sein kann mit 1 bis 2 Substituenten unabhängig voneinander ausgewählt aus der Gruppe bestehend aus C₁-C₃-Alkyl,
und
worin
R¹⁴ für C₁-C₄-Alkyl, ein 5- oder 6-gliediges stickstoffhaltiges Heterocyclyl oder Cyclopropyl steht,
worin Alkyl substituiert sein kann mit einem Substituenten ausgewählt aus der Gruppe bestehend aus Amino, Methylamino, Dimethylamino und einem 5- oder 6-gliedigen stickstoffhaltigen Heterocyclyl,
   worin der Heterocyclyl seinerseits substituiert sein kann mit 1 bis 2 Substituenten unabhängig voneinander ausgewählt aus der Gruppe bestehend aus C₁-C₃-Alkyl,
   und
worin Heterocyclyl substituiert sein kann mit 1 bis 2 Substituenten unabhängig voneinander ausgewählt aus der Gruppe bestehend aus C₁-C₃-Alkyl,
   und
worin Cyclopropyl substituiert sein kann mit einem Substituenten ausgewählt aus der Gruppe bestehend aus Amino, Methylamino und Dimethylamino,

R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
- R⁹: für Wasserstoff oder Fluor steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl steht für einen linearen oder verzweigten Alkylrest mit 1 bis 5 Kohlenstoffatomen, , bevorzugt 1 bis 4 Kohlenstoffatomen, besonders bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert.*-Butyl und 2,2-Dimethylprop-1-yl.
Alkoxy steht für einen linearen oder verzweigten Alkoxyrest mit 1 bis 4 Kohlenstoffatomen, bevorzugt 1 bis 3 Kohlenstoffatomen, beispielhaft und vorzugsweise für Methoxy, Ethoxy, n-Propoxy, iso-Propoxy, 2-Methyl-prop-1-oxy, n-Butoxy und *tert*.-Butoxy.
Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.
5- oder 6-gliediges stickstoffhaltiges Heterocyclyl in der Definition des Restes R¹² steht für einen gesättigten oder teilweise ungesättigten monocyclischen Rest mit 5 oder 6 Ringatomen, in dem ein Ringatom ein Stickstoffatom ist, und der Ring ein weiteres Heteroatome aus der Reihe O und N enthalten kann, beispielhaft und vorzugsweise für Pyrrolidinyl, Piperidinyl, Piperazinyl und Morpholinyl, bevorzugt sind Pyrrolidinyl, Piperidinyl und Piperazinyl, besonders bevorzugt sind Pyrrolidinyl und Piperazinyl.
5- oder 6-gliediges stickstoffhaltiges Heterocyclyl in der Definition des Restes R¹⁴ steht für einen gesättigten oder teilweise ungesättigten monocyclischen Rest mit 5 oder 6 Ringatomen, in dem ein Ringatom ein Stickstoffatom ist, und der Ring ein weiteres Heteroatome aus der Reihe O und N enthalten kann, beispielhaft und vorzugsweise für Pyrrolidinyl, Piperidinyl, Piperazinyl und Morpholinyl, bevorzugt sind Pyrrolidinyl, Piperidinyl und Piperazinyl, besonders bevorzugt sind Pyrrolidinyl und Piperazinyl.
4- bis 6-gliedriges Oxo-Heterocyclyl in der Definition des Restes R³ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

In den Formeln der Gruppe, die für R⁵ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁵ gebunden ist.

Bevorzugt sind Verbindungen der Formel (I), in welcher
- R¹: für eine Gruppe der Formel 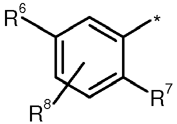 steht,
wobei * die Anknüpfstelle an den Oxopyridinring ist,
R⁶ für Chlor steht,
R⁷ für Cyano, Difluormethyl oder Difluormethoxy steht,
R⁸ für Wasserstoff oder Fluor steht,
- R²: für Chlor, Cyano, Ethoxy, Methoxy oder Difluormethoxy steht,
- R³: für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Cyclopropyl, Cyclobutyl, Cyclohexyl, Tetrahydrofuranyl, Tetrahydro-2H-pyranyl, Oxazolyl und Pyridyl,
   worin Cyclobutyl und Cyclohexyl substituiert sein können mit 1 bis 2 Substituenten unabhängig voneinander ausgewählt aus der Gruppe bestehend aus Hydroxy und Methoxy,
   und
wobei Ethyl, n-Propyl und n-Butyl substituiert sein können mit einem Substituenten ausgewählt aus der Gruppe bestehend aus Methoxy und Trifluormethoxy,
- R⁴: für Wasserstoff steht,
- R⁵: für eine Gruppe der Formel 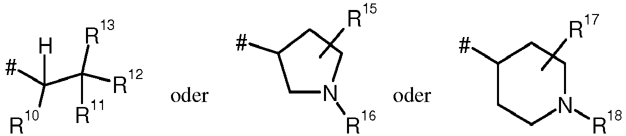 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, -N⁺(CH₃)₃, Pyrrolidinyl, Piperidinyl, Piperazinyl oder für -NH(C=O)R¹⁴ steht,
worin Pyrrolidinyl, Piperidinyl und Piperazinyl substituiert sein können mit einem Substituenten Methyl, und
worin
R¹⁴ für C₁-C₄-Alkyl, Pyrrolidinyl, Piperidinyl, Piperazinyl oder Cyclopropyl steht,
worin Alkyl substituiert sein kann mit einem Substituenten ausgewählt aus der Gruppe bestehend aus Amino, Methylamino, Dimethylamino, Pyrrolidinyl, Piperidinyl und Piperazinyl,
   worin Pyrrolidinyl, Piperidinyl und Piperazinyl ihrerseits substituiert sein können mit einem Substituenten Methyl,
   und
worin Pyrrolidinyl, Piperidinyl und Piperazinyl substituiert sein können mit einem Substituenten Methyl,
   und
worin Cyclopropyl substituiert sein kann mit einem Substituenten ausgewählt aus der Gruppe bestehend aus Amino, Methylamino und Dimethylamino,

R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
- R⁹: für Wasserstoff steht,
und ihre Salze, ihre Solvate und die Solvate ihrer Salze.

Bevorzugt sind auch Verbindungen der Formel (I), in welcher
- R¹: für eine Gruppe der Formel 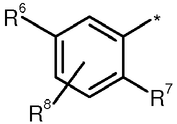 steht,
wobei * die Anknüpfstelle an den Oxopyridinring ist,
R⁶ für Chlor steht,
R⁷ für Cyano steht,
R⁸ für Wasserstoff steht,
- R²: für Methoxy steht,
- R³: für Methyl oder Ethyl steht,
wobei Methyl substituiert sein kann mit einem Substituenten Tetrahydro-2H-pyranyl,
und
wobei Ethyl substituiert sein kann mit einem Substituenten Methoxy,
- R⁴: für Wasserstoff steht,
- R⁵: für eine Gruppe der Formel 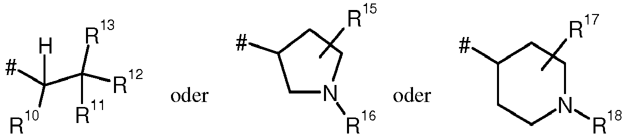 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, Pyrrolidinyl, Piperidinyl, Piperazinyl oder für -NH(C=O)R¹⁴ steht, worin Piperazinyl substituiert sein kann mit einem Substituenten Methyl,
und
worin
R¹⁴ für C₁-C₄-Alkyl, Pyrrolidinyl, Piperidinyl oder Cyclopropyl steht,
worin Alkyl substituiert sein kann mit einem Substituenten ausgewählt aus der Gruppe bestehend aus Amino, Dimethylamino und Pyrrolidinyl,
und
worin Pyrrolidinyl und Piperidinyl substituiert sein können mit einem Substituenten Methyl,
und
worin Cyclopropyl substituiert sein kann mit einem Substituenten Dimethylamino,
R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
- R⁹: für Wasserstoff steht,
und ihre Salze, ihre Solvate und die Solvate ihrer Salze.

Bevorzugt sind auch Verbindungen der Formel (I), in welcher
- R¹: für eine Gruppe der Formel 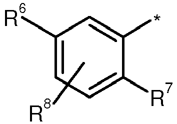 steht,
wobei * die Anknüpfstelle an den Oxopyridinring ist,
R⁶ für Chlor steht,
R⁷ für Cyano steht,
R⁸ für Wasserstoff steht.

Bevorzugt sind auch Verbindungen der Formel (I), in welcher R² für Methoxy steht.

Bevorzugt sind auch Verbindungen der Formel (I), in welcher
- R³: für Methyl oder Ethyl steht,
wobei Methyl substituiert sein kann mit einem Substituenten Tetrahydro-2H-pyranyl,
und
wobei Ethyl substituiert sein kann mit einem Substituenten Methoxy.

Bevorzugt sind auch Verbindungen der Formel (I), in welcher
- R⁵: für eine Gruppe der Formel 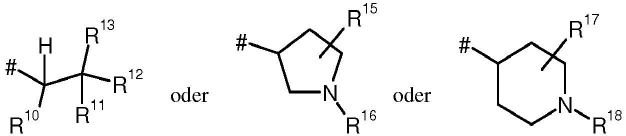 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, Pyrrolidinyl, Piperidinyl, Piperazinyl oder für -NH(C=O)R¹⁴ steht, worin Piperazinyl substituiert sein kann mit einem Substituenten Methyl, und worin
R¹⁴ für C₁-C₄-Alkyl, Pyrrolidinyl, Piperidinyl oder Cyclopropyl steht,
worin Alkyl substituiert sein kann mit einem Substituenten ausgewählt aus der Gruppe bestehend aus Amino, Dimethylamino und Pyrrolidinyl,
und
worin Pyrrolidinyl und Piperidinyl substituiert sein können mit einem Substituenten Methyl,
und
worin Cyclopropyl substituiert sein kann mit einem Substituenten Dimethylamino,
R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht.

Bevorzugt sind auch Verbindungen der Formel (I), in welcher
- R⁵: für eine Gruppe der Formel 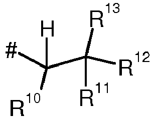 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, Pyrrolidinyl, Piperidinyl, Piperazinyl oder für -NH(C=O)R¹⁴ steht, worin Piperazinyl substituiert sein kann mit einem Substituenten Methyl,
und worin
R¹⁴ für C₁-C₄-Alkyl, Pyrrolidinyl, Piperidinyl oder Cyclopropyl steht,
worin Alkyl substituiert sein kann mit einem Substituenten ausgewählt aus der Gruppe bestehend aus Amino, Dimethylamino und Pyrrolidinyl,
und
worin Pyrrolidinyl und Piperidinyl substituiert sein können mit einem Substituenten Methyl,
und
worin Cyclopropyl substituiert sein kann mit einem Substituenten Dimethylamino,
R¹³ für Wasserstoff oder Hydroxycarbonyl steht.

Bevorzugt sind auch Verbindungen der Formel (I), in welcher
- R⁵: für eine Gruppe der Formel 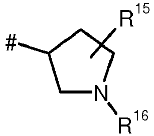 steht,
wobei # die Anknüpfstelle an das Sauerstoffatom ist,
R¹⁵ für Wasserstoff oder Hydroxycarbonyl steht,
R¹⁶ für Wasserstoff oder Methyl steht.

Bevorzugt sind auch Verbindungen der Formel (I), in welcher
- R⁵: für eine Gruppe der Formel 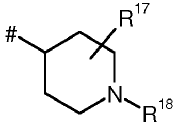 steht,
wobei # die Anknüpfstelle an das Sauerstoffatom ist,
R¹⁷ für Wasserstoff oder Hydroxycarbonyl steht,
R¹⁸ für Wasserstoff oder Methyl steht.

Bevorzugt sind auch Verbindungen der Formel (I), in welcher R⁹ für Wasserstoff steht.

Bevorzugt sind auch Verbindungen, welche die Formel (Ia) aufweisen 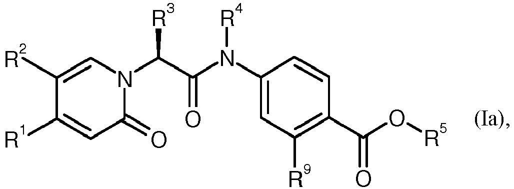 worin R¹, R², R³, R⁴, R⁵ und R⁹ wie oben definiert sind.

Gegenstand der Erfindung ist weiterhin ein Verfahren zur Herstellung der Verbindungen der Formel (I), oder ihrer Salze, ihrer Solvate oder der Solvate ihrer Salze, wobei die Verbindungen der Formel 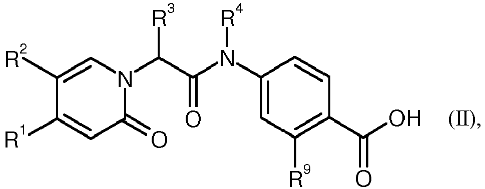 in welcher
R¹, R², R³, R⁴ und R⁹ die oben angegebene Bedeutung haben,
in der ersten Stufe mit Verbindungen der Formel

   HO-R^{5a} (III),

   in welcher
   R^{5a} die oben angegebene Bedeutung von R⁵ hat, wobei die funktionellen Gruppen in R⁵ mit Schutzgruppen, geschützt sein können,
   in Gegenwart eines Dehydratisierungsreagenzes umgesetzt werden,
   und in einer zweiten Stufe durch Abspaltung der Schutzgruppen zu Verbindungen der Formel 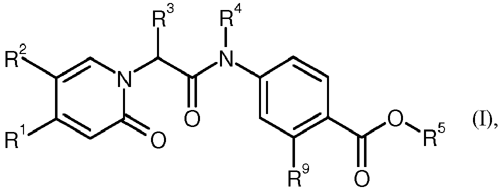 in welcher
      R¹, R², R³, R⁴, R⁵ und R⁹ die oben angegebene Bedeutung haben, umgesetzt werden.

Die Umsetzung der ersten Stufe erfolgt im Allgemeinen in inerten Lösungsmitteln, gegebenenfalls in Gegenwart einer Base, bevorzugt in einem Temperaturbereich von -20°C bis Raumtemperatur bei Normaldruck.

Als Dehydratisierungsreagenzien eignen sich hierbei beispielsweise Carbodiimide wie z.B. *N,N'-*Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylaminomethyliumfluoroborat (TBTU) oder *O*-(7-Azabenzotriazol-1-yl)-*N,N,N',N'*-tetramethyl-uronium-hexafluorophosphat (HATU), oder 1-Hydroxybenztriazol (HOBt), oder Benzotriazol-1-yloxy-tris(dimethylamino)-phosphoniumhexafluorophosphat (BOP), oder Ethyl-cyan(hydroxyimino)acetat (Oxyma), oder (1-Cyano-2-ethoxy-2-oxoethylidenaminooxy)dimethylamino-morpholino-carbenium hexafluorophosphat (COMU), oder N-[(Dimethylamino)(3*H-*[1,2,3]triazolo[4,5-b]pyridin-3-yloxy)methyliden]-N-methylmethanaminium-hexafluorophosphat, oder 2,4,6-Tripropyl-1,3,5,2,4,6-trioxatriphosphinan-2,4,6-trioxid (T3P), oder Mischungen aus diesen, mit Basen. Vorzugsweise wird die Kondensation mit *N*-(3-Dimethylaminoisopropyl)-*N'-*ethylcarbodiimid-Hydrochlorid (EDC) durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, oder Pyridin, oder Mischungen aus diesen Basen. Vorzugsweise wird die Kondensation mit einer Mischung aus Diisopropylethylamin und 4-Dimethylaminopyridin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dichlormethan.

Schutzgruppen für Amino-Gruppen sind beispielsweise *tert*-Butoxycarbonyl, Benzyl oder Benzyloxycarbonyl und Schutzgruppen für Carboxyl-Gruppen sind beispielsweise Benzyloxy oder *tert-*Butyl.

Die Umsetzung der zweiten Stufe erfolgt für Benzyl, Benzyloxycarbonyl und Benzyloxy Schutzgruppen im Allgemeinen mit einem Reduktionsmittel in inerten Lösungsmitteln, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Zinndichlorid, Titantrichlorid oder Ammoniumformiat und Palladium auf Aktivkohle in einem Gemisch aus Ethanol und Essigsäureethylester, bevorzugt ist Palladium auf Aktivkohle und Wasserstoff.

Inerte Lösungsmittel sind beispielsweise Ether wie Diethylether, Methyl-*tert*.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert.-*Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, als Lösungsmittel sind bevorzugt Methanol, Ethanol oder iso-Propanol.

Die Umsetzung der zweiten Stufe für *tert*-Butoxycarbonyl und *tert*-Butyl Schutzgruppen erfolgt wie für Verfahren [A] beschrieben.

Die Verbindungen der Formel (III) sind bekannt oder lassen sich nach bekannten Verfahren aus den entsprechenden Ausgangsverbindungen synthetisieren.

Die Verbindungen der Formel (II) sind bekannt oder können hergestellt werden, indem
[A] die Verbindungen der Formel 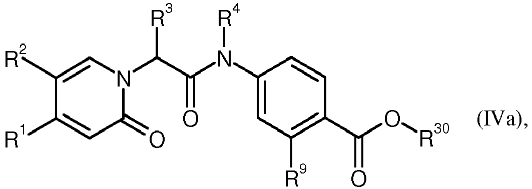 in welcher
   R¹, R², R³, R⁴ und R⁹ die oben angegebene Bedeutung haben, und
   R³⁰ für tert-Butyl steht,
   mit einer Säure umgesetzt werden,
      oder
[B] die Verbindungen der Formel 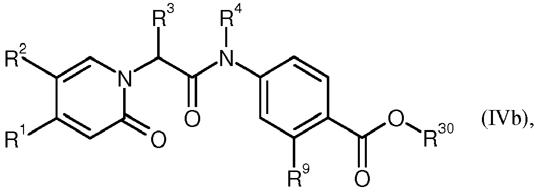 in welcher
   R¹, R², R³, R⁴ und R⁹ die oben angegebene Bedeutung haben, und
   R³⁰ für Methyl oder Ethyl steht,
   mit einer Base umgesetzt werden.

Die Verbindungen der Formeln (IVa) und (IVb) bilden zusammen die Menge der Verbindungen der Formel (IV).

Die Umsetzung nach Verfahren [A] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [B] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser oder ein Gemisch aus Methanol und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid oder Cäsiumcarbonat.

Die Verbindungen der Formel (IV) sind bekannt oder können hergestellt werden, indem Verbindungen der Formel 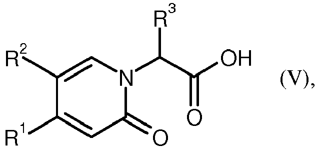 in welcher
R¹, R² und R³ die oben angegebene Bedeutung haben, mit Verbindungen der Formel 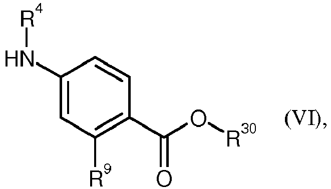 in welcher
R⁴ und R⁹ die oben angegebene Bedeutung haben, und
R³⁰ für Methyl, Ethyl oder tert-Butyl steht,
in Gegenwart eines Dehydratisierungsreagenzes umgesetzt werden.

Die Umsetzung erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'-*Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylamino-methyliumfluoroborat (TBTU) oder *O*-(7-Azabenzotriazol-l-yl)-*N,N,N',N'*-tetramethyl-uronium-hexafluorophosphat (HATU), oder 1-Hydroxybenztriazol (HOBt), oder Benzotriazol-1-yloxy-tris(dimethylamino)-phosphoniumhexafluorophosphat (BOP), oder Ethyl-cyan(hydroxyimino)acetat (Oxyma), oder (1-Cyano-2-ethoxy-2-oxoethylidenaminooxy)dimethylamino-morpholino-carbenium hexafluorophosphat (COMU), oder N-[(Dimethylamino)(3*H-*[1,2,3]triazolo[4,5-b]pyridin-3-yloxy)methyliden]-N-methylmethanaminium-hexafluorophosphat, oder 2,4,6-Tripropyl-1,3,5,2,4,6-trioxatriphosphinan-2,4,6-trioxid (T3P), oder Mischungen aus diesen, mit Basen. Vorzugsweise wird die Kondensation mit HATU oder mit T3P durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, oder Pyridin. Vorzugsweise wird die Kondensation mit Diisopropylethylamin oder Pyridin durchgeführt.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (VI) sind bekannt, lassen sich nach bekannten Verfahren aus den entsprechenden Ausgangsverbindungen synthetisieren.

Die Verbindungen der Formel (V) sind bekannt oder können hergestellt werden, indem
[C] Verbindungen der Formel 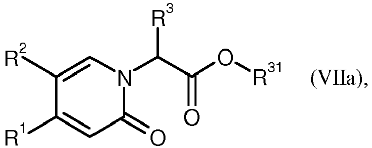 in welcher
   R¹, R² und R³ die oben angegebene Bedeutung haben, und
   R³¹ für tert-Butyl steht,
   mit einer Säure umgesetzt werden,
      oder
[D] Verbindungen der Formel 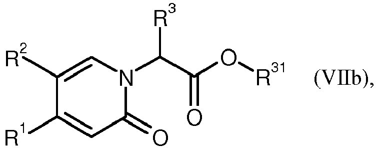 in welcher
   R¹, R² und R³ die oben angegebene Bedeutung haben, und
   R³¹ für Methyl, Ethyl oder Benzyl steht,
   mit einer Base umgesetzt werden.

Die Verbindungen der Formeln (VIIa) und (VIIb) bilden zusammen die Menge der Verbindungen der Formel (VII).

Die Umsetzung nach Verfahren [C] erfolgt wie für Verfahren [A] beschrieben.

Die Umsetzung nach Verfahren [D] erfolgt wie für Verfahren [B] beschrieben.

Die Verbindungen der Formel (VII) sind bekannt oder können hergestellt werden, indem
[E] Verbindungen der Formel 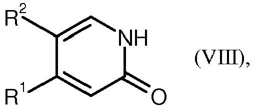 in welcher
   R¹ und R² die oben angegebene Bedeutung haben,
   mit Verbindungen der Formel 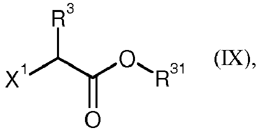 in welcher
      R³ die oben angegebene Bedeutung hat,
      R³¹ für Methyl, Ethyl, Benzyl oder tert-Butyl steht, und
      X¹ für Chlor, Brom, Iod, Methansulfonyloxy oder Trifluormethansulfonyloxy steht,
      umgesetzt werden,
         oder
[F] Verbindungen der Formel 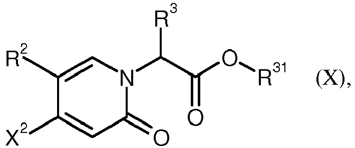 in welcher
   R² und R³ die oben angegebene Bedeutung haben,
   R³¹ für Methyl, Ethyl, Benzyl oder tert-Butyl steht, und
   X² für Chlor, Brom oder Iod steht,
   mit Verbindungen der Formel 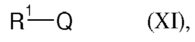 in welcher
      R¹ die oben angegebene Bedeutung hat, und
      Q für -B(OH)₂, einen Boronsäure-Ester, bevorzugt Boronsäurepinakolester, oder -BF₃⁻K⁺ steht,
      unter Suzuki-Kupplungsbedingungen umgesetzt werden.

Die Umsetzung nach Verfahren [E] erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Dimethylformamid.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Kalium- oder Natrium-tert-butylat, Natriumhydrid oder eine Mischung aus diesen Basen oder eine Mischung aus Natriumhydrid und Lithiumbromid, bevorzugt ist Kaliumcarbonat oder Natriumhydrid.

Die Verbindungen der Formel (IX) sind bekannt oder lassen sich nach bekannten Verfahren aus den entsprechenden Ausgangsverbindungen synthetisieren.

Die Umsetzung nach Verfahren [F] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen] - palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formel (XI) sind bekannt oder lassen sich nach bekannten Verfahren aus den entsprechenden Ausgangsverbindungen synthetisieren.

Die Verbindungen der Formel (VIII) sind bekannt oder können hergestellt werden, indem Verbindungen der Formel 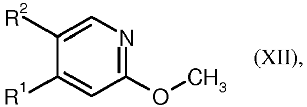 in welcher
R¹ und R² die oben angegebene Bedeutung haben,
mit Pyridinium-Hydrochlorid oder Pyridinium-Hydrobromid umgesetzt werden.

Die Umsetzung erfolgt im Allgemeinen in inerten Lösungsmitteln, bevorzugt in einem Temperaturbereich von 80°C bis 120°C bei Normaldruck.

Inerte Lösungsmittel sind beispielsweise Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen. Besonders bevorzugt ist Dimethylformamid.

Die Verbindungen der Formel (XII) sind bekannt oder können hergestellt werden, indem Verbindungen der Formel 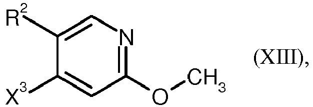 in welcher
R² die oben angegebene Bedeutung hat, und
X³ für Chlor, Brom oder Iod steht,
mit Verbindungen der Formel (XI) unter Suzuki-Kupplungsbedingungen umgesetzt werden.

Die Umsetzung erfolgt wie für Verfahren [F] beschrieben.

Die Verbindungen der Formel (XIII) sind bekannt oder lassen sich nach bekannten Verfahren aus den entsprechenden Ausgangsverbindungen synthetisieren.

Die Verbindungen der Formel (X) sind bekannt oder können hergestellt werden, indem Verbindungen der Formel 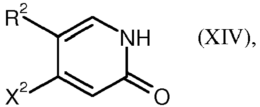 in welcher
R² die oben angegebene Bedeutung hat, und
X² für Chlor, Brom oder Iod steht,
mit Verbindungen der Formel (IX) umgesetzt werden.

Die Umsetzung erfolgt wie für Verfahren [E] beschrieben.

Die Verbindungen der Formel (XIV) sind bekannt oder lassen sich nach bekannten Verfahren aus den entsprechenden Ausgangsverbindungen synthetisieren.

In einem alternativen Verfahren können die Verbindungen der Formel (VII) hergestellt werden, indem Verbindungen der Formel 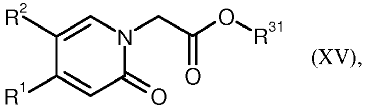 in welcher
R¹ und R² die oben angegebene Bedeutung haben, und
R³¹ für Methyl, Ethyl, Benzyl oder tert-Butyl steht,
mit Verbindungen der Formel 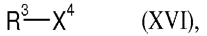 in welcher
   R³ die oben angegebene Bedeutung hat, und
   X⁴ für Chlor, Brom, Iod, Methansulfonyloxy, Trifluormethansulfonyloxy oder para-Toluolsulfonyloxy steht,
   umgesetzt werden.

Die Umsetzung erfolgt im Allgemeinen in inerten Lösungsmitteln, gegebenenfalls in Gegenwart einer Base, bevorzugt in einem Temperaturbereich von -78°C bis Raumtemperatur bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid, N-Butyllithium oder Bis-(trimethylsilyl)-lithiumamid, bevorzugt ist Bis-(trimethylsilyl)-lithiumamid.

Die Verbindungen der Formel (XV) sind bekannt oder lassen sich nach den oben beschriebenen Verfahren, z.B. Verfahren [E], aus den entsprechenden Ausgangsverbindungen synthetisieren.

Die Verbindungen der Formel (XVI) sind bekannt oder lassen sich nach bekannten Verfahren aus den entsprechenden Ausgangsverbindungen synthetisieren.

In einem alternativen Verfahren können die Verbindungen der Formel (V) hergestellt werden, indem Verbindungen der Formel 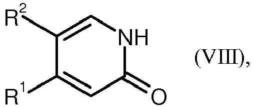 in welcher
R¹ und R² die oben angegebene Bedeutung haben,
mit Verbindungen der Formel 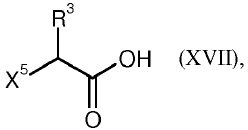 in welcher
   R³ die oben angegebene Bedeutung hat, und
   X⁵ für Chlor, Brom oder Iod steht,
   umgesetzt werden.

Die Umsetzung erfolgt im Allgemeinen in inerten Lösungsmitteln, gegebenenfalls in Gegenwart einer Base, bevorzugt in einem Temperaturbereich von -10°C bis 90°C bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Tetrahydrofuran.

Basen sind beispielsweise Kalium- oder Natrium-tert-butylat, Natriumhydrid oder Bis-(trimethylsilyl)-lithiumamid oder ein Gemisch aus Magnesiumdi-tert-butylat und Kalium-tert-butylat, bevorzugt ist ein Gemisch aus Magnesiumdi-tert-butylat und Kalium-tert-butylat.

Die Verbindungen der Formel (XVII) sind bekannt oder lassen sich nach bekannten Verfahren aus den entsprechenden Ausgangsverbindungen synthetisieren.

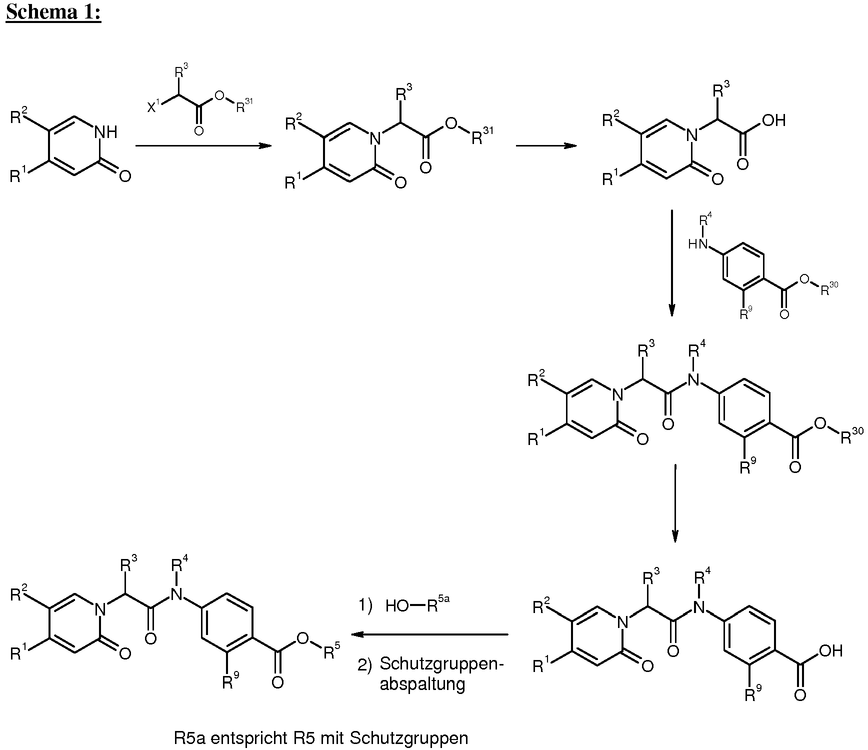

Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Faktor XIa (FXIa) und/oder der Serinprotease Plasmakallikrein (PK) beeinflussen. Die erfindungsgemäßen Verbindungen hemmen die von FXIa und/oder PK katalysierte enzymatische Spaltung von Substraten, die wesentliche Rollen in der Aktivierung der Blutgerinnung, in der Aggregation von Blutplättchen via Reduktion des für die PAR-1-Aktivierung der Plättchen notwendigen Thrombins, und in inflammatorischen Prozessen einnehmen, die insbesondere eine Steigerung der Gefäßpermeabilität miteinschließen.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasmakallikrein-Aktivität in Zusammenhang stehen, wie z.B. das hereditäre Angioödem (HAE) oder chronisch-entzündliche Erkrankungen, insbesondere des Darms, wie z. B. Morbus Crohn.

Faktor XIa (FXIa) ist ein wichtiges Enzym im Rahmen der Koagulation, das sowohl durch Thrombin als auch Faktor XIIa (FXIIa) aktiviert werden kann und somit in zwei wesentlichen Prozessen der Koagulation involviert ist: Es ist ein zentraler Bestandteil des Übergangs von der Initiation zur Amplifikation und Propagation der Koagulation: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/ Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist Faktor XIa wichtiger Bestandteil der intrinsischen Initiation der Gerinnung: Neben der Stimulation über Gewebefaktor (tissue factor, TF) kann die Aktivierung des Gerinnungssystems auch auf insbesondere negativ geladenen Oberflächen erfolgen, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa (FXIIa) statt, der im Folgenden an Zelloberflächen gebundenen FXI zu FXIa aktiviert. Dieser führt wie zuvor beschrieben zur weiteren Aktivierung der Gerinnungskaskade.

Dagegen bleibt die Thrombingenerierung in der Initiationsphase über TF/Faktor VIIa und Faktor X-Aktivierung und letztlich Thrombinbildung, die physiologische Reaktion auf Gefäßverletzungen, unbeeinflußt. Dies könnte erklären, warum keine Verlängerungen der Blutungszeiten in FXIa-Knock- out-Mäusen, wie auch in Kaninchen und anderen Spezies unter FXIa-Inhibitor-Gabe gefunden wurde. Diese niedrige durch die Substanz hervorgerufene Blutungsneigung ist für die Anwendung am Menschen insbesondere bei Patienten mit erhöhtem Blutungsrisiko von großem Vorteil.

Daneben aktiviert Faktor XIIa im Rahmen der intrinsischen Aktivierung ebenfalls Plasmaprokallikrein zu Plasmakallikrein (PK), das unter anderem zu weiterer Faktor XII-Aktivierung im Rahmen einer Potentierungsschleife führt, was insgesamt eine Verstärkung der Initiation der Gerinnungskaskade an Oberflächen zur Folge hat. Eine PK-hemmende Aktivität einer erfindungsgemäßen Verbindung wird also die Gerinnung via Oberflächenaktivierung reduzieren und somit antikoagulatorisch wirken. Ein Vorteil könnte in der Kombination von Faktor XIa- und PK-inhibitorischer Aktivität liegen, die eine balancierte antithrombotische Wirkung zuläßt.

Die erfindungsgemäßen Verbindungen eignen sich daher zur Behandlung und/oder Prophylaxe von Erkrankungen oder Komplikationen, die durch Gerinnselbildung entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen, Inflammation oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und bei Patienten, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen zur Behandlung und/oder Prophylaxe von Erkrankungen in Betracht, bei denen Mikrogerinnselbildungen beziehungsweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Dabei können sowohl die Faktor XIa-inhibitorische Komponente (über Hemmung der Thrombinproduktion) als auch die PK-inhibitorische Komponente zur antikoagulanten und antiinflammatorischen Wirkung (z.B. via Bradikinin) beitragen. Daher kommen unter anderem die Behandlung und/oder Prophylaxe im Rahmen von atherosklerotischen Gefäßerkrankungen, Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Bei der Progression von chronisch-entzündlichen Darmerkrankungen (CED) haben unter anderem mittels Plasmakallikrein generierten Kinine eine tragende Rolle. Deren pro-inflammatorische Wirkung über Aktivierung von Bradykinin-Rezeptoren induziert und potenziert den Krankheitsverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Außerdem können die erfindungsgemäßen Verbindungen zur Inhibition des Tumorwachstums und der Metastasenbildung, sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfläche von geschädigten Endothelzellen, Fremdkörperoberflächen oder vernetztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Erfindungsgemäße Verbindungen, die Plasmakallikrein alleine oder in Kombination mit Faktor XIa hemmen, kommen zusätzlich zur Behandlung und/oder Prophylaxe von Erkrankungen in Betracht, in deren Verlauf Plasmakallikrein involviert ist. Zusätzlich zur antikoagulanten Aktivität stellt Plasmakallikrein eine wichtige Bradikinin-freisetzende Protease dar, die somit unter anderem zum Anstieg der endothelialen Permeabilität führt. Die Verbindungen können somit zur Behandlung und/oder Prophylaxe von Erkrankungen eingesetzt werden, die mit Ödembildungen einhergehen, wie zum Beispiel ophthalmologische Erkrankungen, insbesondere die diabetische Retinopathie oder das Makulaödem, oder das hereditäre Angioödem.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterhin kommen die erfindungsgemäßen Verbindungen zur Primärprophylaxe thrombotischer oder thromboembolischer Erkrankungen und/oder inflammatorischer Erkrankungen und/oder Erkrankungen mit erhöhter Gefäßpermeabilität in Patienten in Frage, in denen Genmutationen zu verstärkter Aktivität der Enzyme oder erhöhten Spiegeln der Zymogene führen und diese durch entsprechende Tests/Messungen der Enzymaktivität bzw. Zymogenkonzentrationen festgestellt werden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa oder Plasmakallikrein enthalten könnten.

Weiterer Gegenstand der vorliegenden Erfindung ist ein Verfahren zur Verhinderung der Blutkoagulation *in vitro,* insbesondere bei Blutkonserven oder biologischen Proben, die Faktor XIa oder Plasmakallikrein oder beide Enzyme enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;

- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse®), Streptokinase, Reteplase und Urokinase oder Plasminogen-modulierende Substanzen, die zu verstärkter Plasmin-Bildung führen;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;
- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;
- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,
- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;
- Plättchenadhäsionshemmern wie GPVI- und/oder GPIb-Antagonisten wie beispielsweise Revacept oder Caplacizumab;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- rekombinantes humanes aktiviertes Protein C wie beispielsweise Xigris oder rekombinantes Thrombomodulin;
- sowie Antiarrhythmika;
- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;
- Hemmer der Angiopoietin-Tie Signalwege wie beispielsweise AMG386;
- Inhibitoren der Tie2 Rezeptortyrosinkinase;
- Hemmer der Integrin-Signalwege wie beispielsweise Volociximab, Cilengitid und ALG1001;
- Hemmer der PI3K-Akt-mTor Signalwege wie beispielsweise XL-147, Perifosin, MK2206, Sirolimus, Temsirolimus und Everolimus;
- Corticosteroid wie beispielsweise Anecortave, Betamethason, Dexamethason, Triamcinolon, Fluocinolon und Fluocinolonacetonid;
- Inhibitoren des ALK1-Smad1/5 Signalweges wie beispielsweise ACE041;
- Cyclooxygenaseinhibitoren wie beispielsweise Bromfenac und Nepafenac;
- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HTla Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikuläre/kolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die parenterale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- Boc: *tert.*-Butyloxycarbonyl
- ca.: circa
- d: Tag(e), Dublett (bei NMR)
- DC: Dünnschicht-Chromatographie
- DCM: Dichlormethan
- DCI: direkte chemische Ionisation (bei MS)
- dd: Doppeltes Dublett (bei NMR)
- DIC: *N,N'*-Diisopropylcarbodiimid
- DIEA: *N,N*-Diisopropylethylamin
- DMAP: 4-Dimethylaminopyridin
- DMF: *N,N*-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N,N,N',N'*-tetramethyluronium-Hexafluorphosphat
- HPLC: Hochdruck-, Hochleistungsflüssigchromatographie
- HV: Hochvakuum
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- Oxima: Hydroxyiminocyanoessigsaeureethylester
- q: Quartett oder Quadruplett (bei NMR)
- quant.: quantitativ
- quin: Quintett (bei NMR)
- RP: reverse phase (bei HPLC)
- RT: Raumtemperatur
- Rₜ: Retentionszeit (bei HPLC)
- s: Singulett (bei NMR)
- sxt: Sextett (bei NMR)
- SFC: superkritische Flüssigkeitschromatographie (mit überkritischem Kohlendioxid als mobile Phase)
- t: Triplett (bei NMR)
- THF: Tetrahydrofuran
- TFA: Trifluoressigsäure
- T3P: 2,4,6-Tripropyl-1,3,5,2,4,6-trioxatriphosphinan-2,4,6-trioxid

### HPLC-. LC/MS- und GC-Methoden:

Methode 1: Instrument: Waters ACQUITY SQD UPLC System; Säule: Waters Acquity UPLC HSS T3 1.8 µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.
Methode 2: Instrument: Waters ACQUITY SQD UPLC System; Säule: Waters Acquity UPLC HSS T3 1.8 µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.
Methode 3: Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.
Methode 4: Instrument MS: Waters (Micromass) Quattro Micro; Instrument HPLC: Agilent 1100 Serie; Säule: YMC-Triart C18 3µ 50 mm x 3 mm; Eluent A: 11 Wasser + 0.01 mol Ammoniumcarbonat, Eluent B: 11 Acetonitril; Gradient: 0.0 min 100% A → 2.75 min 5% A → 4.5 min 5% A; Ofen: 40°C; Fluss: 1.25 ml/min; UV-Detektion: 210 nm.
Methode 5: Instrument MS: Waters (Micromass) QM; Instrument HPLC: Agilent 1100 Serie; Säule: Agient ZORBAX Extend-C18 3.0 mm x 50 mm 3.5-Micron; Eluent A: 11 Wasser + 0.01 mol Ammoniumcarbonat, Eluent B: 11 Acetonitril; Gradient: 0.0 min 98% A → 0.2 min 98% A → 3.0 min 5% A→ 4.5 min 5% A; Ofen: 40°C; Fluss: 1.75 ml/min; UV-Detektion: 210 nm.
Methode 6: Instrument MS: Waters (Micromass) ZQ; Instrument HPLC: Agilent 1100 Serie; Säule: Agient ZORBAX Extend-C18 3.0 mm x 50 mm 3.5-Micron; Eluent A: 11 Wasser + 0.01 mol Ammoniumcarbonat, Eluent B: 11 Acetonitril; Gradient: 0.0 min 98% A → 0.2 min 98% A → 3.0 min 5% A→ 4.5 min 5% A; Ofen: 40°C; Fluss: 1.75 ml/min; UV-Detektion: 210 nm.
Methode 7: Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).
Methode 8: Instrument: Agilent MS Quad 6150; HPLC: Agilent 1290; Säule: Waters Acquity UPLC HSS T3 1.8 µ 50 mm x 2.1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 0.3 min 90% A → 1.7 min 5% A → 3.0 min 5% A; Ofen: 50°C; Fluss: 1.20 ml/min; UV-Detektion: 205-305 nm.
Methode 9: Instrument: Thermo Scientific DSQII, Thermo Scientific Trace GC Ultra; Säule: Restek RTX-35MS, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).
Mikrowelle: Als Mikrowellenreaktor wurde ein "single mode" Gerät vom Typ Emrys™ Optimizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische beziehungsweise saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungsweise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen

### Allgemeine Methode 1A: Darstellung einer Boronsäure

Eine Lösung des entsprechenden Pyridinderivates in Tetrahydrofuran (ca. 3 ml/mmol) wurde bei -78°C mit Lithiumdiisopropylamid (2M in Tetrahydrofuran/Heptan/Ethylbenzol) versetzt, 2 bis 4 h gerührt und anschließend zügig mit Triisopropylborat versetzt. Das Reaktionsgemisch wurde für weitere 2 bis 3 h bei -78°C gehalten und anschließend langsam über Nacht auf RT aufgetaut. Nach Zugabe von Wasser wurde das Tetrahydrofuran im Vakuum entfernt und die wässrige Phase zweimal mit Essigsäureethylester extrahiert. Die wässrige Phase wurde mit wässriger Salzsäure (2M) angesäuert, wobei in der Regel ein Niederschlag ausfiel, der filtriert, mit Wasser gewaschen und getrocknet wurde. Die wässrige Phase wurde dreimal mit Essigsäureethylester extrahiert. Die vereinigten, organischen Phasen wurden getrocknet (Natrium- oder Magnesiumsulfat), filtriert und im Vakuum eingeengt.

### Allgemeine Methode 2A: Suzuki-Kupplung

In einem ausgeheizten, mit Argon gespülten Kolben wurden 1.0 eq. der entsprechenden Boronsäuren, 1.0 eq. des Arylbromides oder Aryliodides, 3.0 eq. Kaliumcarbonat und 0.1 eq. [1,1-Bis-(diphenylphosphino)-ferrocen]-palladium(II)chlorid-Monodichlormethan-addukt oder Tetrakis(triphenylphosphin)palladium(0) vorgelegt. Der Kolben wurde anschließend dreimal evakuiert und immer wieder mit Argon belüftet. Das Reaktionsgemisch wurde mit Dioxan (ca. 6 ml/mmol) versetzt und für mehrere Stunden bis zur weitgehend vollständigen Umsetzung bei 110°C gerührt. Das Reaktionsgemisch wurde anschließend über Celite filtriert, das Filtrat im Vakuum eingeengt. Der Rückstand wurde mit Wasser versetzt. Nach Zugabe von Essigsäureethylester und Phasentrennung wurde die organische Phase einmal mit Wasser und einmal mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 3A: Methoxypyridin Spaltung

Eine Lösung des entsprechenden Methoxypyridins in Dimethylformamid (10-12.5 ml/mmol) wurde mit 20 eq. Pyridinium-Hydrochlorid oder Pyridinium-Hydrobromid versetzt und bei 100°C für mehrere Stunden bis Tage gerührt, eventuell mit weiterem Pyridinium-Hydrochlorid oder Pyridinium-Hydrobromid versetzt, bis zur weitgehend vollständigen Umsetzung. Anschließend wurde die Reaktionslösung im Vakuum eingeengt und der Rückstand mit Wasser verrührt. Der anfallende Niederschlag wurde filtriert, mit Wasser gewaschen und im Vakuum getrocknet.

### Allgemeine Methode 4A: N-Alkylierung von 2-Pyridinon-Derivaten mit den entsprechenden 2-Brom- oder 2-Chlorpropansäureesterderivaten in Gegenwart von Kaliumcarbonat

Eine Lösung von 1.0 eq. des entsprechenden 2-Pyridinon-Derivates in Dimethylformamid (5-10 ml/mmol) wurde unter Argon bei RT mit 1.2 eq. des entsprechenden 2-Brom- oder 2-Chlorpropansäureesterderivates und 1.5 eq. Kaliumcarbonat versetzt und bei 100°C gerührt. Nach Entfernen des Dimethylformamids und Zugabe von Wasser/Essigsäureethylester und Phasentrennung wurde die organische Phase mit Wasser und mit gesättigter, wässriger Natriumchlorid-Lösung gewaschen, getrocknet (Natrium- oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 5A: Amid-Kupplung mit HATU/DIEA

Eine Lösung der entsprechenden Carbonsäure (1.0 eq.) in Dimethylformamid (7-15 ml/mmol) wurde unter Argon bei RT mit dem Amin (1.1 eq.), mit *N,N-*Diisopropylethylamin (2.2 eq.) und einer Lösung von HATU (1.2 eq.) in etwas Dimethylformamid versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6A: Verseifung eines tert.-Butylesters oder eines Boc-geschützten Amins mittels TFA

Eine Lösung von 1.0 eq. des entsprechenden *tert*.-Butylesterderivates in Dichlormethan (ca. 5-10 ml/mmol) wurde bei RT mit 20 eq. TFA versetzt und bei RT für 1 bis 8 h gerührt. Anschließend wurde das Reaktionsgemisch im Vakuum eingeengt, der Rückstand mehrmals mit Dichlormethan und Toluol koevaporiert und im Vakuum getrocknet. Das Rohprodukt wurde gegebenenfalls anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 6B: Verseifung eines Methyl-/Ethyl- oder Benzylesters mit Lithiumhydroxid

Eine Lösung von 1.0 eq. des entsprechenden Methyl- oder Ethylesters in Tetrahydrofuran/Wasser (3:1, ca. 7-15 ml/mmol) wurde bei RT mit Lithiumhydroxid (2-4 eq.) versetzt. Das Reaktionsgemisch wurde bei RT bis 60°C gerührt und anschließend mit wässriger Salzsäure (1N) auf pH 1 gestellt. Nach Zugabe von Wasser/Essigsäureethylester und Phasentrennung wurde die wässrige Phase dreimal mit Essigsäureethylester extrahiert. Die vereinigten, organischen Phasen wurden getrocknet (Natriumsulfat oder Magnesiumsulfat), filtriert und im Vakuum eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Allgemeine Methode 7A: Darstellung von Triflaten

Eine Lösung des entsprechenden Alkohols (1 eq.) wurde in Dichlormethan (0.1M) vorgelegt und bei -20°C nacheinander mit Lutidin (1.1-1.5 eq.) oder Triethylamin (1.1-1.5 eq.) und mit Trifluormethansulfonsäureanhydrid (1.05-1.5 eq.) versetzt. Das Reaktionsgemisch wurde für 1 h bei -20°C nachgerührt und anschließend mit der dreifachen Menge (bezogen aufs Reaktionsvolumen) Methyl-*tert*.-butylether verdünnt. Die organische Phase wurde dreimal mit einer 3:1-Mischung aus gesättigter, wässriger Natriumchlorid-Lösung/IN Salzsäure und abschließend mit gesättigter, wässriger Natriumhydrogencarbonat-Lösung gewaschen, getrocknet (Natrium- oder Magnesiumsulfat), filtriert und das Lösungsmittel unter reduziertem Druck entfernt. Das Rohprodukt wurde ohne weitere Aufreinigung im nächsten Schritt eingesetzt.

### Allgemeine Methode 8A: Alkylierung von Essigsäureestern mit Triflaten

Eine Lösung des entsprechenden Essigsäureesters (1 eq.) in Tetrahydrofuran (0.1-0.2M) wurde unter Argon bei -78°C tropfenweise mit Bis-(trimethylsilyl)-lithiumamid (1.0M in THF, 1.1-1.3 eq.) versetzt und 15 min gerührt. Anschließend wurde das entsprechende Alkyltriflat (1.5-2.0 eq.) als Reinsubstanz oder Lösung in THF hinzugegeben. Die resultierende Reaktionsmischung wurde 15 min bei -78°C und 1 h bei RT nachgerührt. Das Reaktionsgemisch wurde mit gesättigter, wässriger Ammoniumchlorid-Lösung versetzt. Nach Phasentrennung wurde die wässrige Phase mit Essigsäureethylester extrahiert. Die vereinigten, organischen Phasen wurden getrocknet (Natriumsulfat oder Magnesiumsulfat), filtriert und unter reduziertem Druck eingeengt. Das Rohprodukt wurde anschließend entweder mittels Normalphasen-Chromatographie (Cyclohexan-Essigsäureethylester-Gemische oder Dichlormethan-Methanol-Gemische) oder präparativer RP-HPLC (Wasser-Acetonitril-Gradient oder Wasser-Methanol-Gradient) aufgereinigt.

### Beispiel 1.1A

### 2,5-Dimethoxypyridin-4-ylboronsäure

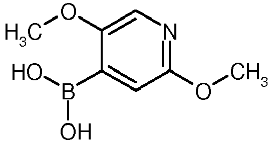

Nach der allgemeinen Methode 1A wurden 11.53 g (82.9 mmol) 2,5-Dimethoxypyridin umgesetzt. Nach Ansäuern der wässrigen Phase fiel das gewünschte Produkt als Niederschlag aus. Ausbeute: 9.53 g (61% d. Th.)
LC/MS [Methode 1]: Rₜ = 0.47 min; MS (ESIpos): m/z = 184 (M+H)⁺.

### Beispiel 1.1B

### 4-Chlor-2-(2,5-dimethoxypyridin-4-yl)benzonitril

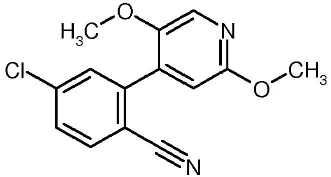

Nach der allgemeinen Methode 2A wurden 7.87 g (95% Reinheit, 40.86 mmol) 2,5-Dimethoxypyridin-4-ylboronsäure mit 8.85 g (40.86 mmol) 2-Brom-4-chlorbenzonitril in Gegenwart von [1,1-Bis-(diphenylphosphino)-ferrocen]-palladium(II)chlorid-dichlormethan-Monoaddukt umgesetzt. Ausbeute: 6.23 g (92% Reinheit, 51 % d. Th.)
LC/MS [Methode 1]: Rₜ = 1.08 min; MS (ESIpos): m/z = 275 (M+H)⁺.

### Beispiel 1.1C

### 4-Chlor-2-(5-methoxy-2-oxo-1,2-dihydropyridin-4-yl)benzonitril

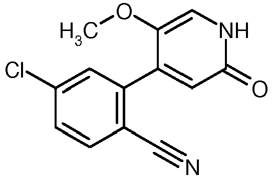

Nach der allgemeinen Methode 3A wurden 7.23 g (92% Reinheit, 24.21 mmol) 4-Chlor-2-(2,5-dimethoxypyridin-4-yl)benzonitril mit Pyridinium-Hydrochlorid umgesetzt. Ausbeute: 6.66 g (91% Reinheit, 96% d. Th.)
LC/MS [Methode 1]: Rₜ = 0.76 min; MS (ESIpos): m/z = 261 (M+H)⁺,
¹H-NMR (400 MHz, DMSO-d₆): δ [ppm] = 11.45 (br. s, 1H), 7.98 (d, 1H), 7.75-7.67 (m, 2H), 7.29 (br. s, 1H), 6.43 (s, 1H), 3.64 (s, 3H).

### Beispiel 1.1D

### [4-(5-Chlor-2-cyanophenyl)-5-methoxy-2-oxopyridin-1(2H)-yl]essigsäure-tert.-butylester

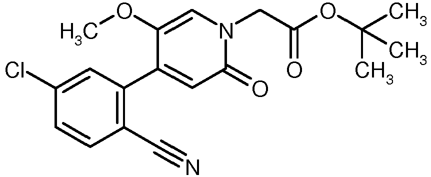

Nach der allgemeinen Methode 4B wurden 516 mg (91% Reinheit, 1.8 mmol) 4-Chlor-2-(5-methoxy-2-oxo-1,2-dihydropyridin-4-yl)benzonitril mit 1.2 eq. Bromessigsäure-*tert*.-butylester bei 100°C umgesetzt. Ausbeute: 464 mg (68% d. Th.)
LC/MS [Methode 1]: Rₜ = 1.00 min; MS (ESIpos): m/z = 375 (M+H)⁺.

### Beispiel 1.1E

### 2-[4-(5-Chlor-2-cyanophenyl)-5-methoxy-2-oxopyridin-1(2H)-yl]butansäure-tert.-butylester (Racemat)

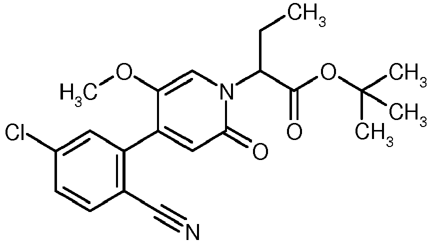

Eine Lösung von 5.0 g (13.3 mmol) [4-(5-Chlor-2-cyanophenyl)-5-methoxy-2-oxopyridin-1(2*H*)-yl]essigsäure-*tert*.-butylester in 100 ml Tetrahydrofuran wurde unter Argon bei -78°C tropfenweise mit 14.0 ml (1.0M in THF, 14.0 mmol, 1.05 eq.) Bis-(trimethylsilyl)-lithiumamid versetzt und 15 min bei -78°C gerührt. Anschließend wurden tropfenweise 2.6 g (14.7 mmol, 1.1 eq.) Ethyltrifluormethansulfonat als Reinsubstanz hinzugegeben. Das Kältebad wurde entfernt und die Reaktionsmischung 1 h bei RT nachgerührt. Das Reaktionsgemisch wurde auf 0°C gekühlt und mit gesättigter, wässriger Ammoniumchlorid-Lösung versetzt. Nach Phasentrennung wurde die wässrige Phase zweimal mit Methyl-*tert*.-butylether extrahiert. Die vereinigten, organischen Phasen wurden getrocknet (Natriumsulfat), filtriert und im Vakuum eingeengt. Das Rohprodukt wurde anschließend mittels Flash-Chromatographie (340 g Kieselgel, Eluent: Cyclohexan-Essigsäureethylester-Gemische 8:1, 4:1) aufgereinigt. Die produkthaltigen Fraktionen wurden vereinigt und im Vakuum eingeengt. Der Rückstand wurde in der Wärme in Methyl-*tert*.-butylether gelöst, die Lösung offen stehen gelassen, wobei nach 10 min das Gemisch fast vollständig durchkristallisierte. Der Kristallisat wurde filtriert und zweimal mit Methyl-*tert*.-butylether gewaschen. Die vereinigten Filtrate wurden im Vakuum eingeengt und der Rückstand wie beschrieben nochmals auskristallisiert. Beide Kristallisate wurden vereinigt und im Vakuum getrocknet. Ausbeute: 4.2 g (78% d. Th.)
LC/MS [Methode 1]: Rₜ = 1.05 min; MS (ESIpos): m/z = 403 (M+H)⁺,
¹H-NMR (400 MHz, DMSO-d₆): δ [ppm] = 7.99 (d, 1H), 7.77-7.70 (m, 2H), 7.36 (s, 1H), 6.50 (s, 1H), 5.03 (dd, 1H), 3.64 (s, 3H), 2.19-2.06 (m, 2H), 1.40 (s, 9H), 0.85 (t, 3H).

### Beispiel 1.1F

### 2-[4-(5-Chlor-2-cyanophenyl)-5-methoxy-2-oxopyridin-1(2H)-yl]butansäure (Racemat)

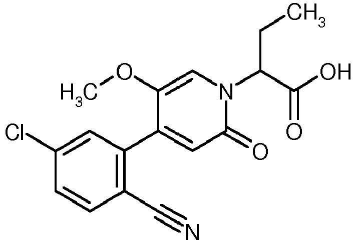

Eine Lösung von 4.1 g (10.2 mmol) 2-[4-(5-Chlor-2-cyanophenyl)-5-methoxy-2-oxopyridin-1(2*H*)-yl]butansäure-*tert*.-butylester (Racemat) in 40 ml Dichlormethan wurde unter Argon bei RT mit 7.8 ml (101.8 mmol, 10 eq.) Trifluoressigsäure versetzt, 1 h bei RT gerührt, mit weiteren 7.8 ml (101.8 mmol, 10 eq.) Trifluoressigsäure versetzt, 1 h bei RT gerührt, mit weiteren 7.8 ml (101.8 mmol, 10 eq.) Trifluoressigsäure versetzt und 1 h bei RT gerührt. Nach vollständiger Umsetzung wurde das Reaktionsgemisch im Vakuum eingeengt, der Rückstand jeweils dreimal mit Dichlormethan und einmal mit Toluol koevaporiert und im Vakuum getrocknet. Der Rückstand wurde in 100 ml Essigsäureethylester aufgenommen und mehrfach mit einer stark verdünnten, wässrigen Natriumhydrogencarbonat-Lösung gewaschen (wobei der pH-Wert des Waschwassers pH 3-4 nicht überschreiten sollte, da das Produkt ansonsten gut in Wasser löslich ist). Die organische Phase wurde anschließend getrocknet (Natriumsulfat), filtriert und im Vakuum eingeengt. Der Rückstand wurde in Methyl-*tert*.-butylether verrührt, filtriert, zweimal mit Methyl-*tert*.-butylether gewaschen und im Vakuum getrocknet. Ausbeute: 2.9 g (83% d. Th.)
LC/MS [Methode 1]: Rₜ = 0.81 min; MS (ESIpos): m/z = 347 (M+H)⁺,
¹H-NMR (400 MHz, DMSO-d₆): δ [ppm] = 12.97 (s, 1H), 7.99 (d, 1H), 7.77-7.70 (m, 2H), 7.41 (s, 1H), 6.49 (s, 1H), 5.09 (dd, 1H), 3.64 (s, 3H), 2.21-2.09 (m, 2H), 0.84 (t, 3H).

### Beispiel 1.1G

### 4-({2-[4-(5-Chlor-2-cyanophenyl)-5-methoxy-2-oxopyridin-1(2H)-yl]batanoyl}amino)-benzoesäureethylester (Racemat)

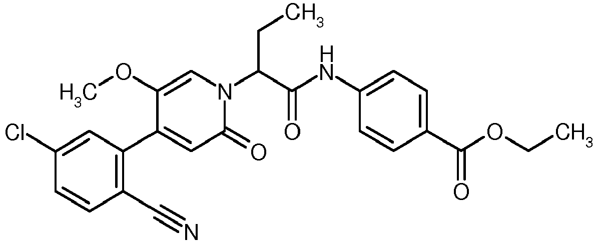

Eine Lösung von 1.0 g (2.9 mmol) 2-[4-(5-Chlor-2-cyanophenyl)-5-methoxy-2-oxopyridin-1(2*H*)-yl]butansäure (Racemat) in 10 ml Dimethylformamid wurde unter Argon bei RT mit 476 mg (2.9 mmol, 1.0 eq.) 4-Amino-benzoesäureethylester, mit 41 mg (0.29 mmol, 0.1 eq.) Oxima und anschließend tropfenweise mit 452 µl (2.9 mmol, 1.0 eq.) *N,N*'-Diisopropylcarbodiimid (DIC) versetzt. Das Reaktionsgemisch wurde 3 h bei 40-45°C gerührt, auf RT abgekühlt, mit Methyl-*tert*.-butylether und Wasser versetzt und 10 min kräftig gerührt, wobei ein Niederschlag ausfiel. Der Niederschlag wurde filtriert, zweimal mit Wasser und Methyl-*tert*.-butylether gewaschen und im Vakuum getrocknet (Niederschlag 1). Nach Phasentrennung der vereinigten Filtrate wurde die wässrige Phase einmal mit Methyl-*tert*.-butylether extrahiert. Die vereinigten, organischen Phasen wurden einmal mit gesättigter, wässriger Natriumchlorid-Lösung gewaschen, getrocknet (Natriumsulfat), filtriert und im Vakuum eingeengt. Der Rückstand wurde in Methyl-*tert*.-butylether verrührt, filtriert, zweimal mit Methyl-*tert*.-butylether gewaschen und im Vakuum getrocknet. Dieser Niederschlag wurde 10 min in wässriger Salzsäure (1N) verrührt, filtriert, zweimal mit Wasser und einmal mit Acetonitril gewaschen und im Vakuum getrocknet (Niederschlag 2). Ausbeute: Niederschlag 1: 1.12 g (79% d. Th.), Niederschlag 2: 127 mg (enthält laut ¹H-NMR noch 1,3-Diisopropylharnstoff)
LC/MS [Methode 1]: Rₜ = 1.11 min; MS (ESIneg): m/z = 492 (M-H)⁻,
¹H-NMR (400 MHz, DMSO-d₆): δ [ppm] = 10.82 (s, 1H), 8.00 (d, 1H), 7.93 (d, 2H), 7.81-7.70 (m, 4H), 7.49 (s, 1H), 6.54 (s, 1H), 5.63 (dd, 1H), 4.29 (q, 2H), 3.69 (s, 3H), 2.26-2.11 (m, 2H), 1.31 (t, 3H), 0.91 (t, 3H).

### Beispiel 1.1H

### 4-({2-[4-(5-Chlor-2-cyanophenyl)-5-methoxy-2-oxopyridin-1(2H)-yl]batanoyl}amino)benzoesäure (Racemat)

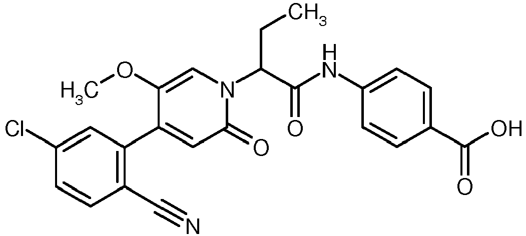

Eine Suspension von 200 mg (0.41 mmol) 4-({2-[4-(5-Chlor-2-cyanophenyl)-5-methoxy-2-oxopyridin-1(2*H*)-yl]butanoyl}amino)benzoesäureethylester (Racemat) in 2.0 ml Ethanol wurde bei RT mit 0.2 ml Wasser und 97 mg (1.22 mmol, 3.0 eq.) Natriumhydroxid versetzt. Das Reaktionsgemisch wurde 1 h bei RT und 6 h bei 60°C Ölbadtemperatur gerührt, über Nacht bei RT stehen gelassen und anschließend auf wässriger Salzsäure (1N) gegeben. Nach Verdünnen mit Wasser fiel ein Niederschlag aus, der filtriert, zweimal mit Wasser gewaschen und im Vakuum getrocknet wurde. Der Niederschlag wurde anschließend mittels Flash-Chromatographie (25 g Kieselgel, Eluent: Dichormethan → Dichlormethan/Methanol 50:1) aufgereinigt. Ausbeute: 144 mg (38% d. Th. bezogen auf 0.82 mmol, da zwei Rohprodukte der angegebenen Größe zusammen aufgereinigt wurden)
LC/MS [Methode 1]: Rₜ = 0.92 min; MS (ESIneg): m/z = 464 (M-H)⁻,
¹H-NMR (400 MHz, DMSO-d₆): δ [ppm] = 12.74 (s, 1H), 10.80 (s, 1H), 8.00 (d, 1H), 7.92 (d, 2H), 7.79-7.70 (m, 4H), 7.50 (s, 1H), 6.54 (s, 1H), 5.65 (dd, 1H), 3.69 (s, 3H), 2.26-2.11 (m, 2H), 0.91 (t, 3H).

### Beispiel 1.1I

### 4-({2-[4-(5-Chlor-2-cyanophenyl)-5-methoxy-2-oxopyridin-1(2H)-yl]batanoyl}amino)benzoesäure (Enantiomer 1)

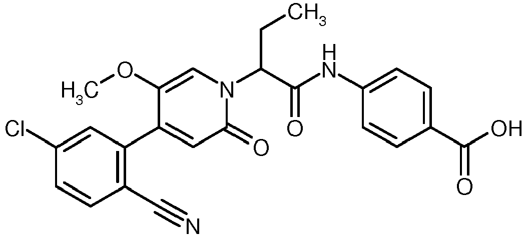

Enantiomerentrennung von 433 mg des Racemates aus Beispiel 1.1H ergab 196 mg der Titelverbindung Beispiel 1.1I (Enantiomer 1): Chirale HPLC: Rₜ = 5.22 min; 99% ee.
Trennmethode: Säule: Daicel Chiralpak IF 5 µm 250 mm x 20 mm; Eluent: 50% iso-Hexan, 50% Ethanol + 0.2% Essigsäure; Temperatur: 40°C; Fluss: 15 ml/min; UV-Detektion: 220 nm.
Analytik: Säule: Daicel Chiralpak AZ-H 5 µm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
¹H-NMR (400 MHz, DMSO-d₆): δ [ppm] = 12.77 (s, 1H), 10.81 (s, 1H), 8.00 (d, 1H), 7.91 (d, 2H), 7.79-7.71 (m, 4H), 7.50 (s, 1H), 6.54 (s, 1H), 5.64 (dd, 1H), 3.69 (s, 3H), 2.26-2.11 (m, 2H), 0.91 (t, 3H).

### Beispiel 1.21

### 4-({2-[4-(5-Chlor-2-cyanophenyl)-5-methoxy-2-oxopyridin-1(2H)-yl]butanoyl}amino)benzoesäure (Enantiomer 2)

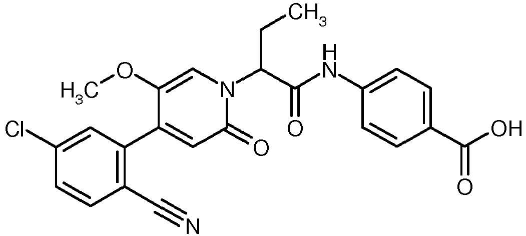

Enantiomerentrennung von 433 mg des Racemates aus Beispiel 1.1H ergab 201 mg der Titelverbindung Beispiel 1.2I (Enantiomer 2): Chirale HPLC: Rₜ = 8.19 min; 99% ee.
Trennmethode: Säule: Daicel Chiralpak IF 5 µm 250 mm x 20 mm; Eluent: 50% iso-Hexan, 50% Ethanol + 0.2% Essigsäure; Temperatur: 40°C; Fluss: 15 ml/min; UV-Detektion: 220 nm.
Analytik: Säule: Daicel Chiralpak AZ-H 5 µm 250 mm x 4.6 mm; Eluent: 50% iso-Hexan, 50% Ethanol + 0.2% TFA + 1% Wasser; Temperatur: 40°C; Fluss: 1 ml/min; UV-Detektion: 220 nm.
¹H-NMR (400 MHz, DMSO-d₆): δ [ppm] = 12.77 (s, 1H), 10.81 (s, 1H), 8.00 (d, 1H), 7.91 (d, 2H), 7.79-7.71 (m, 4H), 7.50 (s, 1H), 6.54 (s, 1H), 5.64 (dd, 1H), 3.69 (s, 3H), 2.26-2.11 (m, 2H), 0.91 (t, 3H).

### Beispiel 2.1A

### 2-[(Benzyloxy)methyl]tetrahydro-2H-pyran (Racemat)

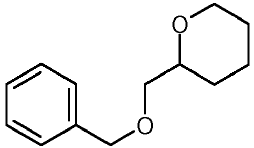

Zu einer Suspension von 9.47 g (237 mmol, 60% in Mineralöl) Natriumhydrid in 500 ml THF wurde bei 0°C eine Lösung von 25.0 g (215 mmol) Tetrahydro-2*H*-pyran-2-ylmethanol (Racemat) in 500 ml THF langsam zugetropft und nach vollständiger Zugabe 30 min bei 0°C nachgerührt. Anschließend wurden 25.7 ml (215 mmol) Benzylbromid zugegeben und für 30 min bei 0°C und 1 h bei Raumtemperatur nachgerührt. Die Reaktion wurde durch Zugabe von 200 ml gesättigter, wässriger Ammoniumchlorid-Lösung beendet und die Phasen getrennt. Die wässrige Phase wurde zweimal mit 200 ml Methyl-*tert*.-butylether extrahiert. Die vereinigten organischen Phasen wurden über Magnesiumsulfat getrocknet, filtriert und das Lösungsmittel unter reduziertem Druck entfernt. Das Rohprodukt wurde säulenchromatographisch gereinigt (Essigsäureethylester-Cyclohexan-Gradient, 340 g Silica Kartusche, Fluss: 100 ml/min) und die Titelverbindung erhalten. Ausbeute: 41.9 g (94% d. Th.)
LC/MS [Methode 3]: Rₜ = 2.18 min; MS (ESIpos): m/z = 207 (M+H)⁺,
¹H-NMR (400 MHz, DMSO-d₆): δ [ppm] = 7.37-7.25 (m, 5H), 4.47 (s, 2H), 3.87-3.81 (m, 1H), 3.47-3.28 (m, 4H), 1.80-1.72 (m, 1H), 1.58-1.37 (m, 4H), 1.25-1.13 (m, 1H).

### Beispiel 2.1B

### (R)-2-[(Benzyloxy)methyl]tetrahydro-2H-pyran

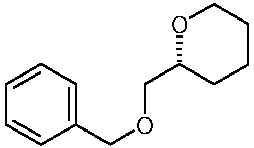

Enantiomerentrennung von 41.9 g des Racemates aus Beispiel 2.1A ergab 16.7 g der Titelverbindung Beispiel 2.1B (Enantiomer 1): Chirale HPLC: Rₜ = 5.28 min; 99% ee, 93% Reinheit.
Drehwert: [α]₅₈₉^{20.0} = +14.9° (c 0.43 g/100 cm³, Chloroform)
Trennmethode: Säule: OD-H 5 µm 250 mm x 20 mm; Eluent: 95% iso-Hexan, 5% 2-Propanol; Temperatur: 25°C; Fluss: 25 ml/min; UV-Detektion: 210 nm.
Analytik: Säule: OD-H 5 µm 250 mm x 4.6 mm; Eluent: 95% iso-Hexan, 5% 2-Propanol; Fluss: 1 ml/min; UV-Detektion: 220 nm.

### Beispiel 2.2B

### (S)-2-[(Benzyloxy)methyl]tetrahydro-2H-pyran

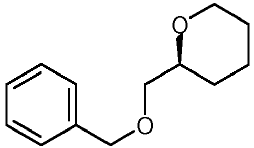

Enantiomerentrennung von 41.9 g des Racemates aus Beispiel 2.1A ergab 17.0 g der Titelverbindung Beispiel 2.2B (Enantiomer 2): Chirale HPLC: Rₜ = 7.36 min; 96% ee, 96% Reinheit.
Drehwert: [α]₅₈₉^{20.0} = -13.9° (c 0.61 g/100 cm³, Chloroform)
Trennmethode: Säule: OD-H 5 µm 250 mm x 20 mm; Eluent: 95% iso-Hexan, 5% 2-Propanol; Temperatur: 25°C; Fluss: 25 ml/min; UV-Detektion: 210 nm.
Analytik: Säule: OD-H 5 µm 250 mm x 4.6 mm; Eluent: 95% iso-Hexan, 5% 2-Propanol; Fluss: 1 ml/min; UV-Detektion: 220 nm.

### Beispiel 2.1C

### (2S)-Tetrahydro-2H-pyran-2-ylmethanol

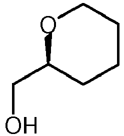

Zu einer Lösung 17.0 g (82.4 mmol) (*S*)-2-[(Benzyloxy)methyl]tetrahydro-2*H*-pyran (96% ee, 96% Reinheit) in 120 ml Ethanol wurde 3.51 g (3.30 mmol) Palladium auf Kohle (10%ig) gegeben und über Nacht bei Raumtemperatur und Normaldruck hydriert. Anschließend wurde noch einmal 1.75 g (1.65 mmol) Palladium auf Kohle (10%ig) hinzugegeben und für weitere 72 h bei Raumtemperatur hydriert. Anschließend wurde die Reaktionsmischung über Celite filtriert und das Filtrat eingeengt. Der Rückstand wurde chromatographisch (Silica, Dichlormethan/Methanol-Gradient) gereinigt und die Produktfraktionen bei < 25°C und > 50 mbar vom Lösungsmittel befreit. Ausbeute: 8.23 g (86% d. Th.)
Drehwert: [α]₅₈₉^{20.0} = + 9.1° (c 0.36 g/100 cm³, Chloroform), vgl. A. Aponick, B. Biannic, Org. Lett. 2011, 13, 1330-1333.
GC/MS [Methode 7]: Rₜ = 1.82 min; MS: m/z = 116 (M)⁺,
¹H-NMR (400 MHz, DMSO-d₆): δ [ppm] = 4.51 (t, 1H), 3.87-3.81 (m, 1H), 3.37-3.18 (m, 4H), 1.80-1.71 (m, 1H), 1.59-1.50 (m, 1H), 1.49-1.36 (m, 3H), 1.19-1.05 (m, 1H).

### Beispiel 2.1D

### (2S)-Tetrahydro-2H-pyran-2-ylmethyltrifluormethansulfonat

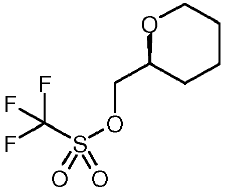

Nach der allgemeinen Methode 7A wurden 330 mg (2.84 mmol) (2*S*)-Tetrahydro-2*H*-pyran-2-ylmethanol mit 0.57 ml (3.41 mmol, 1.2 eq.) Trifluormethansulfonsäureanhydrid in Gegenwart von 0.48 ml (3.41 mmol, 1.2 eq.) Triethylamin umgesetzt. Das Rohprodukt wurde ohne weitere Reinigung in der nächsten Stufe umgesetzt.
¹H-NMR (400 MHz, DMSO-d₆): δ [ppm] = 4.32 (dd, 1H), 4.18 (dd, 1H), 4.00-3.92 (m, 1H), 3.60-3.52 (m, 1H), 3.48-3.39 (m, 1H), 1.85-1.74 (m, 1H), 1.56-1.41 (m, 4H), 1.28-1.14 (m, 1H).

### Beispiel 2.1E

### 2-[4-(5-Chlor-2-cyanophenyl)-5-methoxy-2-oxopyridin-1(2H)-yl]-3-[(2S)-tetrahydro-2H-pyran-2-yl]propansäure-tert.-butylester (Gemisch enantiomerenreiner Diastereomere)

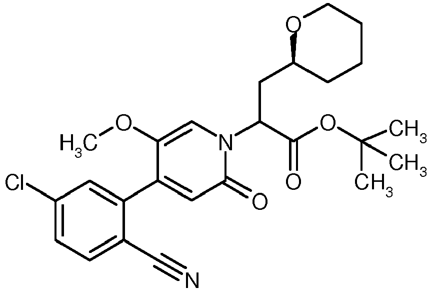

Gemäß allgemeiner Methode 8A wurden 4.10 g (10.9 mmol) [4-(5-Chlor-2-cyanophenyl)-5-methoxy-2-oxopyridin-1(2*H*)-yl]essigsäure-*tert*.-butylester, 4.07 g (16.4 mmol) (2*S*)-Tetrahydro-2*H*-pyran-2-ylmethyltrifluormethansulfonat und 12.0 ml (12.0 mmol) Bis-(trimethylsilyl)-lithiumamid (1M in THF) in 90 ml THF zur Reaktion gebracht. Nach wässriger Aufarbeitung wurde das Rohprodukt mittels Flash-Chromatographie (340 g Silica Kartusche, Fluss: 100 ml/min, Cyclohexan-Essigsäureethylester-Gemisch) aufgereinigt. Ausbeute: 4.2 g (81% d. Th.)
LC/MS [Methode 1]: Rₜ = 1.15 min; MS (ESIpos): m/z = 473 (M+H)⁺.

### Beispiel 2.1F

### 2-[4-(5-Chlor-2-cyanophenyl)-5-methoxy-2-oxopyridin-1(2H)-yl]-3-[(2S)-tetrahydro-2H-pyran-2-yl]propansäure (Gemisch enantiomerenreiner Diastereomere)

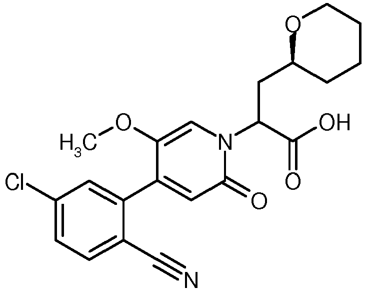

Gemäß allgemeiner Methode 6A wurden 9.8 g (20.7 mmol) 2-[4-(5-Chlor-2-cyanophenyl)-5-methoxy-2-oxopyridin-1(2*H*)-yl]-3-[(2*S*)-tetrahydro-2*H*-pyran-2-yl]propansäure-*tert*.-butylester (Gemisch enantiomerenreiner Diastereomere) in 245 ml Dichlormethan mit 59.9 ml (777 mmol) TFA umgesetzt. Ausbeute: 8.7 g (73% Reinheit, 74% der Th.)
LC/MS [Methode 1]: Rₜ = 0.92 min; MS (ESIpos): m/z = 417 (M+H)⁺.

### Beispiel 2.1G

### 4-({2-[4-(5-Chlor-2-cyanophenyl)-5-methoxy-2-oxopyridin-1(2H)-yl]-3-[(2S)-tetrahydro-2H-pyran-2-yl]propanoyl}amino)benzoesäureethylester (Gemisch enantiomerenreiner Diastereomere)

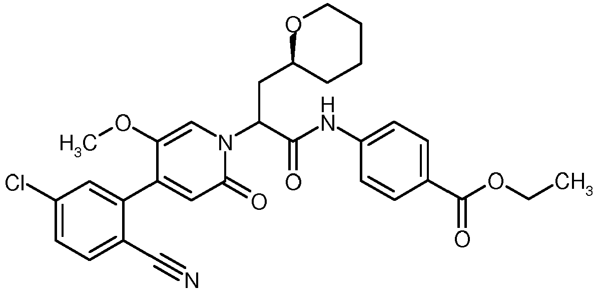

Eine Lösung von 10 g (24 mmol) 2-[4-(5-Chlor-2-cyanophenyl)-5-methoxy-2-oxopyridin-1(2*H*)-yl]-3-[(2*S*)-tetrahydro-2*H*-pyran-2-yl]propansäure (Gemisch enantiomerenreiner Diastereomere) und 4.4 g (26 mmol, 1.1 eq.) 4-Aminobenzoesäureethylester in 100 ml Dimethylformamid wurde bei RT mit 10.4 ml (60 mmol, 2.5 eq.) *N*,*N*-Diisopropylethylamin und tropfenweise mit 23 g (36 mmol, 1.5 eq.) Propylphosphonsäureanhydrid (T3P, 50%ig in Essigsäureethylester) versetzt und 3 h bei 80°C gerührt. Nach Abkühlen auf RT wurde die Reaktionsmischung auf ein Gemisch aus 500 ml 10%iger, wässriger Natriumchlorid-Lösung und 150 ml Essigsäureethylester gegeben. Nach Phasentrennung wurde die wässrige Phase mit 150 ml Essigsäureethylester extrahiert. Die vereinigten, organischen Phasen wurden zweimal mit jeweils 100 ml 10%iger, wässriger Natriumchlorid-Lösung gewaschen, getrocknet und im Vakuum eingeengt. Das Rohprodukt wurde mittels Flash-Chromatographie (Kieselgel 40-63 µm, Petrolether-Essigsäureethylester 1:1) aufgereinigt. Ausbeute: 7.7 g (57% d. Th.)
LC/MS [Methode 2]: Rₜ = 3.75 min/3.81 min; MS (ESIpos): m/z = 564 (M+H)⁺.

### Beispiel 2.1H

### 4-({2-[4-(5-Chlor-2-cyanophenyl)-5-methoxy-2-oxopyridin-1(2H)-yl]-3-[(2S)-tetrahydro-2H-pyran-2-yl]propanoyl}amino)benzoesäure (Gemisch enantiomerenreiner Diastereomere)

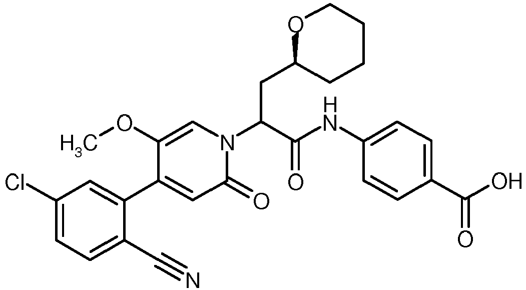

Eine Suspension von 7.6 g (13.5 mmol) 4-({2-[4-(5-Chlor-2-cyanophenyl)-5-methoxy-2-oxopyridin-1(2*H*)-yl]-3-[(2*S*)-tetrahydro-2*H*-pyran-2-yl]propanoyl}amino)benzoesäureethylester (Gemisch enantiomerenreiner Diastereomere) in 152 ml Methanol und 38 ml Wasser wurde mit 8.8 g (26.9 mmol, 2 eq.) Cäsiumcarbonat versetzt und 4 h bei 60°C gerührt. Methanol wurde im Vakuum entfernt. Das übrige Reaktionsgemisch wurde mit 100 ml Wasser verdünnt und mit 100 ml Essigsäureethylester gewaschen. Die wässrige Phase wurde mit 100 ml Essigsäureethylester überschichtet und unter Rühren mit halbkonzentrierter Salzsäure auf pH 2 eingestellt. Nach Abtrennen der organischen Phase wurde die wässrige Phase erneut mit 100 ml Essigsäureethylester extrahiert. Die vereinigten, organischen Phasen wurden getrocknet und im Vakuum eingeengt. Das Rohprodukt wurde mittels Flash-Chromatographie (Kieselgel 40-63 µm, Dichlormethan-Methanol 9:1) aufgereinigt. Ausbeute: 6.3 g (87% d. Th.)
LC/MS [Methode 1]: Rₜ = 0.99 min; MS (ESIpos): m/z = 536 (M+H)⁺,
¹H-NMR (400 MHz, DMSO-d₆): δ [ppm] = 12.7 (br. s, 1H), 10.74/10.66 (2x s, 1H), 8.02-7.97 (m, 1H), 7.94-7.86 (m, 2H), 7.81-7.70 (m, 4H), 7.53/7.49 (2x s, 1H), 6.52/6.51 (2x s, 1H), 5.83/5.75 (t/dd, 1H), 3.90-3.78 (m, 1H), 3.69/3.68 (2x s, 3H), 3.29-3.15 (m, 1H), 3.14-3.05 (m, 1H), 2.43-2.10 (m, 2H), 1.80-1.70 (m, 1H), 1.68-1.55 (m, 1H), 1.48-1.34 (m, 3H), 1.31-1.21 (m, 1H).

### Beispiel 2.11

### 4-({2-[4-(5-Chlor-2-cyanophenyl)-5-methoxy-2-oxopyridin-1(2H)-yl]-3-[(2S)-tetrahydro-2H-pyran-2-yl]propanoyl}amino)benzoesäure (enantiomerenreines Diastereomer 1)

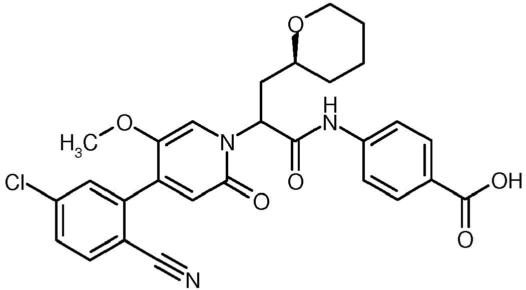

Diastereomerentrennung eines Gemisches aus Beispiel 2.1H ergab Titelverbindung Beispiel 2.1I (enantiomerenreines Diastereomer 1): Chirale HPLC: Rₜ = 3.5 min; 99% ee.
Trennmethode (SFC): Säule: AD-H 20 µm 360 mm x 50 mm; Eluent: 75% Kohlendioxid/25% 2-Propanol → 60% Kohlendioxid/40% 2-Propanol; Temperatur: 20°C; Fluss: 400 ml/min; Druck: 80 bar; UV-Detektion: 220 nm.
Analytik (SFC): Säule: AD-H 5 µm 250 mm x 4.6 mm; Eluent: 70% Kohlendioxid, 30% 2-Propanol; Temperatur: 40°C; Fluss: 3 ml/min; UV-Detektion: 220 nm.
¹H-NMR (400 MHz, DMSO-d₆): δ [ppm] = 12.7 (s, 1H), 10.7 (s, 1H), 8.00 (d, 1H), 7.90 (d, 2H), 7.77-7.72 (m, 4H), 7.49 (s, 1H), 6.51 (s, 1H), 5.78-5.71 (m, 1H), 3.84-3.79 (m, 1H), 3.69 (s, 3H), 3.23-3.15 (m, 1H), 3.13-3.05 (m, 1H), 2.42-2.32 (m, 1H), 2.26-2.18 (m, 1H), 1.78-1.71 (m, 1H), 1.62-1.56 (m, 1H), 1.46-1.37 (m, 3H), 1.30-1.20 (m, 1H).

### Beispiel 2.21

### 4-({2-[4-(5-Chlor-2-cyanophenyl)-5-methoxy-2-oxopyridin-1(2H)-yl]-3-[(2S)-tetrahydro-2H-pyran-2-yl]propanoyl}amino)benzoesäure (enantiomerenreines Diastereomer 2)

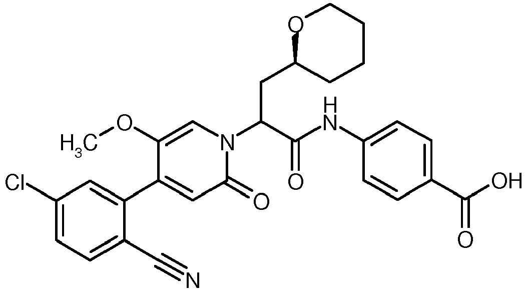

Diastereomerentrennung eines Gemisches aus Beispiel 2.1H ergab Titelverbindung Beispiel 2.2I (enantiomerenreines Diastereomer 2): Chirale HPLC: Rₜ = 6.2 min; 99% ee.
Trennmethode (SFC): Säule: AD-H 20 µm 360 mm x 50 mm; Eluent: 75% Kohlendioxid/25% 2-Propanol → 60% Kohlendioxid/40% 2-Propanol; Temperatur: 20°C; Fluss: 400 ml/min; Druck: 80 bar; UV-Detektion: 220 nm.
Analytik (SFC): Säule: AD-H 5 µm 250 mm x 4.6 mm; Eluent: 70% Kohlendioxid, 30% 2-Propanol; Temperatur: 40°C; Fluss: 3 ml/min; UV-Detektion: 220 nm.
¹H-NMR (400 MHz, DMSO-d₆): δ [ppm] = 12.8 (s, 1H), 10.7 (s, 1H), 7.99 (d, 1H), 7.90 (d, 2H), 7.77 (d, 2H), 7.74-7.71 (m, 2H), 7.52 (s, 1H), 6.52 (s, 1H), 5.82 (t, 1H), 3.90-3.84 (m, 1H), 3.68 (s, 3H), 3.28-3.22 (m, 2H), 2.34-2.27 (m, 1H), 2.19-2.10 (m, 1H), 1.78-1.73 (m, 1H), 1.67-1.60 (m, 1H), 1.47-1.36 (m, 3H), 1.33-1.23 (m, 1H).

### Beispiel 3.1A

### (2R,3S)-4-(Benzyloxy)-3-{[(benzyloxy)carbonyl]amino}-4-oxobutan-2-yl-4-({2-[4-(5-chlor-2-cyanophenyl)-5-methoxy-2-oxopyridin-1(2H)-yl]-3-[(2S)-tetrahydro-2H-pyran-2-yl]propanoyl}-amino)benzoat (enantiomerenreines Diastereomer)

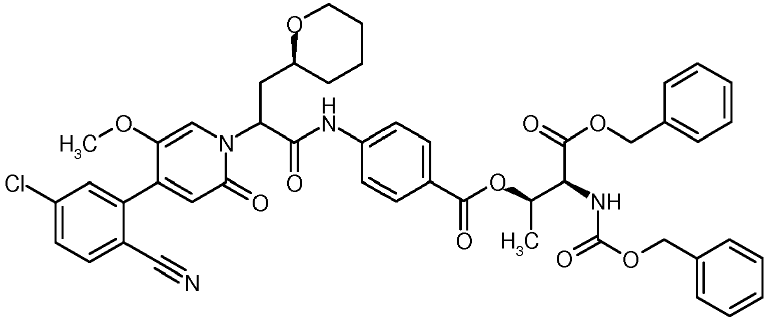

Eine Lösung von 250 mg (0.46 mmol) 4-({2-[4-(5-Chlor-2-cyanophenyl)-5-methoxy-2-oxopyridin-1(2*H*)-yl]-3-[(2*S*)-tetrahydro-2*H*-pyran-2-yl]propanoyl}amino)benzoesäure (enantiomerenreines Diastereomer 2) und 174 mg (0.51 mmol, 1.1 eq.) Benzyl-*N*-[(benzyloxy)carbonyl]-*L*-threoninat in 5 ml Tetrahydrofuran wurde unter Argon bei 0°C mit 11 mg (92 µmol, 0.2 eq.) 4-Dimethylaminopyridin und langsam mit 105 mg (0.51 mmol, 1.1 eq.) 1,3-Dicyclohexylcarbodiimid versetzt. Das Reaktionsgemisch wurde auf RT kommen gelassen, über Nacht bei RT gerührt und anschließend im Vakuum eingeengt. Nach Zugabe von Wasser/Essigsäureethylester und Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) getrennt und die erhaltene Mischfraktion nochmals mittels RP-HPLC (Reprosil C18, Acetonitril-Wasser-Gradient) aufgereinigt. Ausbeute: 191 mg (48% d. Th.)
LC/MS [Methode 1]: Rₜ = 1.34 min; MS (ESIpos): m/z = 861 (M+H)⁺.

### Beispiel 4.1A

### (2R,3S)-4-(Benzyloxy)-3-[(tert.-butoxycarbonyl)amino]-4-oxobutan-2-yl-4-({2-[4-(5-chlor-2-cyanophenyl)-5-methoxy-2-oxopyridin-1(2H)-yl]batanoyl}amino)benzoat (enantiomerenreines Diastereomer)

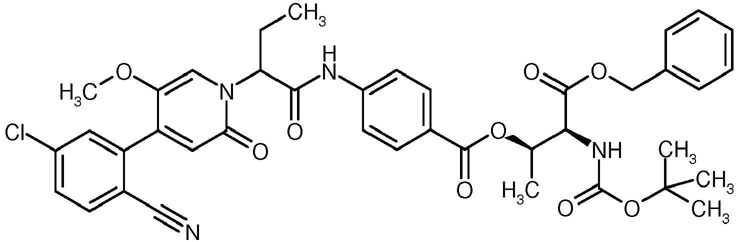

Eine Lösung von 1018 mg (2.16 mmol) 4-({2-[4-(5-Chlor-2-cyanophenyl)-5-methoxy-2-oxopyridin-1(2*H*)-yl]butanoyl}amino)benzoesäure (Enantiomer 2) und 736 mg (2.38 mmol, 1.1 eq.) Benzyl-*N*-(*tert*.-butoxycarbonyl)-*L*-threoninat in 22 ml Tetrahydrofuran wurde unter Argon bei 0°C mit 536 mg (2.60 mmol, 1.2 eq.) 1,3-Dicyclohexylcarbodiimid und 53 mg (0.43 mmol, 0.2 eq.) 4-Dimethylaminopyridin versetzt. Das Reaktionsgemisch wurde auf RT kommen gelassen, 6 h nachgerührt, über Nacht bei RT stehen gelassen und anschließend im Vakuum eingeengt. Nach Zugabe von Wasser/Essigsäureethylester und Phasentrennung wurde die wässrige Phase mit Essigsäureethylester extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde in Dichlormethan aufgenommen und mittels Flash-Chromatographie (Kieselgel-50, Cyclohexan-Essigsäureethylester-Gemisch) aufgereinigt. Ausbeute: 887 mg (54% d. Th.)
LC/MS [Methode 1]: Rₜ = 1.33 min; MS (ESIpos): m/z = 757 (M+H)⁺,
¹H-NMR (400 MHz, DMSO-d₆): δ [ppm] = 10.81 (s, 1H), 8.07-7.97 (m, 3H), 7.78-7.68 (m, 5H), 7.50 (s, 1H), 7.29-7.16 (m, 5H), 6.50 (s, 1H), 5.63 (dd, 1H), 5.51-5.43 (m, 1H), 5.12 (d, 1H), 5.04 (d, 1H), 4.54 (dd, 1H), 3.70 (s, 3H), 2.27-2.13 (m, 2H), 1.42 (s, 9H), 1.28 (d, 3H), 0.92 (t, 3H).

### Beispiel 4.1B

### N-(tert.-Butoxycarbonyl)-O-[4-({2-[4-(5-chlor-2-cyanophenyl)-5-methoxy-2-oxopyridin-1(2H)-yl]butanoyl}amino)benzoyl]-L-threonin (enantiomerenreines Diastereomer)

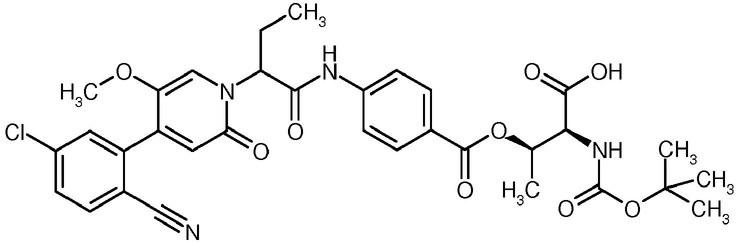

Eine Lösung von 777 mg (1.03 mmol) (2*R*,3*S*)-4-(Benzyloxy)-3-[(*tert*.-butoxycarbonyl)amino]-4-oxobutan-2-yl-4-({2-[4-(5-chlor-2-cyanophenyl)-5-methoxy-2-oxopyridin-1(2*H*)-yl]butanoyl}-amino)benzoat (enantiomerenreines Diastereomer) in 10 ml Ethanol wurde in Gegenwart von 78 mg Palladium (10%ig auf Aktivkohle) 45 min bei RT und Normaldruck hydriert. Anschließend wurde das Reaktionsgemisch über Celite filtriert, der Rückstand mit Ethanol und Wasser gewaschen und Ethanol im Vakuum entfernt. Der ausgefallene Rückstand wurde filtriert und im Vakuum getrocknet. Ausbeute: 594 mg (94% Reinheit, 86% d. Th.)
LC/MS [Methode 1]: Rₜ = 1.07 min; MS (ESIpos): m/z = 667 (M+H)⁺.

### Beispiel 5.1A

### N-(tert.-Butoxycarbonyl)-O-[4-({2-[4-(5-chlor-2-cyanophenyl)-5-methoxy-2-oxopyridin-1(2H)-yl]butanoyl}amino)benzoyl]-2-methyl-L-serin (enantiomerenreines Diastereomer)

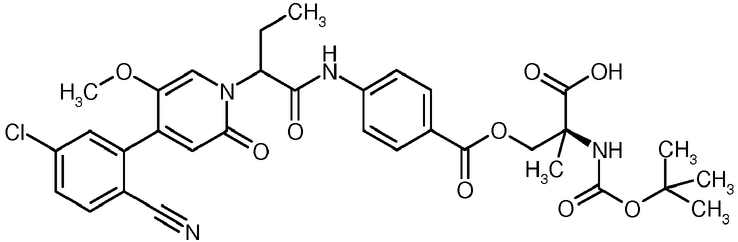

Eine Lösung von 50 mg (0.11 mmol) 4-({2-[4-(5-Chlor-2-cyanophenyl)-5-methoxy-2-oxopyridin-1(2*H*)-yl]butanoyl}amino)benzoesäure (Enantiomer 2) in 1 ml Dichlormethan wurde unter Argon bei -20°C mit 29 mg (0.13 mmol, 1.25 eq.) *N*-(*tert*.-Butoxycarbonyl)-2-methyl-*L*-serin, 3 mg (27 µmol, 0.25 eq.) 4-Dimethylaminopyridin, 23 µl (0.13 mmol, 1.25 eq.) *N,N-*Diisopropylethylamin und zuletzt 25 mg (0.13 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 16 mg (22% d. Th.)
LC/MS [Methode 1]: Rₜ = 1.05 min; MS (ESIpos): m/z = 667 (M+H)⁺,
¹H-NMR (400 MHz, DMSO-d₆): δ [ppm] = 12.7 (br. s, 1H), 10.84 (s, 1H), 8.00 (d, 1H), 7.96 (d, 2H), 7.79 (d, 2H), 7.77-7.70 (m, 2H), 7.49 (s, 1H), 7.27 (br. s, 1H), 6.54 (s, 1H), 5.64 (dd, 1H), 4.67-4.58 (m, 1H), 4.40-4.30 (m, 1H), 3.69 (s, 3H), 2.26-2.12 (m, 2H), 1.40 (s, 3H), 1.35 (s, 9H), 0.91 (t, 3H).

### Beispiel 6.1A

### (2S)-3-(Benzyloxy)-2-[(tert.-butoxycarbonyl)amino]-3-oxopropyl-4-({2-[4-(5-chlor-2-cyanophenyl)-5-methoxy-2-oxopyridin-1(2H)-yl]batanoyl}amino)benzoat (enantiomerenreines Diastereomer)

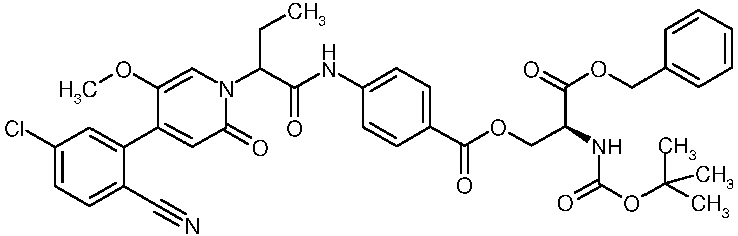

Eine Lösung von 500 mg (1.07 mmol) 4-({2-[4-(5-Chlor-2-cyanophenyl)-5-methoxy-2-oxopyridin-1(2*H*)-yl]butanoyl}amino)benzoesäure (Enantiomer 2) und 396 mg (1.34 mmol, 1.25 eq.) Benzyl-*N*-(*tert*.-butoxycarbonyl)-*L*-serinat in 20 ml Dichlormethan wurde unter Argon bei -20°C mit 33 mg (0.27 mmol, 0.25 eq.) 4-Dimethylaminopyridin, 0.23 ml (1.34 mmol, 1.25 eq.) *N,N-*Diisopropylethylamin und zuletzt 257 mg (1.34 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und 4.5 h bei RT nachgerührt. Nach Zugabe von Wasser/Dichlormethan und Phasentrennung wurde die wässrige Phase zweimal mit Dichlormethan extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 515 mg (61% d. Th.)
LC/MS [Methode 1]: Rₜ = 1.22 min; MS (ESIpos): m/z = 743 (M+H)⁺.

### Beispiel 6.1B

### (2S)-2-Amino-3-(benzyloxy)-3-oxopropyl-4-({2-[4-(5-chlor-2-cyanophenyl)-5-methoxy-2-oxopyridin-1(2H)-yl]batanoyl}amino)benzoat (enantiomerenreines Diastereomer)

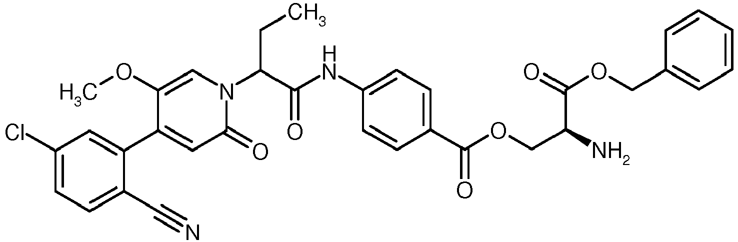

Gemäß der allgemeinen Methode 6A wurden 829 mg (1.08 mmol) (2*S*)-3-(Benzyloxy)-2-[(*tert*.-butoxycarbonyl)amino]-3-oxopropyl-4-({2-[4-(5-chlor-2-cyanophenyl)-5-methoxy-2-oxopyridin-1(2*H*)-yl]butanoyl}amino)benzoat (enantiomerenreines Diastereomer) in Gegenwart von 1.67 ml (21.6 mmol, 20 eq.) Trifluoressigsäure umgesetzt. Das Rohprodukt wurde mittels Flash-Chromatographie (Kieselgel-50, Dichormethan-Methanol-Gemisch) aufgereinigt. Ausbeute: 570 mg (82% d. Th.)
LC/MS [Methode 1]: Rₜ = 0.84 min; MS (ESIpos): m/z = 643 (M+H)⁺.

### Beispiel 7.1A

### (2S)-3-(Benzyloxy)-3-oxo-2-[(pyrrolidin-1-ylacetyl)amino]propyl-4-({2-[4-(5-chlor-2-cyanophenyl)-5-methoxy-2-oxopyridin-1(2H)-yl]batanoyl}amino)benzoat (enantiomerenreines Diastereomer)

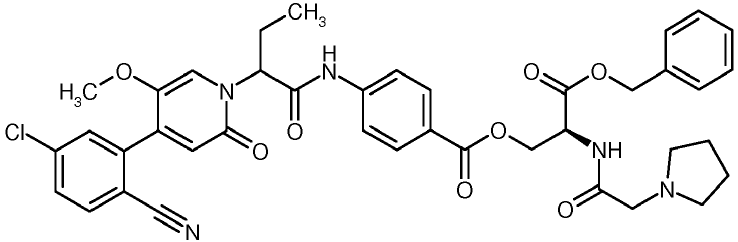

Gemäß der allgemeinen Methode 5A wurden 109 mg (0.17 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]butanoyl}-amino)benzoat (enantiomerenreines Diastereomer) mit 21 mg (0.16 mmol, 0.95 eq.) Pyrrolidin-1-ylessigsäure umgesetzt. Das Reaktionsgemisch wurde im Vakuum eingeengt. Der Rückstand wurde mit Wasser umkristallisiert, der ausfallende Feststoff filtriert, mit Wasser gewaschen und im Vakuum getrocknet. Ausbeute: 132 mg (94% Reinheit, 97% d. Th.)
LC/MS [Methode 1]: Rₜ = 0.89 min; MS (ESIpos): m/z = 754 (M+H)⁺.

### Beispiel 8.1A

### Benzyl-1-benzylprolyl-O-[4-({2-[4-(5-chlor-2-cyanophenyl)-5-methoxy-2-oxopyridin-1(2H)-yl]butanoyl}amino)benzoyl]-L-serinat (Diastereomerengemisch)

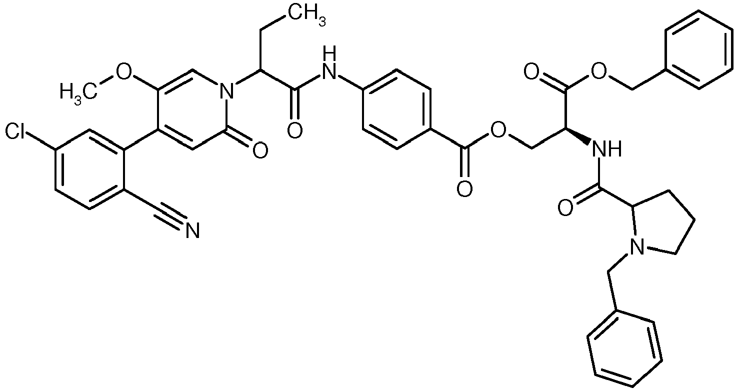

Gemäß der allgemeinen Methode 5A wurden 103 mg (0.16 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]butanoyl}-amino)benzoat (enantiomerenreines Diastereomer) mit 31 mg (0.15 mmol, 0.95 eq.) 1-Benzylprolin (Racemat) umgesetzt. Das Reaktionsgemisch wurde im Vakuum eingeengt. Der Rückstand wurde mit Wasser umkristallisiert, der ausfallende Feststoff filtriert, mit Wasser gewaschen und im Vakuum getrocknet. Ausbeute: 136 mg (quant.)
LC/MS [Methode 1]: Rₜ = 0.98 min; MS (ESIpos): m/z = 830 (M+H)⁺.

### Beispiel 9.1A

### (2S)-3-(Benzyloxy)-2-({[(2R)-1-methylpiperidin-2-yl]carbonyl}amino)-3-oxopropyl-4-({2-[4-(5-chlor-2-cyanophenyl)-5-methoxy-2-oxopyridin-1(2H)-yl]butanoyl}amino)benzoat (enantiomerenreines Diastereomer)

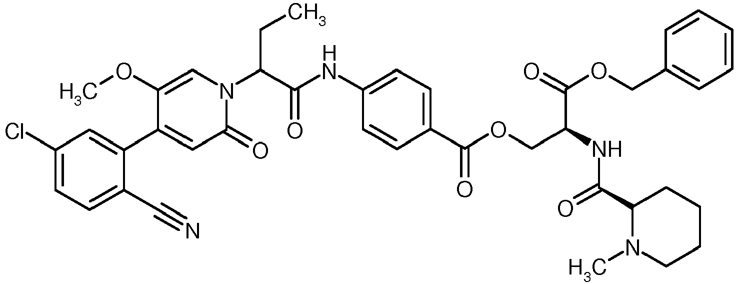

Gemäß der allgemeinen Methode 5A wurden 109 mg (0.17 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]butanoyl}-amino)benzoat (enantiomerenreines Diastereomer) mit 24 mg (0.17 mmol, 1.0 eq.) (2*R*)-1-Methylpiperidin-2-carbonsäure umgesetzt. Das Reaktionsgemisch wurde im Vakuum eingeengt. Der Rückstand wurde mit Wasser umkristallisiert, der ausfallende Feststoff filtriert, mit Wasser gewaschen und im Vakuum getrocknet. Ausbeute: 141 mg (93% Reinheit, quant.)
LC/MS [Methode 1]: Rₜ = 0.86 min; MS (ESIpos): m/z = 768 (M+H)⁺.

### Beispiel 10.1A

### (2S)-3-(Benzyloxy)-2-({[1-(dimethylamino)cyclopropyl]carbonyl}amino)-3-oxopropyl-4-({2-[4-(5-chlor-2-cyanophenyl)-5-methoxy-2-oxopyridin-1(2H)-yl]butanoyl}amino)benzoat (enantiomerenreines Diastereomer)

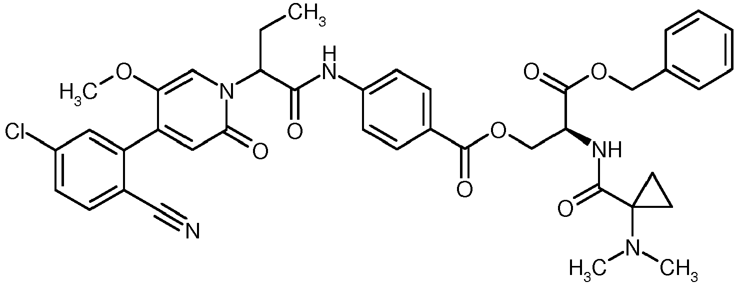

Gemäß der allgemeinen Methode 5A wurden 109 mg (0.17 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]butanoyl}amino)-benzoat (enantiomerenreines Diastereomer) mit 21 mg (0.17 mmol, 1.0 eq.) 1-(Dimethylamino)cyclopropan-carbonsäure umgesetzt. Das Reaktionsgemisch wurde im Vakuum eingeengt. Der Rückstand wurde mit Wasser umkristallisiert, der ausfallende Feststoff filtriert, mit Wasser gewaschen und im Vakuum getrocknet. Ausbeute: 127 mg (85% Reinheit, 87% d. Th.)
LC/MS [Methode 1]: Rₜ = 1.00 min; MS (ESIpos): m/z = 754 (M+H)⁺.

### Beispiel 11.1A

### Benzyl-N,N-dimethyl-L-valyl-O-[4-({2-[4-(5-chlor-2-cyanophenyl)-5-methoxy-2-oxopyridin-1(2H)-yl]butanoyl}amino)benzoyl]-L-serinat (enantiomerenreinesDiastereomer)

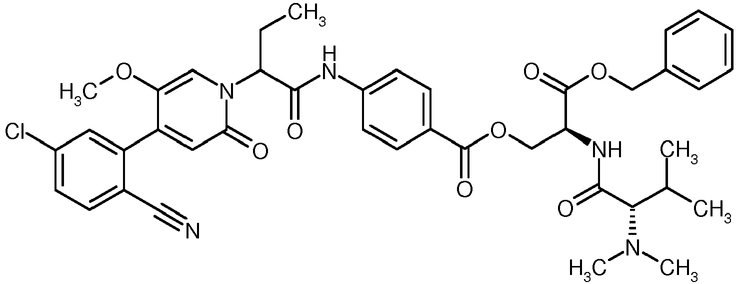

Gemäß der allgemeinen Methode 5A wurden 138 mg (0. 21 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]batanoyl}amino)-benzoat (enantiomerenreines Diastereomer) mit 30 mg (0. 21 mmol, 1.0 eq.) *N,N*-Dimethyl-*L*-valin umgesetzt. Das Reaktionsgemisch wurde im Vakuum eingeengt. Der Rückstand wurde mit Wasser umkristallisiert, der ausfallende Feststoff filtriert, mit Wasser gewaschen und im Vakuum getrocknet. Ausbeute: 158 mg (90% Reinheit, 89% d. Th.)
LC/MS [Methode 1]: Rₜ = 0.87 min; MS (ESIpos): m/z = 770 (M+H)⁺.

### Beispiel 12.1A

### 2-Benzyl-1-tert.-butyl-(2S,3S)-3-hydroxypyrrolidin-1,2-dicarboxylat

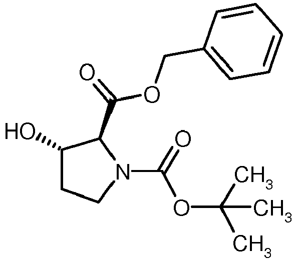

Eine Lösung von 200 mg (0.87 mmol) (3*S*)-1-(*tert*.-Butoxycarbonyl)-3-hydroxy-*L*-prolin in 6.5 ml Methanol und 1.3 ml Wasser wurde mit 141 mg (0.43 mmol, 0.5 eq.) Cäsiumcarbonat versetzt, 1 h bei RT gerührt und im Vakuum eingeengt. Der Rückstand wurde zweimal mit jeweils 10 ml Toluol kodestilliert, 2 h im Hochvakuum getrocknet, in 8 ml Dimethylformamid aufgenommen, bei 0°C mit 98 µl (0.825 mmol, 0.95 eq.) Benzylbromid versetzt und 1 h bei RT gerührt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat), filtriert und im Vakuum eingeengt. Ausbeute: 286 mg (quant.)
LC/MS [Methode 1]: Rₜ = 0.92 min; MS (ESIpos): m/z = 322 (M+H)⁺.

### Beispiel 12.1B

### 2-Benzyl-1-tert.-butyl-(2S,3S)-3-{[4-({2-[4-(5-chlor-2-cyanophenyl)-5-methoxy-2-oxopyridin-1(2H)-yl]butanoyl}amino)benzoyl]oxy}pyrrolidin-1,2-dicarboxylat (enantiomerenreines Diastereomer)

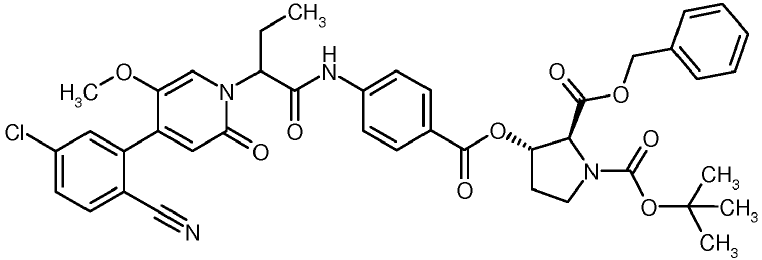

Eine Lösung von 248 mg (75% Reinheit, 0.40 mmol) 4-({2-[4-(5-Chlor-2-cyanophenyl)-5-methoxy-2-oxopyridin-1(2*H*)-yl]butanoyl}amino)benzoesäure (Enantiomer 2) in 4 ml Dichlormethan wurde unter Argon bei -20°C mit 160 mg (0.50 mmol, 1.25 eq.) 2-Benzyl-1-*tert*.-butyl-(2*S*,3*S*)-3-hydroxypyrrolidin-1,2-dicarboxylat, 12 mg (0.1 mmol, 0.25 eq.) 4-Dimethylaminopyridin, 0.87 µl (0.50 mmol, 1.25 eq.) *N,N*-Diisopropylethylamin und zuletzt 96 mg (0.50 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Erneute Zugabe von 0.5 eq. Dimethylaminopyridin, 0.5 eq. *N,N*-Diisopropylethylamin und zuletzt 0.5 eq. 1-Ethyl-3-(3-dimethylaminopropyl)carbodiimid-Hydrochlorid bei -20°C und Rühren bei RT für 2 Tage ergab kaum weiteren Umsatz. Nach Zugabe von Wasser/Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 57 mg (18% d. Th.)
LC/MS [Methode 1]: Rₜ = 1.27 min; MS (ESIpos): m/z = 769 (M+H)⁺.

### Beispiel 12.1C

### Benzyl-(3S)-3-{[4-({2-[4-(5-chlor-2-cyanophenyl)-5-methoxy-2-oxopyridin-1(2H)-yl]butanoyl}-amino)benzoyl]oxy}-L-prolinat (enantiomerenreines Diastereomer)

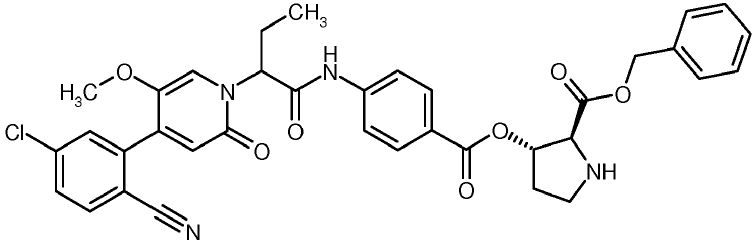

Gemäß der allgemeinen Methode 6A wurden 57 mg (73 µmol) 2-Benzyl-1-*tert*.-butyl-(2*S*,3*S*)-3-{[4-({2-[4-(5-chlor-2-cyanophenyl)-5-methoxy-2-oxopyridin-1(2*H*)-yl]butanoyl}amino)benzoyl]-oxy}pyrrolidin-1,2-dicarboxylat (enantiomerenreines Diastereomer) in Gegenwart von 113 µl (1.47 mmol, 20 eq.) Trifluoressigsäure umgesetzt. Das Rohprodukt wurde ohne weitere Reinigung umgesetzt. Ausbeute: 74 mg (31% Reinheit, 46% d. Th.)
LC/MS [Methode 1]: Rₜ = 0.87 min; MS (ESIpos): m/z = 669 (M+H)⁺.

### Beispiel 13.1A

### 2-Benzyl-1-tert.-butyl-(2R,4R)-4-hydroxypyrrolidin-1,2-dicarboxylat

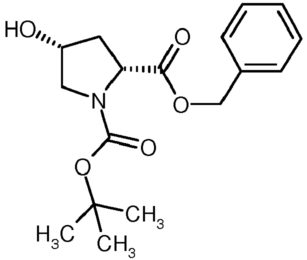

Eine Lösung von 500 mg (2.15 mmol) (4*R*)-1-(*tert*.-Butoxycarbonyl)-4-hydroxy-*D*-prolin in 18 ml Methanol und 3.5 ml Wasser wurde mit 352 mg (1.08 mmol, 0.5 eq.) Cäsiumcarbonat versetzt, 1 h bei RT gerührt und im Vakuum eingeengt. Der Rückstand wurde zweimal mit jeweils 10 ml Toluol kodestilliert, 2 h im Hochvakuum getrocknet, in 10 ml Dimethylformamid aufgenommen, bei 0°C mit 244 µl (2.05 mmol, 0.95 eq.) Benzylbromid versetzt und 1 h bei RT gerührt. Nach Zugabe von Wasser/Essigsäureethylester und Phasentrennung wurde die wässrige Phase zweimal mit Essigsäureethylester extrahiert. Die vereinigten, organischen Phasen wurden getrocknet (Natriumsulfat), filtriert und im Vakuum eingeengt. Ausbeute: 721 mg (quant.)
LC/MS [Methode 1]: Rₜ = 0.90 min; MS (ESIpos): m/z = 322 (M+H)⁺.

### Beispiel 13.1B

### 2-Benzyl-1-tert.-butyl-(2R,4R)-4-{[4-({2-[4-(5-chlor-2-cyanophenyl)-5-methoxy-2-oxopyridin-1(2H)-yl]butanoyl}amino)benzoyl]oxy}pyrrolidin-1,2-dicarboxylat (enantiomerenreines Diastereomer)

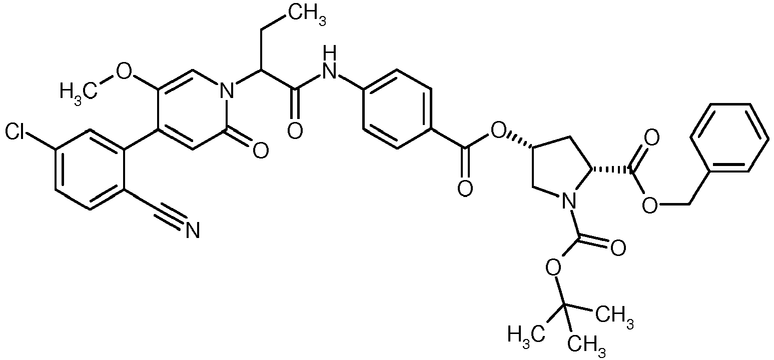

Eine Lösung von 188 mg (0.40 mmol) 4-({2-[4-(5-Chlor-2-cyanophenyl)-5-methoxy-2-oxopyridin-1(2*H*)-yl]butanoyl}amino)benzoesäure (Enantiomer 2) in 4 ml Dichlormethan wurde unter Argon bei -20°C mit 167 mg (0.50 mmol, 1.25 eq.) 2-Benzyl-1-*tert*.-butyl-(2*R*,4*R*)-4-hydroxypyrrolidin-1,2-dicarboxylat, 12 mg (0.1 mmol, 0.25 eq.) 4-Dimethylaminopyridin, 0.87 µl (0.50 mmol, 1.25 eq.) *N,N*-Diisopropylethylamin und zuletzt 96 mg (0.50 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Der Rückstand wurde mittels Flash-Chromatographie (Kieselgel-50, Cyclohexan-Essigsäureethylester-Gemisch) aufgereinigt. Ausbeute: 198 mg (82% Reinheit, 53% d. Th.)
LC/MS [Methode 1]: Rₜ = 1.26 min; MS (ESIpos): m/z = 769 (M+H)⁺.

### Beispiel 13.1C

### Benzyl-(4R)-4-{[4-({2-[4-(5-chlor-2-cyanophenyl)-5-methoxy-2-oxopyridin-1(2H)-yl]butanoyl}amino)benzoyl]oxy}-D-prolinat (enantiomerenreines Diastereomer)

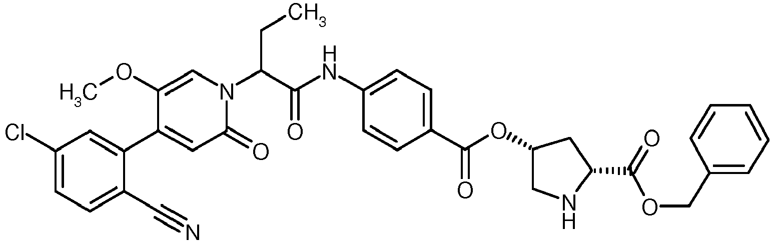

Gemäß der allgemeinen Methode 6A wurden 198 mg (82% Reinheit, 0.21 mmol) 2-Benzyl-1-*tert*.-butyl-(2*R*,4*R*)-4-{[4-({2-[4-(5-chlor-2-cyanophenyl)-5-methoxy-2-oxopyridin-1(2*H*)-yl]butanoyl}-amino)benzoyl]oxy}pyrrolidin-1,2-dicarboxylat (enantiomerenreines Diastereomer) in Gegenwart von 325 µl (4.22 mmol, 20 eq.) Trifluoressigsäure umgesetzt. Das Rohprodukt wurde mittels RP-HPLC (Reprosil C18, Acetonitril-Wasser-Gradient) aufgereinigt. Ausbeute: 71 mg (94% Reinheit, 47% d. Th.)
LC/MS [Methode 1]: Rₜ = 0.87 min; MS (ESIpos): m/z = 669 (M+H)⁺.

### Beispiel 14.1A

### Benzyl-(2S)-3-hydroxy-2-(pyrrolidin-1-yl)propanoat

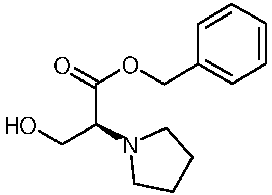

Eine Lösung von 500 mg (2.16 mmol) L-Serinbenzylester-Hydrochlorid und 869 mg (2.81 mmol, 1.3 eq.) 1,4-Diiodbutan in 25 ml 2-Propanol wurde unter Argon bei RT mit 595 mg (5.61 mmol, 2.6 eq.) Natriumcarbonat versetzt und über Nacht bei 90°C gerührt. Nach Abkühlen auf RT und Entfernen des 2-Propanols im Vakuum wurde der Rückstand mit Essigsäureethylester versetzt. Die unlöslichen Salze wurden filtriert. Das Filtrat wurde mit Wasser gewaschen und die organische Phase getrocknet (Natriumsulfat), filtriert und im Vakuum eingeengt. Der Rückstand wurde mittels Flash-Chromatographie (Kieselgel-50, Dichlormethan, dann Dichlormethan/Methanol 5:1) aufgereinigt. Ausbeute: 268 mg (48% d. Th.)
LC/MS [Methode 5]: Rₜ = 2.03 min; MS (ESIpos): m/z = 250 (M+H)⁺.

### Beispiel 14.1B

### (2S)-3-(Benzyloxy)-3-oxo-2-(pyrrolidin-1-yl)propyl-4-({2-[4-(5-chlor-2-cyanophenyl)-5-methoxy-2-oxopyridin-1(2H)-yl]butanoyl}amino)benzoat (enantiomerenreines Diastereomer)

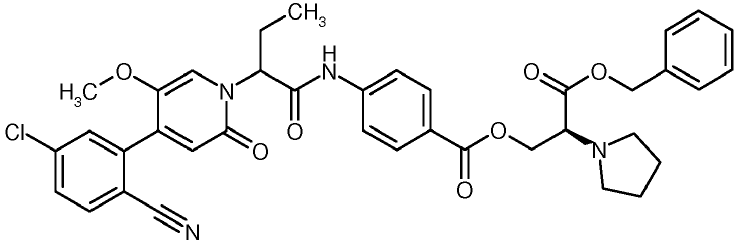

Eine Lösung von 233 mg (0.50 mmol) 4-({2-[4-(5-Chlor-2-cyanophenyl)-5-methoxy-2-oxopyridin-1(2*H*)-yl]butanoyl}amino)benzoesäure (Enantiomer 2) in 8 ml Dichlormethan wurde unter Argon bei -20°C mit 162 mg (0.63 mmol, 1.25 eq.) Benzyl-(2*S*)-3-hydroxy-2-(pyrrolidin-1-yl)propanoat, 15 mg (0.13 mmol, 0.25 eq.) 4-Dimethylaminopyridin, 109 µl (0.63 mmol, 1.25 eq.) *N,N-*Diisopropylethylamin und zuletzt 120 mg (0.63 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels Flash-Chromatographie (Kieselgel-50, Cyclohexan-Essigsäureethylester-Gemisch) und weiter mittels RP-HPLC (Reprosil C18, Acetonitril-Wasser-Gradient) aufgereinigt. Ausbeute: 73 mg (21% d. Th.)
LC/MS [Methode 1]: Rₜ = 0.90 min; MS (ESIpos): m/z = 697 (M+H)⁺.

### Beispiel 15.1A

### 4-(2-{[4-({2-[4-(5-Chlor-2-cyanophenyl)-5-methoxy-2-oxopyridin-1(2H)-yl]butanoyl}amino)-benzoyl]oxy}ethyl)piperidin-1-carbonsäure-tert.-butylester (Enantiomer 2)

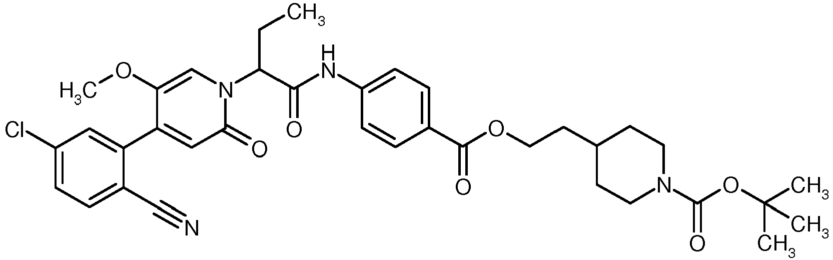

Eine Lösung von 93 mg (0.20 mmol) 4-({2-[4-(5-Chlor-2-cyanophenyl)-5-methoxy-2-oxopyridin-1(2*H*)-yl]butanoyl}amino)benzoesäure (Enantiomer 2) in 2 ml Dichlormethan wurde unter Argon bei -20°C mit 57 mg (0.25 mmol, 1.25 eq.) 4-(2-Hydroxyethyl)piperidin-1-carbonsäure-*tert*.-butylester, 6 mg (50 µmol, 0.25 eq.) 4-Dimethylaminopyridin, 44 µl (0.25 mmol, 1.25 eq.) *N,N-*Diisopropylethylamin und zuletzt 48 mg (0.50 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 66 mg (49% d. Th.)
LC/MS [Methode 1]: Rₜ = 1.28 min; MS (ESIpos): m/z = 677 (M+H)⁺.

### Ausführungsbeispiele

### Beispiel 1

### O-[4-({2-[4-(5-Chlor-2-cyanophenyl)-5-methoxy-2-oxopyridin-1(2H)-yl]-3-[(2S)-tetrahydro-2H-pyran-2-yl]propanoyl}amino)benzoyl]-L-threonin (enantiomerenreines Diastereomer)

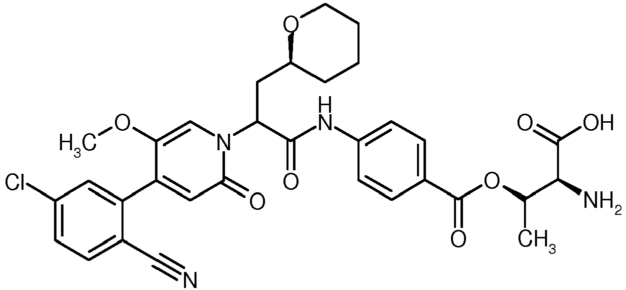

Eine Lösung von 191 mg (0.22 mmol) (2*R*,3*S*)-4-(Benzyloxy)-3-{[(benzyloxy)carbonyl]amino}-4-oxobutan-2-yl-4-({2-[4-(5-chlor-2-cyanophenyl)-5-methoxy-2-oxopyridin-1(2*H*)-yl]-3-[(2*S*)-tetrahydro-2*H*-pyran-2-yl]propanoyl}amino)benzoat (enantiomerenreines Diastereomer) in 5 ml Ethanol wurde in Gegenwart von 19 mg Palladium (10%ig auf Aktivkohle) 2.5 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der ausgefallene Rückstand wurde filtriert und im Vakuum getrocknet. Das Rohprodukt wurde mittels RP-HPLC (Reprosil C18, Acetonitril-Wasser-Gradient) aufgereinigt. Ausbeute: 40 mg (28% d. Th.)
LC/MS [Methode 1]: Rₜ = 0.85 min; MS (ESIpos): m/z = 637 (M+H)⁺,
¹H-NMR (400 MHz, DMSO-d₆): δ [ppm] = 10.75 (s, 1H), 8.05-7.96 (m, 3H), 7.78 (d, 2H), 7.75-7.70 (m, 2H), 7.53 (s, 1H), 6.52 (s, 1H), 5.81 (t, 1H), 5.43-5.34 (m, 1H), 3.90-3.82 (m, 1H), 3.68 (s, 3H), 2.35-2.26 (m, 1H), 2.22-2.13 (m, 1H), 1.79-1.71 (m, 1H), 1.67-1.59 (m, 1H), 1.47-1.39 (m, 3H), 1.38 (d, 3H), 1.34-1.22 (m, 1H).

### Beispiel 2

### O-[4-({2-[4-(5-Chlor-2-cyanophenyl)-5-methoxy-2-oxopyridin-1(2H)-yl]butanoyl}amino)-benzoyl]-L-threonin (enantiomerenreines Diastereomer)

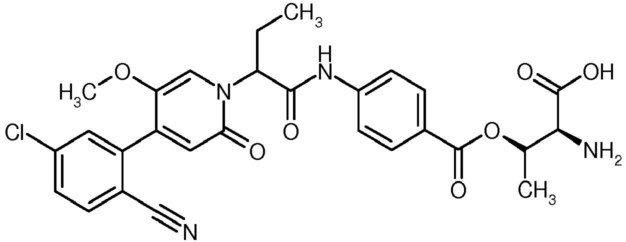

Eine Lösung von 492 mg (94% Reinheit, 0.69 mmol) *N*-(*tert*.-Butoxycarbonyl)-*O*-[4-({2-[4-(5-chlor-2-cyanophenyl)-5-methoxy-2-oxopyridin-1(2*H*)-yl]butanoyl}amino)benzoyl]-*L*-threonin (enantiomerenreines Diastereomer) in 5 ml Dichlormethan wurde unter Argon bei 0°C tropfenweise mit 0.8 ml (10.4 mmol, 15 eq.) TFA versetzt. Das Reaktionsgemisch wurde auf RT kommen gelassen, 6 h bei RT nachgerührt und anschließend im Vakuum eingeengt. Der Rückstand wurde zweimal mit jeweils 10 ml Toluol koevaporiert und im Vakuum getrocknet. Das Rohprodukt wurde mittels RP-HPLC (Reprosil C18, Acetonitril-Wasser-Gradient) vorgereinigt (Ausbeute: 405 mg, 69% Reinheit) und mittels zweiter RP-HPLC (Rohprodukt in 8 ml Acetonitril/4 ml Wasser gelöst; Phase: Shield RP18 5 µm, 19 mm x 100 mm; Eluent: 40% Acetonitril/48% Wasser/12% Ameisensäure in Wasser, Fluss: 25 ml/min; Temperatur: 40°C) weiter aufgereinigt. Ausbeute: 9 mg (95% Reinheit, 2% d. Th.) und 39 mg (91% Reinheit, 9% d. Th.)
LC/MS [Methode 1]: Rₜ = 0.80 min; MS (ESIpos): m/z = 567 (M+H)⁺,
¹H-NMR (400 MHz, DMSO-d₆): δ [ppm] = 10.82 (s, 1H), 8.06-7.97 (m, 3H), 7.82-7.55 (br. s, 2H), 7.79-7.70 (m, 4H), 7.50 (s, 1H), 6.54 (s, 1H), 5.63 (dd, 1H), 5.43-5.34 (m, 1H), 3.69 (s, 3H), 3.41-3.36 (m, 1H), 2.26-2.13 (m, 2H), 1.38 (d, 3H), 0.90 (t, 3H).

### Beispiel 3

### O-[4-({2-[4-(5-Chlor-2-cyanophenyl)-5-methoxy-2-oxopyridin-1(2H)-yl]butanoyl}amino)-benzoyl]-2-methyl-L-serin (enantiomerenreines Diastereomer)

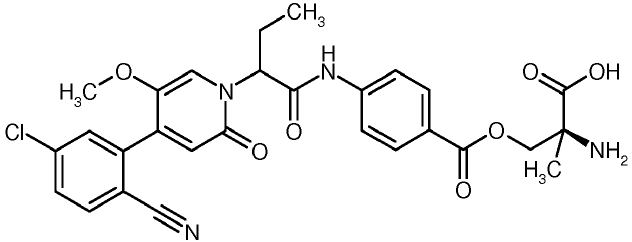

Eine Lösung von 16 mg (20 µmol) *N*-(*tert*.-Butoxycarbonyl)-*O*-[4-({2-[4-(5-chlor-2-cyanophenyl)-5-methoxy-2-oxopyridin-1(2*H*)-yl]butanoyl}amino)benzoyl]-2-methyl-*L*-serin (enantiomerenreines Diastereomer) in 2 ml Dichlormethan wurde bei 0°C mit 37 µl (0.48 mmol, 20 eq.) Trifluoressigsäure versetzt und über Nacht bei RT gerührt. Das Reaktionsgemisch wurde im Vakuum eingeengt und der Rückstand mittels RP-HPLC (Reprosil C18, Acetonitril-Wasser-Gradient) aufgereinigt. Ausbeute: 5 mg (33% d. Th.)
LC/MS [Methode 1]: Rₜ = 0.74 min; MS (ESIpos): m/z = 567 (M+H)⁺,
¹H-NMR (400 MHz, DMSO-d₆): δ [ppm] = 10.85 (br. s, 1H), 8.09 (d, 2H), 8.00 (d, 1H), 7.92-7.64 (br. s, 2H), 7.78 (d, 2H), 7.75-7.70 (m, 2H), 7.50 (s, 1H), 6.54 (s, 1H), 5.63 (dd, 1H), 4.39-4.28 (m, 2H), 3.69 (s, 3H), 2.27-2.12 (m, 2H), 1.33 (s, 3H), 0.91 (t, 3H).

### Beispiel 4

### O-[4-({2-[4-(5-Chlor-2-cyanophenyl)-5-methoxy-2-oxopyridin-1(2H)-yl]butanoyl}amino)-benzoyl]-L-serin (enantiomerenreines Diastereomer)

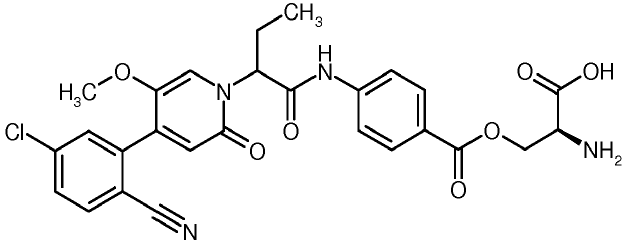

Eine Lösung von 193 mg (0.30 mmol) (2*S*)-2-Amino-3-(benzyloxy)-3-oxopropyl-4-({2-[4-(5-chlor-2-cyanophenyl)-5-methoxy-2-oxopyridin-1(2*H*)-yl]butanoyl}amino)benzoat (enantiomerenreines Diastereomer) in 5 ml Ethanol wurde in Gegenwart von 19 mg Palladium (10%ig auf Aktivkohle) 30 min bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 101 mg (61% d. Th.)
LC/MS [Methode 1]: Rₜ = 0.77 min; MS (ESIpos): m/z = 553 (M+H)⁺,
¹H-NMR (400 MHz, DMSO-d₆): δ [ppm] = 10.83 (s, 1H), 8.04 (d, 2H), 8.00 (d, 1H), 7.78 (d, 2H), 7.76-7.71 (m, 2H), 7.49 (s, 1H), 6.54 (s, 1H), 5.63 (dd, 1H), 4.63-4.56 (m, 1H), 4.35 (dd, 1H), 3.69 (s, 3H), 3.59 (dd, 1H), 2.27-2.12 (m, 2H), 0.91 (t, 3H).

### Beispiel 5

### O-[4-({2-[4-(5-Chlor-2-cyanophenyl)-5-methoxy-2-oxopyridin-1(2H)-yl]butanoyl}amino)-benzoyl]-N-(pyrrolidin-1-ylacetyl)-L-serin (enantiomerenreines Diastereomer)

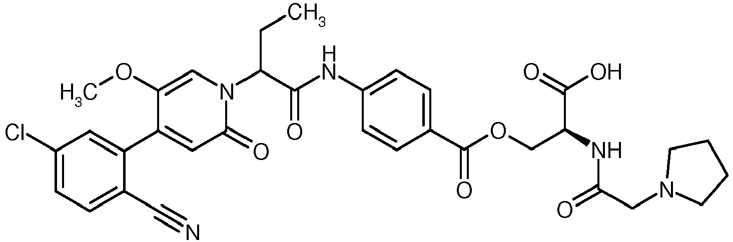

Eine Lösung von 132 mg (94% Reinheit, 0.18 mmol) (2*S*)-3-(Benzyloxy)-3-oxo-2-[(pyrrolidin-1-ylacetyl)amino]propyl-4-({2-[4-(5-chlor-2-cyanophenyl)-5-methoxy-2-oxopyridin-1(2*H*)-yl]-butanoyl}amino)benzoat (enantiomerenreines Diastereomer) in 10 ml Ethanol wurde in Gegenwart von 15 mg Palladium (10%ig auf Aktivkohle) 1 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 47 mg (43% d. Th.)
LC/MS [Methode 1]: Rₜ = 0.71 min; MS (ESIpos): m/z = 664 (M+H)⁺,
¹H-NMR (400 MHz, DMSO-d₆): δ [ppm] = 10.85 (s, 1H), 8.23 (d, 1H), 8.00 (d, 1H), 7.90 (d, 2H), 7.81-7.70 (m, 4H), 7.49 (s, 1H), 6.54 (s, 1H), 5.63 (dd, 1H), 4.65 (br. s, 1H), 4.57 (dd, 1H), 4.49 (dd, 1H), 3.69 (s, 3H), 3.27 (s, 2H), 2.65 (br. s, 4H), 2.26-2.10 (m, 2H), 1.71 (br. s, 4H), 0.90 (t, 3H).

### Beispiel 6

### Prolyl-O-[4-({2-[4-(5-chlor-2-cyanophenyl)-5-methoxy-2-oxopyridin-1(2H)-yl]butanoyl}amino)-benzoyl]-L-serin (Diastereomerengemisch)

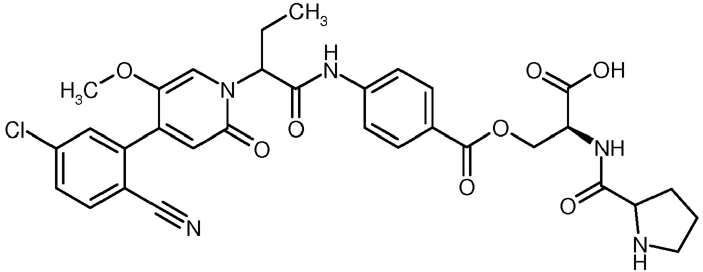

Eine Lösung von 132 mg (0.16 mmol) Benzyl-1-benzylprolyl-*O*-[4-({2-[4-(5-chlor-2-cyanophenyl)-5-methoxy-2-oxopyridin-1(2*H*)-yl]butanoyl}amino)benzoyl]-*L*-serinat (Diastereomerengemisch) in 10 ml Ethanol wurde in Gegenwart von 15 mg Palladium (10%ig auf Aktivkohle) 4 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 5 mg (90% Reinheit; 5% d. Th.)
LC/MS [Methode 1]: Rₜ = 0.75 min/0.77 min; MS (ESIpos): m/z = 650 (M+H)⁺,
¹H-NMR (400 MHz, DMSO-d₆): δ [ppm] = 10.85 (s, 1H), 8.5 (br. s, 1H), 8.00/7.95 (2x d, 1H), 7.90 (d, 2H), 7.81-7.70 (m, 4H), 7.49/7.48 (2x s, 1H), 6.54/6.47 (2x s, 1H), 5.63 (dd, 1H), 4.62-4.53 (m, 1H), 4.50-4.37 (m, 2H), 3.93-3.83 (m, 1H), 3.69/3.68 (2x s, 3H), 3.02-2.86 (m, 3H), 2.27-2.12 (m, 2H), 2.12-2.02 (m, 1H), 1.80-1.58 (m, 3H), 0.90 (t, 3H).

### Beispiel 7

### O-[4-({2-[4-(5-Chlor-2-cyanophenyl)-5-methoxy-2-oxopyridin-1(2H)-yl]butanoyl}amino)-benzoyl]-N-{[(2R)-1-methylpiperidin-2-yl]carbonyl}-L-serin (enantiomerenreines Diastereomer)

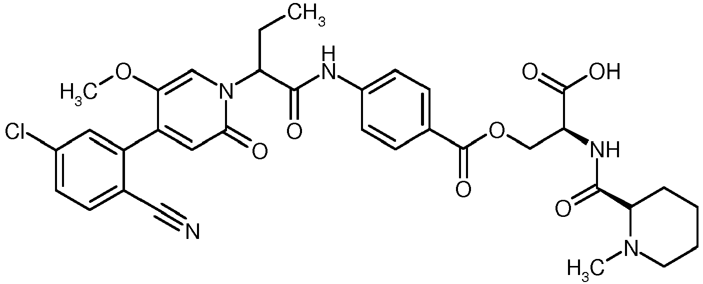

Eine Lösung von 141 mg (93% Reinheit, 0.17 mmol) (2*S*)-3-(Benzyloxy)-2-({[(2*R*)-1-methylpiperidin-2-yl]carbonyl}amino)-3-oxopropyl-4-({2-[4-(5-chlor-2-cyanophenyl)-5-methoxy-2-oxopyridin-1(2*H*)-yl]butanoyl}ainino)benzoat (enantiomerenreines Diastereomer) in 10 ml Ethanol wurde in Gegenwart von 20 mg Palladium (10%ig auf Aktivkohle) 1 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 8 mg (7% d. Th.)
LC/MS [Methode 1]: Rₜ = 0.73 min; MS (ESIpos): m/z = 678 (M+H)⁺,
¹H-NMR (400 MHz, DMSO-d₆): δ [ppm] = 10.84 (s, 1H), 8.1 (br. s, 1H), 8.00 (d, 1H), 7.90 (d, 2H), 7.81-7.70 (m, 4H), 7.49 (s, 1H), 6.54 (s, 1H), 5.63 (dd, 1H), 4.64-4.52 (m, 2H), 4.50-4.41 (m, 1H), 3.69 (s, 3H), 2.97-2.87 (m, 1H), 2.27-1.99 (m, 6H), 1.76-1.62 (m, 2H), 1.62-1.54 (m, 1H), 1.54-1.46 (m, 2H), 1.27-1.11 (m, 2H), 0.91 (t, 3H).

### Beispiel 8

### O-[4-({2-[4-(5-Chlor-2-cyanophenyl)-5-methoxy-2-oxopyridin-1(2H)-yl]butanoyl}amino)-benzoyl]-N-{[1-(dimethylamino)cyclopropyl]carbonyl}-L-serin (enantiomerenreines Diastereomer)

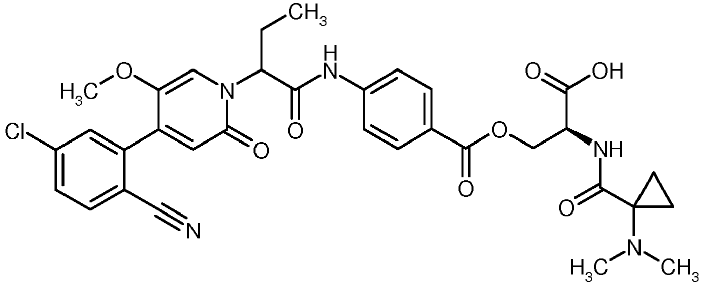

Eine Lösung von 127 mg (85% Reinheit, 0.14 mmol) (2*S*)-3-(Benzyloxy)-2-({[1-(dimethylamino)-cyclopropyl]-carbonyl}amino)-3-oxopropyl-4-({2-[4-(5-chlor-2-cyanophenyl)-5-methoxy-2-oxopyridin-1(2*H*)-yl]batanoyl}amino)benzoat (enantiomerenreines Diastereomer) in 10 ml Ethanol wurde in Gegenwart von 20 mg Palladium (10%ig auf Aktivkohle) 1 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 47 mg (49% d. Th.)
LC/MS [Methode 1]: Rₜ = 0.75 min; MS (ESIpos): m/z = 664 (M+H)⁺,
¹H-NMR (400 MHz, DMSO-d₆): δ [ppm] = 13.1 (br. s, 1H), 10.83 (s, 1H), 8.33 (d, 1H), 8.00 (d, 1H), 7.89 (d, 2H), 7.81-7.70 (m, 4H), 7.49 (s, 1H), 6.54 (s, 1H), 5.63 (dd, 1H), 4.72-4.65 (m, 1H), 4.62 (dd, 1H), 4.50 (dd, 1H), 3.69 (s, 3H), 2.26-2.16 (m, 2H), 2.15 (s, 6H), 1.00-0.85 (m, 7H).

### Beispiel 9

### N,N-Dimethyl-L-valyl-O-[4-({2-[4-(5-chlor-2-cyanophenyl)-5-methoxy-2-oxopyridin-1(2H)-yl]butanoyl}amino)benzoyl]-L-serin (enantiomerenreines Diastereomer)

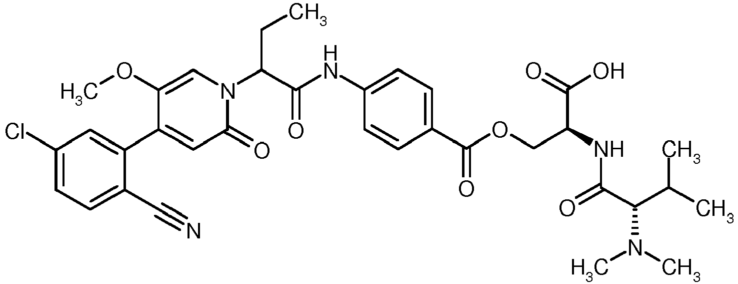

Eine Lösung von 158 mg (90% Reinheit, 0.19 mmol) Benzyl-*N,N*-dimethyl-*L*-valyl-*O*-[4-({2-[4-(5-chlor-2-cyanophenyl)-5-methoxy-2-oxopyridin-1(2*H*)-yl]butanoyl}amino)benzoyl]-*L*-serinat (enantiomerenreines Diastereomer) in 10 ml Ethanol wurde in Gegenwart von 20 mg Palladium (10%ig auf Aktivkohle) 1.5 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 32 mg (25% d. Th.)
LC/MS [Methode 1]: Rₜ = 0.76 min; MS (ESIpos): m/z = 680 (M+H)⁺,
¹H-NMR (400 MHz, DMSO-d₆): δ [ppm] = 12.9 (br. s, 1H), 10.83 (s, 1H), 8.3 (br. s, 1H), 8.00 (d, 1H), 7.94 (d, 2H), 7.81-7.70 (m, 4H), 7.49 (s, 1H), 6.54 (s, 1H), 5.63 (dd, 1H), 4.80-4.71 (m, 1H), 4.53 (dd, 1H), 4.46 (dd, 1H), 3.69 (s, 3H), 2.27-2.09 (m, 8H), 2.01-1.88 (m, 1H), 0.91 (t, 3H), 0.88 (d, 3H), 0.81 (d, 3H).

### Beispiel 10

### (3S)-3-{[4-({2-[4-(5-Chlor-2-cyanophenyl)-5-methoxy-2-oxopyridin-1(2H)-yl]butanoyl}amino)-benzoyl]oxy}-L-prolin (enantiomerenreines Diastereomer)

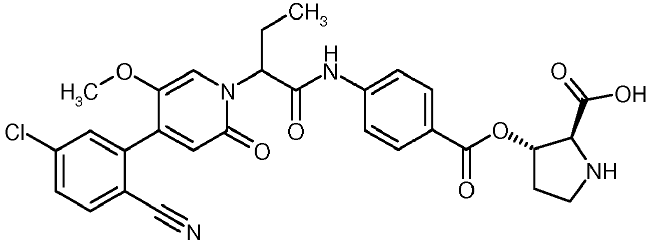

Eine Lösung von 74 mg (31% Reinheit, 34 µmol) Benzyl-(3*S*)-3-{[4-({2-[4-(5-chlor-2-cyanophenyl)-5-methoxy-2-oxopyridin-1(2*H*)-yl]batanoyl}amino)benzoyl]oxy}-*L*-prolinat (enantiomerenreines Diastereomer) in 2 ml Ethanol wurde in Gegenwart von 7 mg Palladium (10%ig auf Aktivkohle) 1.0 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 4 mg (91% Reinheit, 18% d. Th.)
LC/MS [Methode 1]: Rₜ = 0.80 min; MS (ESIpos): m/z = 579 (M+H)⁺,
¹H-NMR (400 MHz, DMSO-d₆): δ [ppm] = 10.84 (s, 1H), 8.03-7.98 (m, 3H), 7.79 (d, 2H), 7.76-7.71 (m, 2H), 7.49 (s, 1H), 6.54 (s, 1H), 5.66-5.60 (m, 2H), 3.94 (s, 1H), 3.69 (s, 3H), 3.53-3.45 (m, 1H), 2.26-2.12 (m, 2H), 2.11-1.91 (m, 2H), 0.91 (t, 3H).

### Beispiel 11

### (4R)-4-{[4-({2-[4-(5-Chlor-2-cyanophenyl)-5-methoxy-2-oxopyridin-1(2H)-yl]butanoyl}amino)-benzoyl]oxy}-D-prolin (enantiomerenreines Diastereomer)

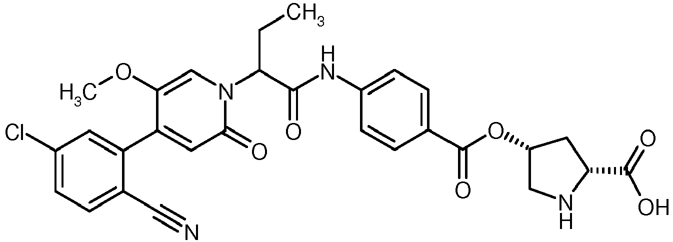

Eine Lösung von 71 mg (94% Reinheit, 0.10 mmol) Benzyl-(4*R*)-4-{[4-({2-[4-(5-chlor-2-cyanophenyl)-5-methoxy-2-oxopyridin-1(2*H*)-yl]butanoyl}amino)benzoyl]oxy}-*D*-prolinat (enantiomerenreines Diastereomer) in 2 ml Ethanol wurde in Gegenwart von 7 mg Palladium (10%ig auf Aktivkohle) 2 h bei RT und Normaldruck hydriert, nochmals mit 7 mg Palladium (10%ig auf Aktivkohle) und 1 ml Tetrahydrofuran versetzt und weiter 1 h bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 37 mg (92% Reinheit, 59% d. Th.)
LC/MS [Methode 2]: Rₜ = 2.23 min; MS (ESIpos): m/z = 579 (M+H)⁺,
¹H-NMR (400 MHz, DMSO-d₆): δ [ppm] = 10.90 (s, 1H), 10.2 (br. s, 1H), 9.0 (br. s, 1H), 8.04 (d, 2H), 8.0 (d, 1H), 7.80 (d, 2H), 7.76-7.71 (m, 2H), 7.48 (s, 1H), 6.55 (s, 1H), 5.63 (dd, 1H), 5.54 (t, 1H), 4.6 (br. s, 1H), 3.69 (s, 3H), 2.61-2.55 (m, 1H), 2.48-2.35 (m, 2H), 2.28-2.11 (m, 2H), 0.91 (t, 1H).

### Beispiel 12

### (2S)-3-{[4-({2-[4-(5-Chlor-2-cyanophenyl)-5-methoxy-2-oxopyridin-1(2H)-yl]butanoyl}amino)-benzoyl]oxy}-2-(pyrrolidin-1-yl)propansäure (enantiomerenreines Diastereomer)

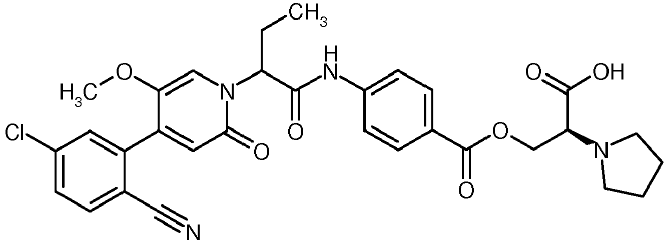

Eine Lösung von 73 mg (0.11 mmol) (2S)-3-(Benzyloxy)-3-oxo-2-(pyrrolidin-1-yl)propyl-4-({2-[4-(5-chlor-2-cyanophenyl)-5-methoxy-2-oxopyridin-1(2*H*)-yl]butanoyl}amino)benzoat (enantiomerenreines Diastereomer) in 10 ml Ethanol wurde in Gegenwart von 10 mg Palladium (10%ig auf Aktivkohle) 45 min bei RT und Normaldruck hydriert. Anschließend wurde das Reaktionsgemisch über Celite filtriert und der Rückstand mit Ethanol gewaschen. Die vereinigten Filtrate wurden im Vakuum eingeengt. Der Rückstand wurde mittels RP-HPLC (Reprosil C18, Acetonitril-Wasser-Gradient) aufgereinigt. Ausbeute: 22 mg (35% d. Th.)
LC/MS [Methode 1]: Rₜ = 0.78 min; MS (ESIpos): m/z = 607 (M+H)⁺,
¹H-NMR (400 MHz, DMSO-d₆): δ [ppm] = 10.85 (s, 1H), 8.00 (d, 1H), 7.97 (d, 2H), 7.79 (d, 2H), 7.76-7.70 (m, 2H), 7.50 (s, 1H), 6.54 (s, 1H), 5.63 (dd, 1H), 4.58 (d, 2H), 3.72-3.66 (m, 1H), 3.69 (s, 3H), 3.25-3.15 (m, 2H), 3.10-3.01 (m, 2H), 2.26-2.12 (m, 2H), 1.89-1.80 (m, 4H), 0.91 (t, 3H).

### Beispiel 13

### 1-Methylpiperidin-4-yl-4-({2-[4-(5-chlor-2-cyanophenyl)-5-methoxy-2-oxopyridin-1(2H)-yl]butanoyl} amino)benzoat (Enantiomer)

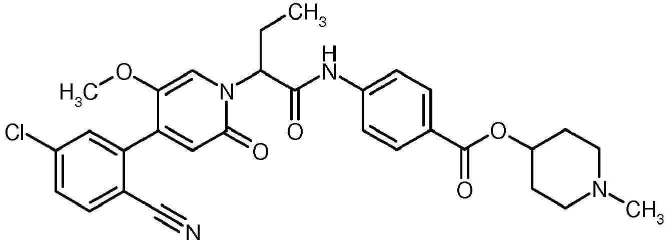

Eine Lösung von 70 mg (0.15 mmol) 4-({2-[4-(5-Chlor-2-cyanophenyl)-5-methoxy-2-oxopyridin-1(2*H*)-yl]butanoyl}amino)benzoesäure (Enantiomer 2) in 1 ml Dichlormethan wurde unter Argon bei -20°C mit 22 mg (0.19 mmol, 1.25 eq.) 1-Methylpiperidin-4-ol, 5 mg (37 µmol, 0.25 eq.) 4-Dimethylaminopyridin, 33 µl (0.19 mmol, 1.25 eq.) *N,N*-Diisopropylethylamin und zuletzt 36 mg (0.19 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Das Reaktionsgemisch wurde bei RT mit weiteren 9 mg (75 mmol, 0.5 eq.) versetzt und eine weitere Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 16 mg (19% d. Th.)
LC/MS [Methode 1]: Rₜ = 0.74 min; MS (ESIpos): m/z = 563 (M+H)⁺,
¹H-NMR (400 MHz, DMSO-d₆): δ [ppm] = 10.83 (s, 1H), 8.00 (d, 1H), 7.94 (d, 2H), 7.78 (d, 2H), 7.76-7.70 (m, 2H), 7.49 (s, 1H), 6.54 (s, 1H), 5.64 (dd, 1H), 4.95-4.85 (m, 1H), 3.69 (s, 3H), 2.65-2.55 (m, 2H), 2.28-2.11 (m, 4H), 2.19 (s, 3H), 1.96-1.86 (m, 2H), 1.77-1.65 (m, 2H), 0.91 (t, 3H).

### Beispiel 14

### 2-(4-Methylpiperazin-1-yl)ethyl-4-({2-[4-(5-chlor-2-cyanophenyl)-5-methoxy-2-oxopyridin-1(2H)-yl]butanoyl}amino)benzoat (Enantiomer 2)

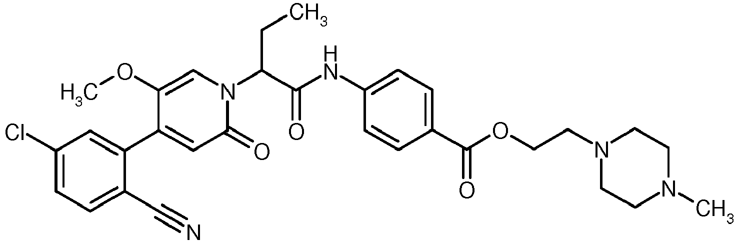

Eine Lösung von 70 mg (0.15 mmol) 4-({2-[4-(5-Chlor-2-cyanophenyl)-5-methoxy-2-oxopyridin-1(2*H*)-yl]butanoyl}amino)benzoesäure (Enantiomer 2) in 2 ml Dichlormethan wurde unter Argon bei -20°C mit 27 mg (0.19 mmol, 1.25 eq.) 2-(4-Methylpiperazin-1-yl)ethanol, 18 mg (0.15 mmol, 1.0 eq.) 4-Dimethylaminopyridin, 33 µl (0.19 mmol, 1.25 eq.) *N,N*-Diisopropylethylamin und zuletzt 36 mg (0.19 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 54 mg (61% d. Th.)
LC/MS [Methode 1]: Rₜ = 0.76 min; MS (ESIpos): m/z = 592 (M+H)⁺,
¹H-NMR (400 MHz, DMSO-d₆): δ [ppm] = 10.83 (s, 1H), 8.00 (d, 1H), 7.93 (d, 2H), 7.78 (d, 2H), 7.76-7.70 (m, 2H), 7.49 (s, 1H), 6.54 (s, 1H), 5.64 (dd, 1H), 4.34 (t, 2H), 3.69 (s, 3H), 2.66 (t, 2H), 2.48-2.40 (m, 4H), 2.35-2.24 (m, 4H), 2.24-2.15 (m, 2H), 2.13 (s, 3H), 0,91 (t, 3H).

### Beispiel 15

### 2-(Piperidin-4-yl)ethyl-4-({2-[4-(5-chlor-2-cyanophenyl)-5-methoxy-2-oxopyridin-1(2H)-yl]butanoyl}amino)benzoat (Enantiomer)

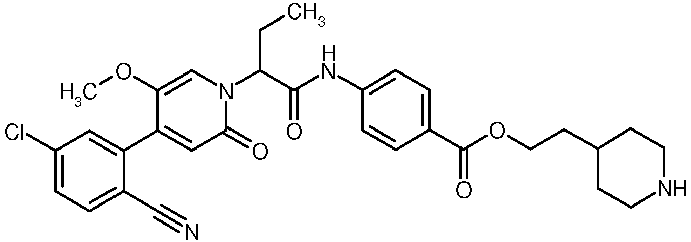

Eine Lösung von 66 mg (98 µmol) 4-(2-{[4-({2-[4-(5-Chlor-2-cyanophenyl)-5-methoxy-2-oxopyridin-1(2*H*)-yl]batanoyl}amino)benzoyl]oxy}ethyl)piperidin-1-carbonsäure-*tert*.-butylester (Enantiomer) in 2 ml Dichlormethan wurde bei 0°C mit 151 µl (1.96 mmol, 20 eq.) Trifluoressigsäure versetzt und 1.5 h bei RT gerührt. Das Reaktionsgemisch wurde im Vakuum eingeengt und der Rückstand mittels RP-HPLC (Reprosil C18, Acetonitril-Wasser-Gradient) aufgereinigt. Ausbeute: 32 mg (56% d. Th.)
LC/MS [Methode 2]: Rₜ = 2.27 min; MS (ESIpos): m/z = 577 (M+H)⁺,
¹H-NMR (400 MHz, DMSO-d₆): δ [ppm] = 10.84 (s, 1H), 8.00 (d, 1H), 7.94 (d, 2H), 7.79 (d, 2H), 7.76-7.71 (m, 2H), 7.49 (s, 1H), 6.54 (s, 1H), 5.63 (dd, 1H), 4.30 (t, 2H), 3.69 (s, 3H), 3.26 (d, 2H), 2.87 (t, 2H), 2.26-2.10 (m, 2H), 1.87 (d, 2H), 1.81-1.63 (m, 3H), 1.40-1.26 (m, 2H), 0.91 (t, 3H).

### Beispiel 16

### 2-(Pyrrolidin-1-yl)ethyl-4-({2-[4-(5-chlor-2-cyanophenyl)-5-methoxy-2-oxopyridin-1(2H)-yl]butanoyl}amino)benzoat (Enantiomer 2)

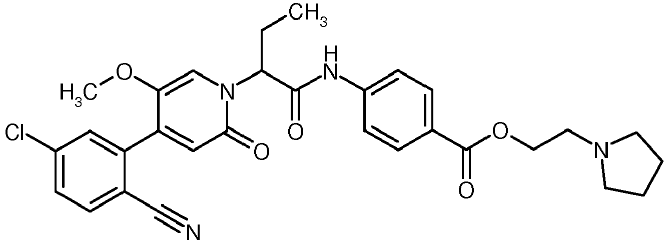

Eine Lösung von 70 mg (0.15 mmol) 4-({2-[4-(5-Chlor-2-cyanophenyl)-5-methoxy-2-oxopyridin-1(2*H*)-yl]butanoyl}amino)benzoesäure (Enantiomer 2) in 2 ml Dichlormethan wurde unter Argon bei -20°C mit 22 mg (0.19 mmol, 1.25 eq.) 2-(Pyrrolidin-1-yl)ethanol, 18 mg (0.15 mmol, 1.0 eq.) 4-Dimethylaminopyridin, 33 µl (0.19 mmol, 1.25 eq.) *N,N*-Diisopropylethylamin und zuletzt 36 mg (0.19 mmol, 1.25 eq.) 1-Ethyl-3-(3-dimethylaminopropyl)carbodiimid-Hydrochlorid versetzt. Das Reaktionsgemisch wurde 20 min bei -20°C gerührt, auf RT kommen gelassen und über Nacht bei RT gerührt. Nach Zugabe von Wasser/Dichlormethan und Phasentrennung wurde die wässrige Phase zweimal mit Dichlormethan extrahiert. Die vereinigten, organischen Phasen wurden mit gesättigter, wässriger Natriumchlorid-Lösung gewaschen, getrocknet (Natriumsulfat), filtriert und im Vakuum eingeengt. Der Rückstand wurde mittels RP-HPLC (Reprosil C18, Acetonitril-Wasser-Gradient) aufgereinigt. Ausbeute: 30 mg (36% d. Th.)
LC/MS [Methode 1]: Rₜ = 0.74 min; MS (ESIpos): m/z = 563 (M+H)⁺,
¹H-NMR (400 MHz, DMSO-d₆): δ [ppm] = 10.83 (s, 1H), 8.00 (d, 1H), 7.94 (d, 2H), 7.78 (d, 2H), 7.76-7.70 (m, 2H), 7.49 (s, 1H), 6.54 (s, 1H), 5.64 (dd, 1H), 4.34 (t, 2H), 3.69 (s, 3H), 2.76 (t, 2H), 2.26-2.11 (m, 2H), 1.72-1.62 (m, 4H), 0.91 (t, 3H).

### B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7.4; 100 mM Natriumchlorid; 5 mM Calciumchlorid; 0,1% bovines Serumalbumin) und 20 µl Faktor XIa der Firma Kordia (0.45 nM in Assaypuffer) hinzugefügt. Nach 15 min Inkubation wird die Enzymreaktion durch Zugabe von 20 µl des in Assaypuffer gelösten Faktor XIa Substrates Boc-Glu(OBzl)-Ala-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet. Wirkdaten aus diesem Test sind in der folgenden Tabelle A aufgeführt:

**Tabelle A**

| **Beispiel-Nr.** | **IC₅₀ [nM]** | | **Beispiel-Nr.** | **IC₅₀ [nM]** |
|---|---|---|---|---|
| 1 | 2.0 | | 2 | 54 |
| 3 | 82 | | 4 | 54 |
| 5 | 60 | | 6 | 65 |
| 7 | 91 | | 8 | 110 |
| 9 | 63 | | 10 | 14 |
| 11 | 260 | | 12 | 26 |
| 13 | 230 | | 14 | 120 |
| 15 | 230 | | 16 | 33 |

### a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l NaCl, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Faktor Xa und Trypsin, 5 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 µM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope Software" wird das Thrombogramm berechnet und graphisch dargestellt. Die folgenden Parameter werden berechnet: lag time, time to Peak, Peak, ETP (endogenous thrombin potential) und start tail.

### a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

### a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7,4; 100 mM Natriumchlorid-Lösung; 5 mM Calciumchlorid-Lösung; 0.1% bovines Serumalbumin) und 20 µl Plasma-Kallikrein der Firma Kordia (0.6 nM in Assaypuffer) hinzugefügt. Nach 15 min Inkubation wird die Enzymreaktion durch Zugabe von 20 µl des in Assaypuffer gelösten Substrates H-Pro-Phe-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

**Tabelle B**

| **Beispiel-Nr.** | **IC₅₀ [nM]** | | **Beispiel-Nr.** | **IC₅₀ [nM]** |
|---|---|---|---|---|
| 1 | 9.1 | | 2 | 93 |
| 3 | 60 | | 4 | 92 |
| 5 | 35 | | 6 | 44 |
| 7 | 52 | | 8 | 55 |
| 9 | 51 | | 10 | 91 |
| 11 | 280 | | 12 | 43 |
| 13 | 120 | | 14 | 38 |
| 15 | 44 | | 16 | 40 |

### a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der entstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentrationen der Verbindungen sind 1 x 10⁻¹⁰ bis 1 x 10⁻⁶ M.

### a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 µM Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### b) Bestimmung der antithrombotischen Wirkung (in vivo)

### b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden intravenös über die Vena femoralis den anästhesierten Tieren jeweils 5 min vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

### c) Bestimmung der Wirkung auf die Extravasation/Ödembildung und/oder Neovaskularisierung im Auge ( in vivo)

### c.1) Testung der Wirksamkeit von Substanzen im Laser-induzierten choroidalen Neovaskularisierungs- Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Kochsalzlösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera (z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in µm² bzw. µm³) wird durch morphometrische Analyse mittels Axiovision 4.6 Software berechnet.

### c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.

Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57B1 / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Kochsalzlösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

### C) Bestimmung von Stabilität, Löslichkeit und Freisetzungsverhalten

### a) Messung der Stabilität in Puffer bei verschiedenen pH-Werten (HPLC Detektion)

Testbeschreibung: 0.3 mg der Testverbindung werden in 0.1 ml Dimethylsulfoxid gelöst und mit 0.4 ml Acetonitril versetzt. Um eine komplette Auflösung zu erreichen, wird das Probengemisch für ca. 20 Sekunden im Ultraschallbad behandelt. Anschließend werden 1.0 ml des entsprechenden Puffers hinzugefügt und das Probengemisch erneut im Ultraschallbad behandelt.

### Eingesetzte Pufferlösungen

pH 4.0: Fluka, Bestellnummer 33643 (11.76 g Zitronensäure, 2.57 g Natriumchlorid und 2.72 g Natriumhydroxid)
pH 7.4: 90 g Natriumchlorid, 13.61 g Kaliumdihydrogenphosphat und 83.35 g einer wässrigen, 1M Lösung von Natriumhydroxid werden auf 1 Liter mit Millipore-Wasser aufgefüllt und dann im Verhältnis 1:10 verdünnt.

10 µl Portionen der Probenlösungen werden mittels HPLC zu verschiedenen Zeitpunkten (0 h, 1 h, 2 h, 4 h und 24 h) bei 37°C analysiert. Quantifiziert wird über die Peakflächen in Prozent.

HPLC Methode: Agilent 1100 mit DAD (G1315B), binäre Pumpe (G1312A), Autosampler (G1329A), Säulenofen (G1316A), Thermostat (G1330B); Säule: Kromasil 100 C18, 250 mm x 4 mm, 5 µm; Säulentemperatur: 37°C; Flussrate: 1.5 ml/min; UV-Detektion: 210 nm; Injektionsvolumen: 10 µl; Eluent A: 5 ml Perchlorsäure/Liter Wasser; Eluent B: Acetonitril; Gradient: 0.0 min: 98% A, 2% B → 3.0 min: 85% A, 15% B → 8.0 min: 50% A, 50% B → 16.0 min: 50% A, 50% B → 20.0 min: 10% A, 90% B → 21.0 min: 10% A, 90% B → 24.0 min: 98% A, 2% B → 25.0 min: 98% A, 2% B.

Ermittelt wird das Verhältnis der Peakflächen (F) zu verschiedenen Zeitpunkten im Verhältnis zur Peakfläche am Startpunkt.

### b) Messung der Stabilität in vitro in Ratten- und Humanplasma (HPLC Detektion)

Testbeschreibung: 1 mg der Testverbindung wird in einem Gemisch von 0.5 ml Acetonitril/Dimethylsufoxid (9:1) gelöst. 20 µl dieser Probenlösung werden zu 1.0 ml heparinisiertem, 37°C warmen Plasma (Wistar-Rattenplasma oder Humanplasma) gegeben. Zu verschiedenen Zeitpunkten (0, 30, 60, 90, 120 und 240 min) wird ein Aliquot von 100 µl der Probenlösung mit 300 µl Acetonitril gemischt. Dieser Schritt beendet die enzymatische Reaktion mit den Plasmaenzymen. Die Acetonitril-Probenlösung wird anschließend für 10 min bei 5000 Umdrehungen pro Minute zentrifugiert. Die Menge an Testverbindung im Überstand wird durch Quantifizierung mittels HPLC bestimmt.

HPLC Methode: Agilent 1100 mit DAD (G1315B), binäre Pumpe (G1312A), Autosampler (G1329A), Säulenofen (G1316A), Thermostat (G1330B); Säule: Kromasil 100 C18, 250 mm x 4 mm, 5 µm; Säulentemperatur: 45°C; Flussrate: 1.5 ml/min; UV-Detektion: 230 nm; Injektionsvolumen: 20 µl; Eluent A: 5 ml Perchlorsäure/Liter Wasser; Eluent B: Acetonitril; Gradient: 0.0 min: 98% A, 2% B → 3.0 min: 85% A, 15% B → 8.0 min: 50% A, 50% B → 16.0 min: 50% A, 50% B → 20.0 min: 10% A, 90% B → 21.0 min: 10% A, 90% B → 24.0 min: 98% A, 2% B → 25.0 min: 98% A, 2% B.

Ermittelt wird das Verhältnis der Peakflächen (F) zu verschiedenen Zeitpunkten im Verhältnis zur Peakfläche am Startpunkt.

### c) Messung der Löslichkeit von Testverbindungen in PBS Puffer bei pH 6.5

Testbeschreibung: 2 mg der festen Form einer Testverbindung werden in 40 µl Dimethylsulfoxid (c = 50 g/1) gelöst. 10 µl dieser Probenlösung werden zu 990 µl PBS-Puffer pH 6.5 (c = 500 µg/ml) in einer Mikrotiterplatte gegeben. Um Gleichgewichtsbedingungen zu erreichen, wird diese Mikrotiterplatte mit 96 verschiedenen Probenlösungen für 24 h bei 25°C geschüttelt. Anschließend werden die Probenlösungen oder -suspensionen in Zentrifugenröhrchen überführt. Ungelöstes Material wird durch Ultrazentrifugation über 30 min bei 114000 Umdrehungen pro Minute abgetrennt. Die im Überstand gelöste Testverbindung wird mit einem Gemisch von Acetonitril/Wasser (8:2) verdünnt und deren Gehalt mittels LC-MSMS Detektion bestimmt.

Zur Erstellung einer Kalibrationskurve werden 10 µl der Dimethylsulfoxid-Stammlösung zu 823 µl Dimethylsulfoxid (c = 600 µg/ml) gegeben. Der Gehalt an Testverbindung wird für 5 verschiedene Konzentrationsstufen bestimmt.

LC-MSMS Apparatur: AB Sciex TRIPLE QUAD 4500, Agilent 1260 Pumpe (G1312B Infinity), Entgasser (G4225a Infinity), Thermostat (G1316C Infinity), CTC Analytics PAL Injection System THC-xt.

HPLC Methode: Säule: Waters OASIS HLB, 2.1 mm x 20 mm, 25 µm; Säulentemperatur: 30°C; Flussrate: 2.5 ml/min; Injektionsvolumen: 5 µl; Fluss-Auftrennung: 1:20; Eluent A: 0.5 ml Ameisensäure (50%ig in Wasser)/Liter Wasser; Eluent B: 0.5 ml Ameisensäure (50%ig in Wasser)/Liter Acetonitril; Gradient: 0 min: 90% A, 10% B → 0.5 min: 5% A, 95% B → 0.84 min: 5% A, 95% B → 0.85 min: 90% A, 10% B → 1.50 min: 90% A, 10% B.

MS Methode: Flow Injection Analysis (FIA) zur Optimierung, Multiple Reaction Monitoring (MRM) zur Quantifizierung; Säule: Edelstahl-Kapillarrohr; Kapillar-Temperatur: 22°C; Fluss: 0.25 ml/min; Injektionsvolumen: 5 µl; Eluent A: 0.5 ml Ameisensäure (50%ig in Wasser)/Liter Wasser; Eluent B: 0.5 ml Ameisensäure (50%ig in Wasser)/Liter Acetonitril

### d) Löslichkeitsscreening von Substanzen in für i.v. Applikation geeigneten Medien

Versuchsdurchführung: Pro Testverbindung und Medium werden jeweils 0.5 bis 0.6 mg exakt eingewogen (8 Einwaagen). Es wird jeweils soviel Medium zu der Probe gegeben, dass man eine Konzentration von 500 µg/ml erhält. Diese Probenlösung wird für 24 h bei RT und 1400 Umdrehungen pro Minute geschüttelt.

Eine weitere Einwaage von 0.5 bis 0.6 mg wird für die Dimethylsulfoxid-Kalibrationslösung (Einwaage 9) benötigt. Diese Probe wird mit DMSO bis zu einer Konzentration von 600 µg/ml aufgefüllt. Aus dieser Stammlösung werden 2 Kalibrationslösungen angesetzt. In einem 2 ml-HPLC-Gefäß werden 1000 µl Dimethylsulfoxid vorgelegt und 34.4 µl der Stammlösung hinzu pipettiert (c = 20 µg/ml). Von dieser Lösung (c = 20 µg/ml) werden 714 µl in ein weiteres 2 ml-HPLC-Gefäß gegeben, in dem sich 500 µl Dimethylsulfoxid befinden (c = 2.5 µg/ml).

Nach dem Schütteln der Probenlösungen werden jeweils 220 µl des Überstandes in ein Zentrifugenröhrchen überführt und bei 42000 Umdrehungen pro Minute für 30 min zentrifugiert.

Dann werden 160 µl des Überstandes abgenommen, mit Dimethylsulfoxid im Verhältnis 1:5 und 1:100 verdünnt und in 2 ml-HPLC-Gläschen überführt. Die beiden Kalibrierlösungen und die verdünnten Probenlösungen werden per HPLC analysiert. Quantifiziert wird über die entsprechenden Peakflächen.

Lösemittel: Destilliertes Wasser, Perchlorsäure (Fluka; 77227), Acetonitril (Leitungsware), DMSO (Merck; 8.02912.2500)

### Medien

Natriumchlorid 0.9%ig in Wasser
PEG400/Ethanol/Wasser 30/10/60
Solutol/Ethanol/Wasser 20/10/70
Citratpuffer pH 4
PBS-Puffer pH 7
Tris-Puffer pH 8.5
HP-β-Cyclodextrin 10%ig in Wasser
D-Mannitol 5%ig in Wasser

Geräte: Agilent 1100 oder vergleichbares Gerät mit UV-Detektion, variable Wellenlänge (z.B. Dioden-Array), Ultraschallbad, Vibromix von Janke & Kunkel, Thermomixer von Eppendorf

HPLC-Methode: Säule: Nucleodur 100 C18 ec, 50 mm x 2 mm, 3 µm; Säulentemperatur: 30°C; Flussrate: 0.75 ml/min; UV-Detektion: 210 nm; Injektionsvolumen: 60 µl; Eluent A: 5 ml Perchlorsäure/Liter Wasser; Eluent B: Acetonitril; Gradient: 0.0 min: 98% A, 2% B → 0.5 min: 98% A, 2% B → 4.55 min: 10% A, 90% B → 6.55 min: 10% A, 90% B → 6.75 min: 98% A, 2% B → 7.55 min: 98% A, 2% B.

### e) Hepatozytenassay zur Bestimmung der metabilischen Stabilität

Metabolische Stabilitäten neuer Testverbindungen gegenüber Hepatozyten werden bestimmt, indem die Verbindungen bei niedrigen Konzentrationen (bevorzugt unter luM) und bei niedrigen Zellzahlen (bevorzugt bei 1*10^6Zellen/ml) inkubiert werden, um möglichst lineare kinetische Bedingungen im Versuch sicherzustellen. Sieben Proben aus der Inkubationslösung werden in einem festgelegten Zeitraster für die LC-MS Analytik entnommen, um die Halbwertszeit (den Abbau) der Verbindung zu bestimmen. Aus dieser Halbwertszeit werden unterschiedliche "Clearance - Parameter (CL)" (s.u.) und "Fmax"- Werte (s.u.) berechnet.

Die CL und Fmax - Werte stellen ein Maß für den "Phase 1" und "Phase 2" Metabolismus der Verbindung in den Hepatozyten dar. Um den Einfluss des organischen Lösungsmittels auf die Enzyme in den Inkubationsansätzen möglichst klein zu halten, werden deren Konzentrationen im allgemeinen auf 1% (Acetonitril) bzw. auf 0.1% (DMSO) begrenzt.

Für alle Spezies und Rassen wird mit einer Hepatozytenzellzahl in der Leber von 1.1*10^8 Zellen/ g Leber gerechnet. CL Parameter deren Berechnung auf Halbwertszeiten beruhen, die über die Inkubationszeit hinausgehen (üblicherweise 90 Minuten), können nur als grobe Richtwerte angesehen werden.

Die berechneten Parameter und deren Bedeutung sind:

| (QH = speziesspezifischerLeberblutfluss) | |
|---|---|
| **Fmax-well-stirred [%]:** | maximal mögliche Bioverfügbarkeit nach oraler Applikation |
| Berechnung: | (1-CL_{blood} well-stirred /QH)*100 |
| **CL_{blood} well-stirred [L/(h*kg)]:** | berechnete Blut Clearance (Well-stirred Modell) |
| Berechnung: | (QH* CL'_{intrinsic}) / (QH+ CL'_{intrinsic}) |
| **CL'_{intrinsic} [ml/(min*kg)]:** | maximale Fähigkeit der Leber (der Hepatozyten) eine Verbindung zu metabolisieren, unter der Annahme, dass der Leberblutfluss nicht geschwindigkeitslimitierend ist |
| Berechnung: | CL'_{intrinsic,apparent} * speziesspezifischer Hepatozytenzahl [1.1*10^8/g Leber]* speziesspezifischem Lebergewicht [g/kg] |
| **CL'_{intrinsic},ₐₚₚₐᵣₑₙₜ [ml/(min*mg)]:** | "normiert" die Eliminationskonstante, indem diese durch die eingesetzte Hepatozytenzellzahl x (x*10^6/ml) dividiert wird |
| Berechnung: | kₑₗ[1/min] / (Zellzahl [x*10^6] / Inkubationsvolumen [ml]) |

### f) i.v.-Pharmakokinetik in Wistar-Ratten

Am Tag vor der Substanzgabe wird den Versuchstieren (männliche Wistar-Ratten, Körpergewicht 200-250 g) unter Isofluran®-Narkose ein Katheter für die Blutgewinnung in die Vena Jugularis implantiert.

Am Versuchstag wird eine definierte Dosis der Prüfsubstanz als Lösung mit einer Hamilton®-Glasspritze in die Schwanzvene appliziert (Bolusgabe, Applikationsdauer <10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ, T_{1/2} (Halbwertszeit) und CL (Clearance) erfolgt aus den gemessenen Plasmakonzentrationen.

### D) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchloridlösung, Glucoselösung 5% und/oder Polyethylenglykol 400 / Wasser 30% m/m) gelöst. Die Lösung wird steril filtriert und in sterile und pyrogenfreie Injektionsbehältnisse abgefüllt.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

## Patentansprüche

1. Verbindung der Formel 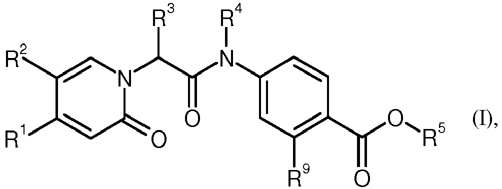 in welcher
R¹ für eine Gruppe der Formel 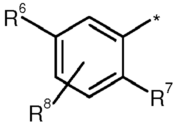 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
R² für Wasserstoff, Brom, Chlor, Fluor, Cyano, C₁-C₃-Alkyl, Difluormethyl, Trifluormethyl, 1,1-Difluorethyl, 2,2-Difluorethyl, 2,2,2-Trifluorethyl, C₁-C₃-Alkoxy, Difluormethoxy, Trifluormethoxy, 1,1-Difluorethoxy, 2,2-Difluorethoxy, 2,2,2-Trifluorethoxy, Methylcarbonyl oder Cyclopropyl steht,
R³ für Wasserstoff, C₁-C₅-Alkyl, C₁-C₄-Alkoxy, Difluormethyl, Trifluormethyl, 1,1-Difluorethyl, 1,1,2,2,2-Pentadeuteroethyl, 3,3,3-Trifluor-2-hydroxyprop-1-yl, 3,3,3-Trifluor-2-methoxyprop-1-yl, 3,3,3-Trifluor-2-ethoxyprop-1-yl, Prop-2-in-1-yl, Cyclopropyloxy oder Cyclobutyloxy steht,
wobei Alkyl substituiert sein kann mit einem Substituenten ausgewählt aus der Gruppe bestehend aus Fluor, Cyano, Hydroxy, Difluormethyl, Trifluormethyl, Methoxy, Ethoxy, Difluormethoxy, Trifluormethoxy, C₃-C₆-Cycloalkyl, 4- bis 6-gliedriges Oxo- Heterocyclyl, 1,4-Dioxanyl, Oxazolyl, Phenyl und Pyridyl,
worin Cycloalkyl substituiert sein kann mit 1 bis 2 Substituenten unabhängig voneinander ausgewählt aus der Gruppe bestehend aus Fluor, Hydroxy, Methyl, Ethyl, Methoxy, Ethoxy, Difluormethyl, Trifluormethyl, Difluormethoxy und Trifluormethoxy,
und
worin Oxo-Heterocyclyl substituiert sein kann mit 1 bis 2 Substituenten unabhängig voneinander ausgewählt aus der Gruppe bestehend aus Oxo, Fluor, Methyl, Ethyl, Difluormethyl und Trifluormethyl,
R⁴ für Wasserstoff steht,
R⁵ für eine Gruppe der Formel 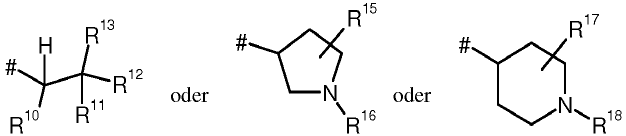 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, -N⁺(CH₃)₃, ein 5- oder 6-gliediges stickstoffhaltiges Heterocyclyl oder -NH(C=O)R¹⁴ steht,
worin Heterocyclyl substituiert sein kann mit 1 bis 2 Substituenten unabhängig voneinander ausgewählt aus der Gruppe bestehend aus C₁-C₃-Alkyl,
und
worin
R¹⁴ für C₁-C₄-Alkyl, ein 5- oder 6-gliediges stickstoffhaltiges Heterocyclyl oder Cyclopropyl steht,
worin Alkyl substituiert sein kann mit einem Substituenten ausgewählt aus der Gruppe bestehend aus Amino, Methylamino, Dimethylamino und einem 5- oder 6-gliedigen stickstoffhaltigen Heterocyclyl,
worin der Heterocyclyl seinerseits substituiert sein kann mit 1 bis 2 Substituenten unabhängig voneinander ausgewählt aus der Gruppe bestehend aus C₁-C₃-Alkyl,
und
worin Heterocyclyl substituiert sein kann mit 1 bis 2 Substituenten unabhängig voneinander ausgewählt aus der Gruppe bestehend aus C₁-C₃-Alkyl,
und
worin Cyclopropyl substituiert sein kann mit einem Substituenten ausgewählt aus der Gruppe bestehend aus Amino, Methylamino und Dimethylamino,
R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
R⁹ für Wasserstoff oder Fluor steht,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 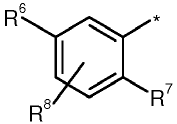 steht,
wobei * die Anknüpfstelle an den Oxopyridinring ist,
R⁶ für Chlor steht,
R⁷ für Cyano, Difluormethyl oder Difluormethoxy steht,
R⁸ für Wasserstoff oder Fluor steht,
R² für Chlor, Cyano, Ethoxy, Methoxy oder Difluormethoxy steht,
R³ für Methyl, Ethyl, n-Propyl, 2-Methyl-prop-1-yl oder n-Butyl steht,
wobei Methyl substituiert sein kann mit einem Substituenten ausgewählt aus der Gruppe bestehend aus Cyclopropyl, Cyclobutyl, Cyclohexyl, Tetrahydrofuranyl, Tetrahydro-2H-pyranyl, Oxazolyl und Pyridyl,
worin Cyclobutyl und Cyclohexyl substituiert sein können mit 1 bis 2 Substituenten unabhängig voneinander ausgewählt aus der Gruppe bestehend aus Hydroxy und Methoxy,
und
wobei Ethyl, n-Propyl und n-Butyl substituiert sein können mit einem Substituenten ausgewählt aus der Gruppe bestehend aus Methoxy und Trifluormethoxy,
R⁴ für Wasserstoff steht,
R⁵ für eine Gruppe der Formel 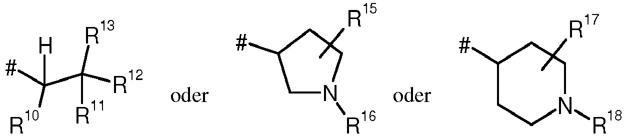 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, -N⁺(CH₃)₃, Pyrrolidinyl, Piperidinyl, Piperazinyl oder für -NH(C=O)R¹⁴ steht,
worin Pyrrolidinyl, Piperidinyl und Piperazinyl substituiert sein können mit einem Substituenten Methyl,
und
worin
R¹⁴ für C₁-C₄-Alkyl, Pyrrolidinyl, Piperidinyl, Piperazinyl oder Cyclopropyl steht,
worin Alkyl substituiert sein kann mit einem Substituenten ausgewählt aus der Gruppe bestehend aus Amino, Methylamino, Dimethylamino, Pyrrolidinyl, Piperidinyl und Piperazinyl,
worin Pyrrolidinyl, Piperidinyl und Piperazinyl ihrerseits substituiert sein können mit einem Substituenten Methyl,
und
worin Pyrrolidinyl, Piperidinyl und Piperazinyl substituiert sein können mit einem Substituenten Methyl,
und
worin Cyclopropyl substituiert sein kann mit einem Substituenten ausgewählt aus der Gruppe bestehend aus Amino, Methylamino und Dimethylamino,
R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
R⁹ für Wasserstoff steht,
oder eines ihrer Salze, ihrer Solvate oder der Solvate ihrer Salze.

3. Verbindung nach einem der Ansprüche 1 oder 2, **dadurch gekennzeichnet, dass**
R¹ für eine Gruppe der Formel 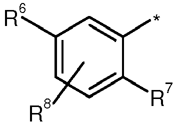 steht,
wobei * die Anknüpfstelle an den Oxopyridinring ist,
R⁶ für Chlor steht,
R⁷ für Cyano steht,
R⁸ für Wasserstoff steht,
R² für Methoxy steht,
R³ für Methyl oder Ethyl steht,
wobei Methyl substituiert sein kann mit einem Substituenten Tetrahydro-2H-pyranyl,
und
wobei Ethyl substituiert sein kann mit einem Substituenten Methoxy,
R⁴ für Wasserstoff steht,
R⁵ für eine Gruppe der Formel 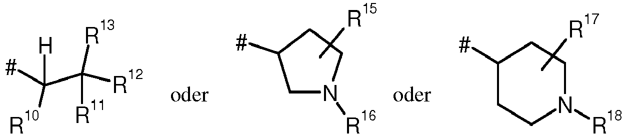 steht,
wobei # die Anknüpfstelle an das Sauerstoffatom ist,
R¹⁰ für Wasserstoff oder Methyl steht,
R¹¹ für Wasserstoff oder Methyl steht,
R¹² für Amino, Pyrrolidinyl, Piperidinyl, Piperazinyl oder für -NH(C=O)R¹⁴ steht,
worin Piperazinyl substituiert sein kann mit einem Substituenten Methyl,
und
worin
R¹⁴ für C₁-C₄-Alkyl, Pyrrolidinyl, Piperidinyl oder Cyclopropyl steht,
worin Alkyl substituiert sein kann mit einem Substituenten ausgewählt aus der Gruppe bestehend aus Amino, Dimethylamino und Pyrrolidinyl,
und
worin Pyrrolidinyl und Piperidinyl substituiert sein können mit einem Substituenten Methyl,
und
worin Cyclopropyl substituiert sein kann mit einem Substituenten Dimethylamino,
R¹³ für Wasserstoff oder Hydroxycarbonyl steht,
R¹⁵ für Wasserstoff oder Hydroxycarbonyl steht,
R¹⁶ für Wasserstoff oder Methyl steht,
R¹⁷ für Wasserstoff oder Hydroxycarbonyl steht,
R¹⁸ für Wasserstoff oder Methyl steht,
R⁹ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** eine Verbindung der Formel 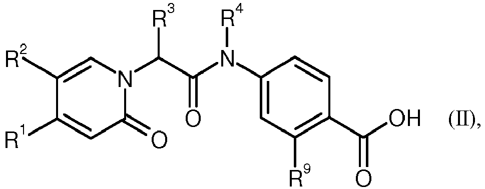 in welcher
R¹, R², R³, R⁴ und R⁹ die in Anspruch 1 angegebene Bedeutung haben,
in der ersten Stufe mit einer Verbindung der Formel
HO-R^{5a} (III),
in welcher
R^{5a} die in Anspruch 1 angegebene Bedeutung von R⁵ hat, wobei die funktionellen Gruppen in R⁵ mit Schutzgruppen, geschützt sein können,
in Gegenwart eines Dehydratisierungsreagenzes umgesetzt wird,
und in einer zweiten Stufe durch Abspaltung der Schutzgruppen zu einer Verbindung der Formel 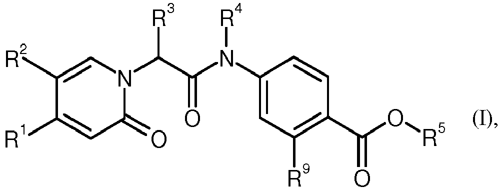 in welcher
R¹, R², R³, R⁴, R⁵ und R⁹ die in Anspruch 1 angegebene Bedeutung haben, umgesetzt wird.

5. Verbindung nach einem der Ansprüche 1 bis 3 zur Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

8. Verwendung einer Verbindung nach einem der Ansprüche 1 bis 3 zur Herstellung eines Arzneimittels zur Behandlung und/oder Prophylaxe von ophthalmologischen Erkrankungen.

9. Verwendung einer Verbindung nach einem der Ansprüche 1 bis 3 zur Herstellung eines Arzneimittels zur Behandlung und/oder Prophylaxe von hereditärem Angioödem oder entzündlichen Erkrankungen des Darms, wie Morbus Crohn oder Colitis ulcerosa.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Behandlung und/oder Prophylaxe von thrombotischen beziehungsweise thromboembolischen Erkrankungen.

12. Arzneimittel nach Anspruch 10 zur Behandlung und/oder Prophylaxe von ophthalmologischen Erkrankungen.

13. Arzneimittel nach Anspruch 10 zur Behandlung und/oder Prophylaxe von hereditärem Angioödem oder entzündlichen Erkrankungen des Darms, wie Morbus Crohn oder Colitis ulcerosa.

## Claims

1. Compound of formula 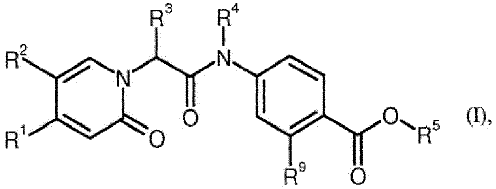 in which
R¹ represents a group of the formula 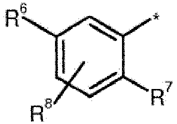 wherein * is the point of attachment to the oxopyridine ring,
R⁶ represents bromine, chlorine, fluorine, methyl, difluoromethyl, trifluoromethyl, methoxy, difluoromethoxy or trifluoromethoxy,
R⁷ represents bromine, chlorine, fluorine, cyano, nitro, hydroxy, methyl, difluoromethyl, trifluoromethyl, methoxy, ethoxy, difluoromethoxy, trifluoromethoxy, ethynyl, 3,3,3-trifluoroprop-1-yn-1-yl or cyclopropyl,
R⁸ represents hydrogen, chlorine or fluorine,
R² represents hydrogen, bromine, chlorine, fluorine, cyano, C₁-C₃-alkyl, difluoromethyl, trifluoromethyl, 1,1-difluoroethyl, 2,2-difluoroethyl, 2,2,2-trifluoroethyl, C₁-C₃-alkoxy, difluoromethoxy, trifluoromethoxy, 1,1-difluoroethoxy, 2,2-difluoroethoxy, 2,2,2-trifluoroethoxy, methylcarbonyl or cyclopropyl,
R³ represents hydrogen, C₁-C₅-alkyl, C₁-C₄-alkoxy, difluoromethyl, trifluoromethyl, 1,1-difluoroethyl, 1,1,2,2,2-pentadeuteroethyl, 3,3,3-trifluoro-2-hydroxyprop-1-yl, 3,3,3-trifluoro-2-methoxyprop-1-yl, 3,3,3-trifluoro-2-ethoxyprop-1-yl, prop-2-yn-1-yl, cyclopropyloxy or cyclobutyloxy,
wherein alkyl can be substituted by a substituent selected from the group consisting of fluorine, cyano, hydroxy, difluoromethyl, trifluoromethyl, methoxy, ethoxy, difluoromethoxy, trifluoromethoxy, C₃-C₆-cycloalkyl, 4- to 6-membered oxo-heterocyclyl, 1,4-dioxanyl, oxazolyl, phenyl and pyridyl,
wherein cycloalkyl can be substituted by from 1 to 2 substituents selected independently of one another from the group consisting of fluorine, hydroxy, methyl, ethyl, methoxy, ethoxy, difluoromethyl, trifluoromethyl, difluoromethoxy and trifluoromethoxy,
and
wherein oxo-heterocyclyl can be substituted by from 1 to 2 substituents selected independently of one another from the group consisting of oxo, fluorine, methyl, ethyl, difluoromethyl and trifluoromethyl,
R⁴ represents hydrogen,
R⁵ represents a group of the formula 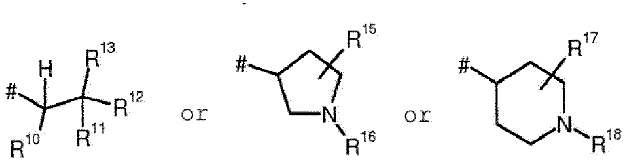 wherein # is the point of attachment to the oxygen atom,
R¹⁰ represents hydrogen or methyl,
R¹¹ represents hydrogen or methyl,
R¹² represents amino, -N⁺(CH₃)₃, a 5- or 6-membered nitrogen-containing heterocyclyl or -NH (C=O)R¹⁴,
wherein heterocyclyl can be substituted by from 1 to 2 substituents selected independently of one another from the group consisting of C₁-C₃-alkyl,
and
wherein
R¹⁴ represents C₁-C₄-alkyl, a 5- or 6-membered nitrogen-containing heterocyclyl or cyclopropyl,
wherein alkyl can be substituted by a substituent selected from the group consisting of amino, methyl-amino, dimethylamino and a 5- or 6-membered nitrogen-containing heterocyclyl,
wherein the heterocyclyl can in turn be substituted by from 1 to 2 substituents selected independently of one another from the group consisting of C₁-C₃-alkyl,
and
wherein heterocyclyl can be substituted by from 1 to 2 substituents selected independently of one another from the group consisting of C₁-C₃-alkyl,
and
wherein cyclopropyl can be substituted by a substituent selected from the group consisting of amino, methylamino and dimethylamino,
R¹³ represents hydrogen or hydroxycarbonyl,
R¹⁵ represents hydrogen or hydroxycarbonyl,
R¹⁶ represents hydrogen or methyl,
R¹⁷ represents hydrogen or hydroxy-carbonyl,
R¹⁸ represents hydrogen or methyl,
R⁹ represents hydrogen or fluorine,
or one of its salts, its solvates or the solvates of its salts.

2. Compound according to Claim 1, **characterized in that**
R¹ represents a group of the formula 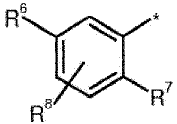 wherein * is the point of attachment to the oxopyridine ring,
R⁶ represents chlorine,
R⁷ represents cyano, difluoromethyl or difluoromethoxy,
R⁸ represents hydrogen or fluorine,
R² represents chlorine, cyano, ethoxy, methoxy or difluoromethoxy,
R³ represents methyl, ethyl, n-propyl, 2-methyl-prop-1-yl or n-butyl,
wherein methyl can be substituted by a substituent selected from the group consisting of cyclopropyl, cyclobutyl, cyclohexyl, tetrahydrofuranyl, tetrahydro-2H-pyranyl, oxazolyl and pyridyl,
wherein cyclobutyl and cyclohexyl can be substituted by from 1 to 2 substituents selected independently of one another from the group consisting of hydroxy and methoxy,
and
wherein ethyl, n-propyl and n-butyl can be substituted by a substituent selected from the group consisting of methoxy and trifluoromethoxy,
R⁴ represents hydrogen,
R⁵ represents a group of the formula 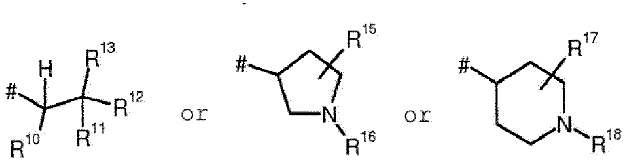 wherein # is the point of attachment to the oxygen atom,
R¹⁰ represents hydrogen or methyl,
R¹¹ represents hydrogen or methyl,
R¹² represents amino, -N⁺(CH₃)₃, pyrrolidinyl, piperidinyl, piperazinyl or -NH(C=O)R¹⁴,
wherein pyrrolidinyl, piperidinyl and piperazinyl can be substituted by a substituent methyl,
and
wherein
R¹⁴ represents C₁-C₄-alkyl, pyrrolidinyl, piperidinyl, piperazinyl or cyclopropyl,
wherein alkyl can be substituted by a substituent selected from the group consisting of amino, methyl-amino, dimethylamino, pyrrolidinyl, piperidinyl and piperazinyl,
wherein pyrrolidinyl, piperidinyl and piperazinyl can in turn be substituted by a substituent methyl,
and
wherein pyrrolidinyl, piperidinyl and piperazinyl can be substituted by a substituent methyl,
and
wherein cyclopropyl can be substituted by a substituent selected from the group consisting of amino, methylamino and dimethyl-amino,
R¹³ represents hydrogen or hydroxycarbonyl,
R¹⁵ represents hydrogen or hydroxycarbonyl,
R¹⁶ represents hydrogen or methyl,
R¹⁷ represents hydrogen or hydroxycarbonyl,
R¹⁸ represents hydrogen or methyl,
R⁹ represents hydrogen,
or one of its salts, its solvates or the solvates of its salts.

3. Compound according to either Claim 1 or Claim 2, **characterized in that**
R¹ represents a group of the formula 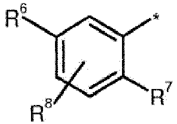 wherein * is the point of attachment to the oxopyridine ring,
R⁶ represents chlorine,
R⁷ represents cyano,
R⁸ represents hydrogen,
R² represents methoxy,
R³ represents methyl or ethyl,
wherein methyl can be substituted by a substituent tetrahydro-2H-pyranyl,
and
wherein ethyl can be substituted by a substituent methoxy,
R⁴ represents hydrogen,
R⁵ represents a group of the formula 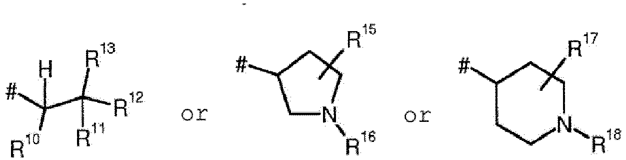 wherein # is the point of attachment to the oxygen atom,
R¹⁰ represents hydrogen or methyl,
R¹¹ represents hydrogen or methyl,
R¹² represents amino, pyrrolidinyl, piperidinyl, piperazinyl or -NH (C=O)R¹⁴,
wherein piperazinyl can be substituted by a substituent methyl,
and
wherein
R¹⁴ represents C₁-C₄-alkyl, pyrrolidinyl, piperidinyl or cyclopropyl,
wherein alkyl can be substituted by a substituent selected from the group consisting of amino, dimethylamino and pyrrolidinyl,
and
wherein pyrrolidinyl and piperidinyl can be substituted by a substituent methyl,
and
wherein cyclopropyl can be substituted by a substituent dimethylamino,
R¹³ represents hydrogen or hydroxycarbonyl,
R¹⁵ represents hydrogen or hydroxycarbonyl,
R¹⁶ represents hydrogen or methyl,
R¹⁷ represents hydrogen or hydroxycarbonyl,
R¹⁸ represents hydrogen or methyl,
R⁹ represents hydrogen,
or one of its salts, its solvates or the solvates of its salts.

4. Process for the preparation of a compound of formula (I) or of one of its salts, its solvates or the solvates of its salts according to Claim 1, **characterized in that** a compound of formula 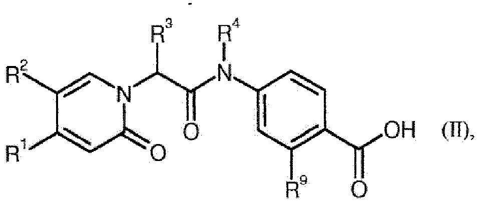 in which
R¹, R², R³, R⁴ and R⁹ have the meaning given in Claim 1,
is reacted in the first stage with a compound of formula
HO-R^{5a} (III),
in which
R^{5a} has the meaning given in Claim 1 for R⁵, wherein the functional groups in R⁵ can be protected with protecting groups,
in the presence of a dehydrating reagent,
and is reacted in a second stage by cleavage of the protecting groups to give a compound of formula 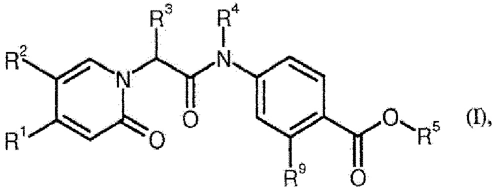 in which
R¹, R², R³, R⁴, R⁵ and R⁹ have the meaning given in Claim 1.

5. Compound according to one of Claims 1 to 3 for the treatment and/or prophylaxis of diseases.

6. Use of a compound according to one of Claims 1 to 3 in the preparation of a medicament for the treatment and/or prophylaxis of diseases.

7. Use of a compound according to one of Claims 1 to 3 in the preparation of a medicament for the treatment and/or prophylaxis of thrombotic or thromboembolic disorders.

8. Use of a compound according to one of Claims 1 to 3 in the preparation of a medicament for the treatment and/or prophylaxis of ophthalmological disorders.

9. Use of a compound according to one of Claims 1 to 3 in the preparation of a medicament for the treatment and/or prophylaxis of hereditary angioedema or inflammatory disorders of the intestine, such as Crohn's disease or ulcerative colitis.

10. Medicament comprising a compound according to one of Claims 1 to 3 in combination with an inert, non-toxic, pharmaceutically suitable excipient.

11. Medicament according to Claim 10 for the treatment and/or prophylaxis of thrombotic or thromboembolic disorders.

12. Medicament according to Claim 10 for the treatment and/or prophylaxis of ophthalmological disorders.

13. Medicament according to Claim 10 for the treatment and/or prophylaxis of hereditary angioedema or inflammatory disorders of the intestine, such as Crohn's disease or ulcerative colitis.

## Revendications

1. Composé de formule 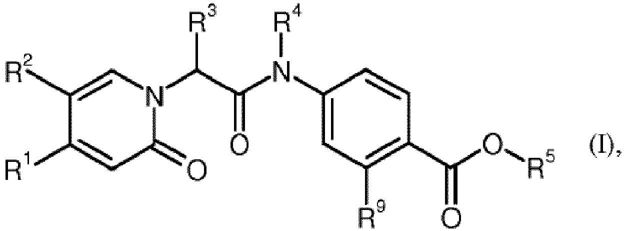 dans laquelle
R¹ représente un groupe de formule 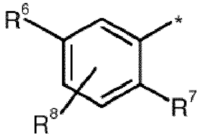
dans laquelle * est l'emplacement de liaison au cycle oxopyridine,
R⁶ représente brome, chlore, fluor, méthyle, difluorométhyle, trifluorométhyle, méthoxy, difluorométhoxy ou trifluorométhoxy,
R⁷ représente brome, chlore, fluor, cyano, nitro, hydroxy, méthyle, difluorométhyle, trifluorométhyle, méthoxy, éthoxy, difluorométhoxy, trifluorométhoxy, éthynyle, 3,3,3-trifluoroprop-1-yn-1-yle ou cyclopropyle,
R⁸ représente hydrogène, chlore ou fluor,
R² représente hydrogène, brome, chlore, fluor, cyano, alkyle en C₁-C₃, difluorométhyle, trifluorométhyle, 1,1-difluoroéthyle, 2,2-difluoroéthyle, 2,2,2-trifluoroéthyle, alcoxy en C₁-C₃, difluorométhoxy, trifluorométhoxy, 1,1-difluoroéthoxy, 2,2-difluoroéthoxy, 2,2,2-trifluoroéthoxy, méthylcarbonyle ou cyclopropyle,
R³ représente hydrogène, alkyle en C₁-C₅, alcoxy en C₁-C₄, difluorométhyle, trifluorométhyle, 1,1-difluoroéthyle, 1,1,2,2,2-pentadeutéroéthyle, 3,3,3-trifluoro-2-hydroxyprop-1-yle, 3,3,3-trifluoro-2-méthoxyprop-1-yle, 3,3,3-trifluoro-2-éthoxyprop-1-yle, prop-2-yn-1-yle, cyclopropyloxy ou cyclobutyloxy,
l'alkyle pouvant être substitué avec un substituant choisi dans le groupe constitué par fluor, cyano, hydroxy, difluorométhyle, trifluorométhyle, méthoxy, éthoxy, difluorométhoxy, trifluorométhoxy, cycloalkyle en C₃-C₆, oxo-hétérocyclyle de 4 à 6 chaînons, 1,4-dioxanyle, oxazolyle, phényle et pyridyle,
le cycloalkyle pouvant être substitué avec 1 à 2 substituants choisis indépendamment l'un de l'autre dans le groupe constitué par fluor, hydroxy, méthyle, éthyle, méthoxy, éthoxy, difluorométhyle, trifluorométhyle, difluorométhoxy et trifluorométhoxy,
et
l'oxo-hétérocyclyle pouvant être substitué avec 1 à 2 substituants choisis indépendamment l'un de l'autre dans le groupe constitué par oxo, fluor, méthyle, éthyle, difluorométhyle et trifluorométhyle,
R⁴ représente l'hydrogène,
R⁵ représente un groupe de formule 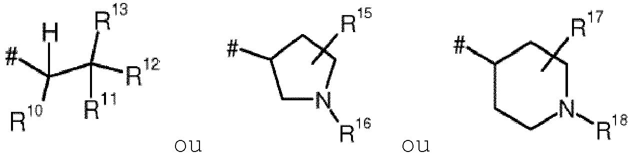
dans lesquelles # est l'emplacement de liaison à l'atome d'oxygène,
R¹⁰ représente hydrogène ou méthyle,
R¹¹ représente hydrogène ou méthyle,
R¹² représente amino, -N⁺(CH₃)₃, un hétérocyclyle azoté à 5 ou 6 chaînons ou -NH(C=O)R¹⁴,
l'hétérocyclyle pouvant être substitué avec 1 à 2 substituants choisis indépendamment l'un de l'autre dans le groupe constitué par alkyle en C₁-C₃,
et
R¹⁴ représentant alkyle en C₁-C₄, un hétérocyclyle azoté à 5 ou 6 chaînons ou cyclopropyle,
l'alkyle pouvant être substitué avec un substituant choisi dans le groupe constitué par amino, méthylamino, diméthylamino et un hétérocyclyle azoté à 5 ou 6 chaînons,
l'hétérocyclyle pouvant lui-même être substitué avec 1 à 2 substituants choisis indépendamment l'un de l'autre dans le groupe constitué par alkyle en C₁-C₃,
et
l'hétérocyclyle pouvant être substitué avec 1 à 2 substituants choisis indépendamment l'un de l'autre dans le groupe constitué par alkyle en C₁-C₃,
et
le cyclopropyle pouvant être substitué avec un substituant choisi dans le groupe constitué par amino, méthylamino et diméthylamino,
R¹³ représente hydrogène ou hydroxycarbonyle,
R¹⁵ représente hydrogène ou hydroxycarbonyle,
R¹⁶ représente hydrogène ou méthyle,
R¹⁷ représente hydrogène ou hydroxycarbonyle,
R¹⁸ représente hydrogène ou méthyle,
R⁹ représente hydrogène ou fluor,
ou un de ses sels, de ses solvates ou des solvates de ses sels.

2. Composé selon la revendication 1, **caractérisé en ce que**
R¹ représente un groupe de formule 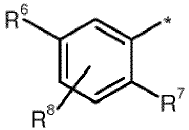
dans laquelle * est l'emplacement de liaison au cycle oxopyridine,
R⁶ représente chlore,
R⁷ représente cyano, difluorométhyle ou difluorométhoxy,
R⁸ représente hydrogène ou fluor,
R² représente chlore, cyano, éthoxy, méthoxy ou difluorométhoxy,
R³ représente méthyle, éthyle, n-propyle, 2-méthyl-prop-1-yle ou n-butyle,
le méthyle pouvant être substitué avec un substituant choisi dans le groupe constitué par cyclopropyle, cyclobutyle, cyclohexyle, tétrahydrofuranyle, tétrahydro-2H-pyranyle, oxazolyle et pyridyle,
le cyclobutyle et le cyclohexyle pouvant être substitués avec 1 à 2 substituants choisis indépendamment l'un de l'autre dans le groupe constitué par hydroxy et méthoxy,
et
l'éthyle, le n-propyle et le n-butyle pouvant être substitués avec un substituant choisi dans le groupe constitué par méthoxy et trifluorométhoxy,
R⁴ représente l'hydrogène,
R⁵ représente un groupe de formule 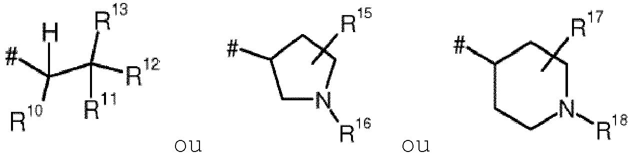
dans lesquelles # est l'emplacement de liaison à l'atome d'oxygène,
R¹⁰ représente hydrogène ou méthyle,
R¹¹ représente hydrogène ou méthyle,
R¹² représente amino, -N⁺(CH₃)₃, pyrrolidinyle, pipéridinyle, pipérazinyle ou -NH(C=O)R¹⁴,
le pyrrolidinyle, le pipéridinyle et le pipérazinyle pouvant être substitués avec un substituant méthyle,
et
R¹⁴ représentant alkyle en C₁-C₄, pyrrolidinyle, pipéridinyle, pipérazinyle ou cyclopropyle,
l'alkyle pouvant être substitué avec un substituant choisi dans le groupe constitué par amino, méthylamino, diméthylamino, pyrrolidinyle, pipéridinyle et pipérazinyle,
le pyrrolidinyle, le pipéridinyle et le pipérazinyle pouvant eux-mêmes être substitués avec un substituant méthyle,
et
le pyrrolidinyle, le pipéridinyle et le pipérazinyle pouvant être substitués avec un substituant méthyle,
et
le cyclopropyle pouvant être substitué avec un substituant choisi dans le groupe constitué par amino, méthylamino et diméthylamino,
R¹³ représente hydrogène ou hydroxycarbonyle,
R¹⁵ représente hydrogène ou hydroxycarbonyle,
R¹⁶ représente hydrogène ou méthyle,
R¹⁷ représente hydrogène ou hydroxycarbonyle,
R¹⁸ représente hydrogène ou méthyle,
R⁹ représente hydrogène,
ou un de ses sels, de ses solvates ou des solvates de ses sels.

3. Composé selon l'une quelconque des revendications 1 ou 2, **caractérisé en ce que**
R¹ représente un groupe de formule 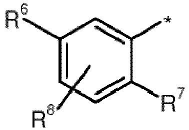
dans laquelle * est l'emplacement de liaison au cycle oxopyridine,
R⁶ représente chlore,
R⁷ représente cyano,
R⁸ représente hydrogène,
R² représente méthoxy,
R³ représente méthyle ou éthyle,
le méthyle pouvant être substitué avec un substituant tétrahydro-2H-pyranyle,
et
l'éthyle pouvant être substitué avec un substituant méthoxy,
R⁴ représente l'hydrogène,
R⁵ représente un groupe de formule 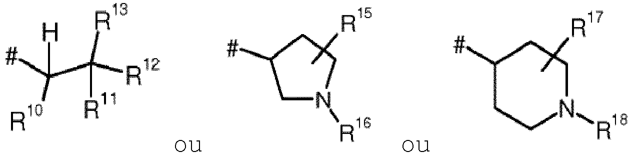
dans lesquelles # est l'emplacement de liaison à l'atome d'oxygène,
R¹⁰ représente hydrogène ou méthyle,
R¹¹ représente hydrogène ou méthyle,
R¹² représente amino, pyrrolidinyle, pipéridinyle, pipérazinyle ou -NH(C=O)R¹⁴,
le pipérazinyle pouvant être substitué avec un substituant méthyle,
et
R¹⁴ représentant alkyle en C₁-C₄, pyrrolidinyle, pipéridinyle ou cyclopropyle,
l'alkyle pouvant être substitué avec un substituant choisi dans le groupe constitué par amino, diméthylamino et pyrrolidinyle,
et
le pyrrolidinyle et le pipéridinyle pouvant être substitués avec un substituant méthyle,
et
le cyclopropyle pouvant être substitué avec un substituant diméthylamino,
R¹³ représente hydrogène ou hydroxycarbonyle,
R¹⁵ représente hydrogène ou hydroxycarbonyle,
R¹⁶ représente hydrogène ou méthyle,
R¹⁷ représente hydrogène ou hydroxycarbonyle,
R¹⁸ représente hydrogène ou méthyle,
R⁹ représente hydrogène,
ou un de ses sels, de ses solvates ou des solvates de ses sels.

4. Procédé de fabrication d'un composé de formule (I) ou d'un de ses sels, de ses solvates ou des solvates de ses sels selon la revendication 1, **caractérisé en ce qu'**un composé de formule 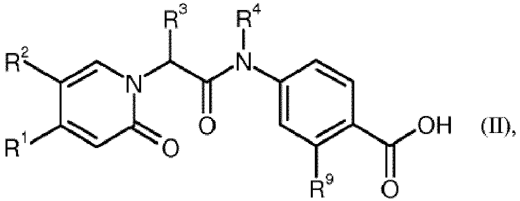 dans laquelle
R¹, R², R³, R⁴ et R⁹ ont la signification indiquée dans la revendication 1,
est mis en réaction lors de la première étape avec un composé de formule
HO-R^{5a} (III)
dans laquelle
R^{5a} a la signification indiquée pour R⁵ dans la revendication 1, les groupes fonctionnels dans R⁵ pouvant être protégés avec des groupes protecteurs,
en présence d'un réactif de déshydratation,
et, lors d'une deuxième étape, mis en réaction par clivage des groupes protecteurs pour former un composé de formule 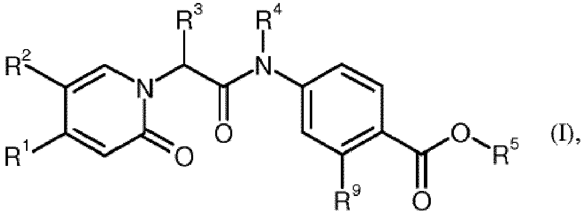 dans laquelle
R¹, R², R³, R⁴, R⁵ et R⁹ ont la signification indiquée dans la revendication 1.

5. Composé selon l'une quelconque des revendications 1 à 3, pour le traitement et/ou la prophylaxie de maladies.

6. Utilisation d'un composé selon l'une quelconque des revendications 1 à 3 pour la fabrication d'un médicament pour le traitement et/ou la prophylaxie de maladies.

7. Utilisation d'un composé selon l'une quelconque des revendications 1 à 3 pour la fabrication d'un médicament pour le traitement et/ou la prophylaxie de maladies thrombotiques ou thromboemboliques.

8. Utilisation d'un composé selon l'une quelconque des revendications 1 à 3 pour la fabrication d'un médicament pour le traitement et/ou la prophylaxie de maladies ophtalmiques.

9. Utilisation d'un composé selon l'une quelconque des revendications 1 à 3 pour la fabrication d'un médicament pour le traitement et/ou la prophylaxie d'angio-oedèmes héréditaires ou de maladies inflammatoires de l'intestin, telles que la maladie de Crohn ou la colite ulcéreuse.

10. Médicament contenant un composé selon l'une quelconque des revendications 1 à 3 en combinaison avec un adjuvant inerte, non toxique, pharmaceutiquement approprié.

11. Médicament selon la revendication 10 pour le traitement et/ou la prophylaxie de maladies thrombotiques ou thromboemboliques.

12. Médicament selon la revendication 10 pour le traitement et/ou la prophylaxie de maladies ophtalmiques.

13. Médicament selon la revendication 10 pour le traitement et/ou la prophylaxie d'angio-oedèmes héréditaires ou de maladies inflammatoires de l'intestin, telles que la maladie de Crohn ou la colite ulcéreuse.
